# EUROPEAN PATENT APPLICATION

(11) **EP 4 755 892 A1**
(43) Date of publication of application: **10.06.2026**
(21) Application number: 24848346.3
(22) Date of filing: 01.08.2024
(51) Int. Cl.: C07D 498/18, C07D 487/08, C07D 487/18, C07D 487/22, A61K 31/5386, A61K 31/529, A61P 35/00

(54) **KINESIN KIF18A INHIBITOR AND USE THEREOF**

(30) Priority: 02.08.2023 CN 202310967283; 26.09.2023 CN 202311254609
(71) Applicant: Shanghai Apeiron Therapeutics Company Limited, Shanghai 201210 (CN)
(72) Inventor: XIAO, Yisong, Shanghai 201210 (CN); LAI, Kunmin, Shanghai 201210 (CN); GU, Xiaohui, Shanghai 201210 (CN); WANG, Minghua, Shanghai 201210 (CN); DENG, Jiachen, Shanghai 201210 (CN); HU, Min, Shanghai 201210 (CN)
(74) Representative: Bayramoglu et al.
(86) International application number: PCT/CN2024/109189
(87) International publication number: WO 2025/026392

(57) **Abstract**

The present disclosure provides a KIF18 inhibitor and its synthetic method. Compounds of the present disclosure are capable of regulating the KIF18A protein, thereby influencing cell cycle and proliferation processes for the treatment of cancers and cancer-related diseases. The present disclosure also encompasses pharmaceutical compositions containing the compounds and methods for treating conditions associated with KIF18A activity.

## Description

### TECHNICAL FIELD

The present disclosure belongs to the field of medicine and relates to a class of kinesin KIF18A inhibitors and their use for inhibiting cancer cell proliferation and treating cancers.

### BACKGROUND ART

KIF18A is a member of the kinesin-8 family. It moves towards the plus ends of microtubules within cells using microtubules as tracks, relying on the energy released from ATP hydrolysis. Upon reaching the plus ends of microtubules, KIF18A regulates the dynamic instability of microtubules and exerts an activity similar to that of microtubule depolymerase. During mitosis, KIF18A regulates spindle microtubule dynamics and chromosome amplitude, playing a critical role in the timely completion of chromosome alignment at mitosis, maintenance of genomic stability and successful completion of mitosis.

The KIF18A gene belongs to the kinesin-8 subfamily and is a plus-end directed motor. KIF18A is believed to influence the dynamics of the plus ends of centromeric microtubules, thereby regulating correct chromosome orientation and spindle tension. Depletion of human KIF18A results in longer spindles, increased chromosome oscillation at metaphase and activated mitotic spindle assembly checkpoint in HeLa cervical cancer cells (MI Mayr et al., Current Biology 17, 488-98, 2007). KIF18A appears to be a viable target for cancer therapy. KIF18A is overexpressed in multiple types of cancers, including but not limited to colon, breast, lung, pancreatic, prostatic, bladder, head, neck, cervical and ovarian cancers. Furthermore, in cancer cell lines, gene deletion or knockout or KIF18A inhibition affects the mitotic spindle apparatus. In particular, inhibition of KIF18A has been found to induce mitotic cell arrest, a known vulnerability that promotes mitotic cell death through apoptosis, mitotic catastrophe, polyploidy-driven lethality, or death following mitotic slippage into interphase. As a result, there is a strong interest in finding inhibitors of KIF18A protein. Therefore, inhibition of KIF18A ATPase activity is a promising approach to developing new anticancer agents.

### CONTENT OF INVENTION

The present disclosure belongs to the field of medicine and relates to a class of kinesin KIF18A inhibitors, and specifically relates to the compounds or their stereoisomers, tautomers, mesomers, racemates, enantiomers, diastereomers, or mixtures thereof, or pharmaceutically acceptable salts, co-crystals, metabolites, solvates, prodrugs, or isotopically labeled compounds thereof, the preparation methods thereof, pharmaceutical compositions containing such compounds, and their use as therapeutic agents, particularly for inhibiting cancer cell proliferation and treating cancer.

The present disclosure provides a novel class of compounds that regulate KIF18A protein, alone or in microtubule-bound complexes, for the treatment of KIF18A-mediated disorders and/or diseases, including cancer, inflammation or ciliopathy.

The compounds of the present disclosure exhibit MT-based regulatory activity on KIF18A, specifically inhibitory activity on KIF18A. To this end, the present disclosure also provides the use of these compounds and their pharmaceutically acceptable salts in the preparation and manufacture of pharmaceutical compositions or medicines for therapeutic, prophylactic, acute or chronic treatment of KIF18A-mediated diseases and disorders (including but not limited to cancers).

The present disclosure provides an Example: a compound having the structure of Formula I, or pharmaceutically acceptable salts, stereoisomers or isotope isomers thereof: 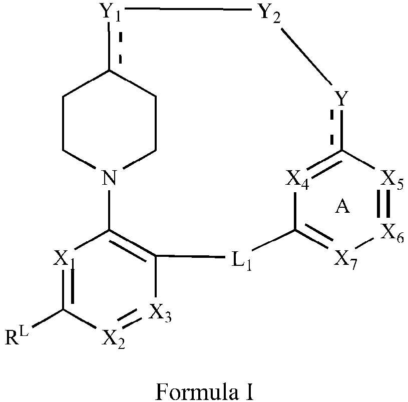
wherein, -̅ -̅ -̅ represents a single bond or double bond;
wherein, X₁ represents CR^{W1} or N;
wherein, X₂ represents CR^{W2} or N;
wherein, X₃ represents CR^{W3} or N;
wherein, X₄ represents CR^{W4} or N;
wherein, X₅ represents CR^{W5} or N;
wherein, X₆ represents CR^{W6} or N;
wherein, X₇ represents CR^{W7} or N;
wherein, R^{W1}, R^{W2}, R^{W3}, R^{W4}, R^{W5}, R^{W6} and R^{W7} each independently represent hydrogen, halogen, cyano, nitro, hydroxy (C₁-C₆) alkyl, C₁-C₆ alkyl, C₃-C₆ cycloalkyl, C₁-C₆ haloalkyl, -OR^{a}, -SO₃R^{a}, -S(O)R^{a}, -O-C₁-C₆ haloalkyl, -SR^{a}, -C(=O)ORa, -C(=O)NR^{a}R^{b}, -SF₅ or -NR^{a}R^{b};
wherein, the chemical bond between X₂ and X₃, or between X₅ and X₆, or between X₆ and X₇ may be fused with ring A to form a 5-6-membered saturated or unsaturated ring which may optionally contain 0, 1 or 2 heteroatoms selected from O, S and N; the ring may be optionally substituted with 0-3 substituents selected from the following: halogen, cyano, nitro, hydroxy (C₁-C₆) alkyl, C₁-C₆ alkyl, C₃-C₈ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b};
wherein, L₁ represents -C(O)NH- or triazolyl;
wherein, Y₁ represents -(CR^{a}R^{b})ₘ₋; Y₂ represents -(CR^{a}R^{b})ₙ₋;
wherein, optionally, CR^{a}R^{b} may be substituted with -O-, -S-, -NR^{a}-, -NR^{a}SO₂-, -NR^{a}C(O)-, -C(O)NR^{a}-, -SO₂NR^{a}-, -S(=O)(NR^{a})-, -P(O)(OR^{a})₂-, -NR^{a}P(O)(OR^{a})₂- or -NR^{a}P(O)(R^{a})₂-, -CR^{a}=CR^{b}-, 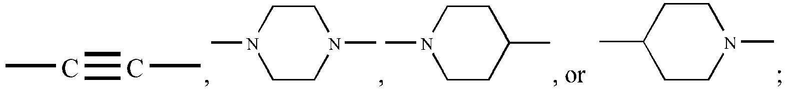
wherein, R^{L} represents L₂-R^{M};
wherein, L₂ represents absence, -C₁-C₆ alkyl-, -NR^{a}-, -NR^{a}SO₂-, -SO₂NR^{a}-, -NR^{a}-S(=O)(=NH), -S(=O) (=NH)-, -S-, -S(=O)-, -SO₂-, -C₁-C₆ alkyl-O-, -(C=O)-, -(C=O)NR^{a}-, -C=N(OH)-, -NR^{a} (C=O), -P(O)(OR^{a})₂, -NR^{a}P(O)(OR^{a})₂ or -NR^{a}P(O)(R^{a})₂-;
wherein, R^{M} represents C₁-C₆ alkyl or C₃-C₆ cycloalkyl, wherein the C₁-C₆ alkyl or C₃-C₆ cycloalkyl can be optionally independently substituted with 0-3 substituents selected from halogen, -OR^{a}, -NR^{a}R^{b}, cyano and -O-C₁-C₆haloalkyl;
alternatively, L-R^{M} represents -N=S(=O)-(R^{L})₂, wherein the two R^{L} groups, together with the sulfur atom attached thereto, form a saturated or unsaturated 3-, 4-, 5- or 6-membered monocyclic ring containing 0, 1 or 2 heteroatoms selected from N, O and S;
wherein, m and n each independently represent an integer from 0 to 10;
wherein, when -̅ -̅ -̅ represents a single bond, Y represents -O-, -NH-, or the following groups: 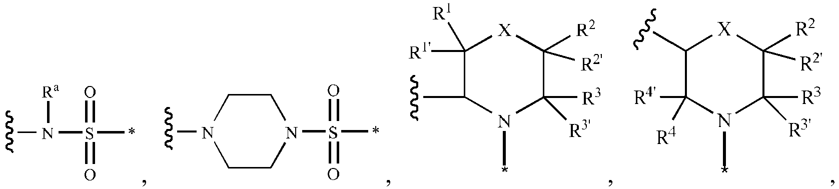 or 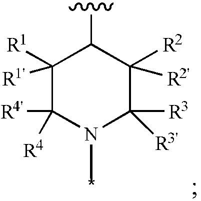
wherein, * represents the site of connection with ring A; the wavy line represents the site of connection with Y₂;
wherein, X represents CR¹R^{1'}, O or NR^{a};
wherein, R¹, R^{1'}, R², R^{2'}, R³, R^{3'}, R⁴ and R^{4'} each independently represent hydrogen, C₁-C₆ alkyl, halogen or C₃-C₆ cycloalkyl; hydroxy (C₁-C₆) alkyl;
alternatively, R¹ and R^{1'}, together with the carbon atom attached thereto, form a 3-6 membered ring;
alternatively, R² and R^{2'}, together with the carbon atom attached thereto, form a 3-6 membered ring;
alternatively, R³ and R^{3'}, together with the carbon atom they are attached to, form a 3-6 membered ring;
alternatively, R⁴ and R^{4'}, together with the carbon atom they are attached to, form a 3-6 membered ring;
wherein, R^{a} and R^{b} each independently represent hydrogen, halogen, C₁-C₆ alkyl, C₃-C₆ cycloalkyl, C₁-C₆ haloalkyl, hydroxy (C₁-C₆) alkyl, C₁-C₆ alkyl substituted with saturated or unsaturated 5-6-membered cycloalkyl, or C₁-C₆ alkyl substituted with saturated or unsaturated 5-6-membered heterocycloalkyl; alternatively, R^{a} and R^{b}, together with the atom attached thereto, form a 3-6-membered saturated or unsaturated ring which may optionally contain 0-2 heteroatoms selected from O, S and N.

In an embodiment of the present disclosure, X₁, X₂, X₃, X₄ and X₅ represent CH or N.

In an embodiment of the present disclosure, X₁ and X₂ represent CH or N.

In an embodiment of the present disclosure, X₄ and X₅ represent CH or N.

In an embodiment of the present disclosure, Y₁ represents -(CR^{a}R^{b})ₘ-.

In an embodiment of the present disclosure, Y₁ represents -NH-C(=O)-.

In an embodiment of the present disclosure, Y₁ represents -O(CR^{a}R^{b})ₘ- or -(CR^{a}R^{b}) ₘO-.

In an embodiment of the present disclosure, Y₁ represents -CR^{a}=CR^{a}- or -CR^{a}R^{b}-CR^{a}=CR^{b}-.

In an embodiment of the present disclosure, Y₂ represents -(CR^{a}R^{b})ₙ-.

In an embodiment of the present disclosure, Y₂ represents -NR^{a}-C(=O)-.

In an embodiment of the present disclosure, Y₂ represents -O(CR^{a}R^{b})ₙ- or -(CR^{a}R^{b})ₙO-.

In an embodiment of the present disclosure, Y₂ represents -CR^{a}=CR^{a}-, -CR^{a}=CR^{b}-CR^{a}R^{b}- or -CR^{a}R^{b}-CR^{a}=CR^{a}-.

In an embodiment of the present disclosure, -Y₁-Y₂ represents -CR^{a}=CR^{a}-.

In an embodiment of the present disclosure, -Y₁-Y₂ represents -CR^{a}R^{b}-NR^{a}C(O)-.

In an embodiment of the present disclosure, -Y₁-Y₂ represents

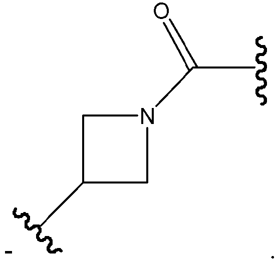

In an embodiment of the present disclosure, Y represents -O- or -NH-.

In an embodiment of the present disclosure, Y represents

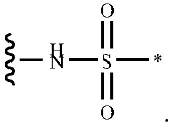

In an embodiment of the present disclosure, Y represents

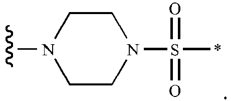

In an embodiment of the present disclosure, Y represents

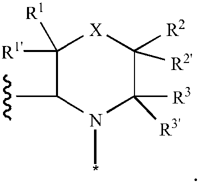

In an embodiment of the present disclosure, Y represents

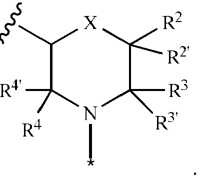

In an embodiment of the present disclosure, Y represents

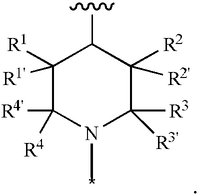

In an embodiment of the present disclosure, X represents O, NH or CR¹R^{1'}.

In an embodiment of the present disclosure, X represents CH₂ or CF₂.

In an embodiment of the present disclosure, R², R^{2'}, R³, R^{3'}, R⁴ and R^{4'} each independently represent hydrogen, CH₃, or together with the carbon atom attached thereto form a cyclopropyl.

In an embodiment of the present disclosure, the chemical bond between X₅ and X₆ in ring A is fused with ring A to form a 5-6-membered saturated or unsaturated ring which may optionally contain 0, 1 or 2 heteroatoms selected from O, S and N; the ring may be optionally substituted with 0-3 substituents selected from the following: halogen, cyano, nitro, hydroxy (C₁-C₆) alkyl, C₁-C₆ alkyl, C₃-C₈ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b};

In an embodiment of the present disclosure, the chemical bond between X₅ and X₆ in ring A is fused with ring A to form a benzene ring or a pyridine ring; the ring may be optionally substituted with 0-3 substituents selected from the following: halogen, cyano, nitro, hydroxy (C₁-C₆) alkyl, C₁-C₆ alkyl, C₃-C₈ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b}.

**In** an embodiment of the present disclosure, ring A is fused with a pyrrole ring at the bond between X₅ and X₆; the ring may be optionally substituted with 0-3 substituents selected from the following: halogen, cyano, nitro, hydroxy (C₁-C₆) alkyl, C₁-C₆ alkyl, C₃-C₈ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b}.

In an embodiment of the present disclosure, R^{L} represents L-R^{M}; wherein, L represents -NR^{a}SO₂- or -SO₂NR^{a}-.

In an embodiment of the present disclosure, R^{M} represents C₁-C alkyl substituted with 0, 1, 2 or 3 OH groups.

In particular, the present disclosure provides a compound having the following structures:

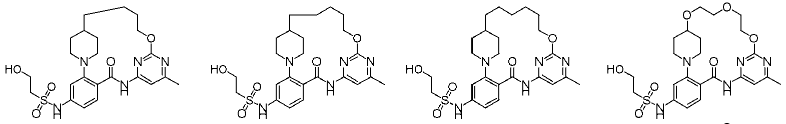

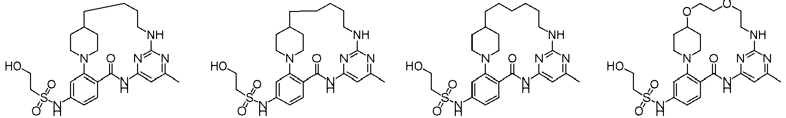

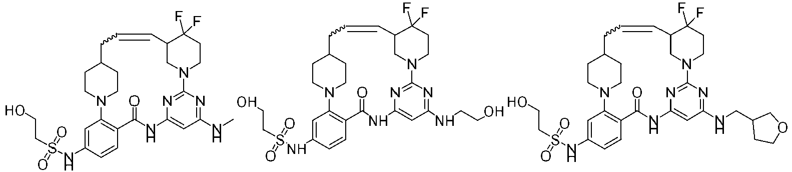

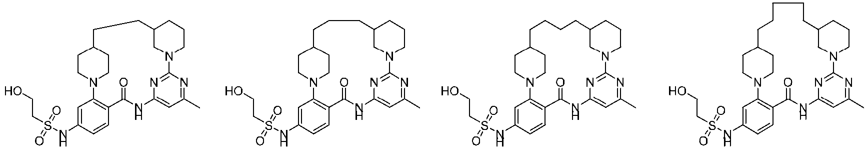

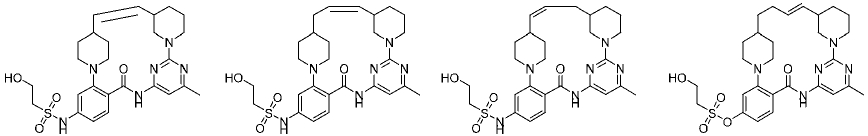

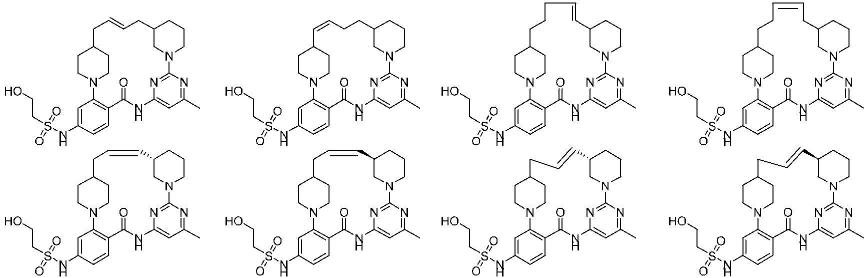

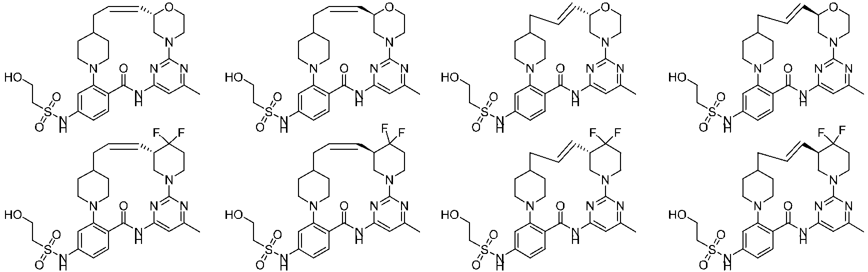

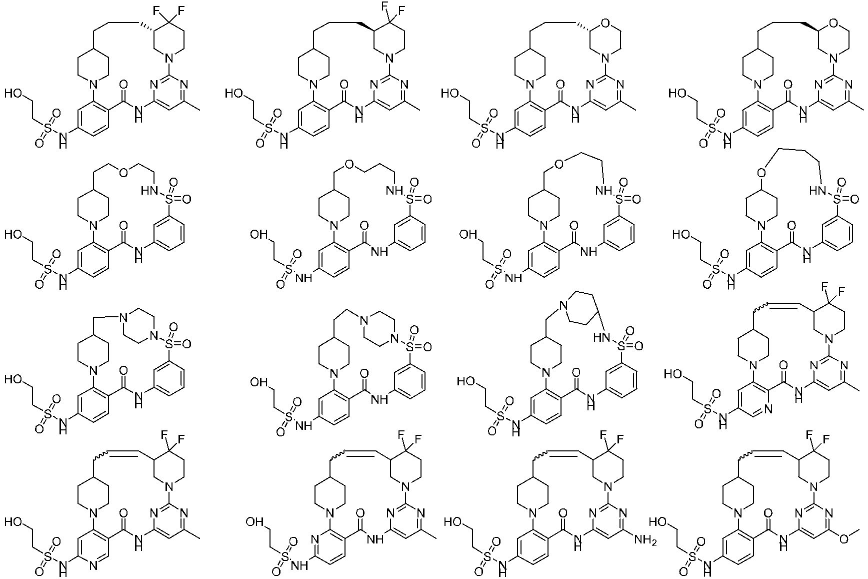

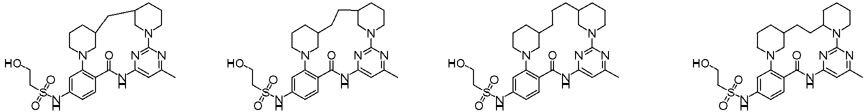

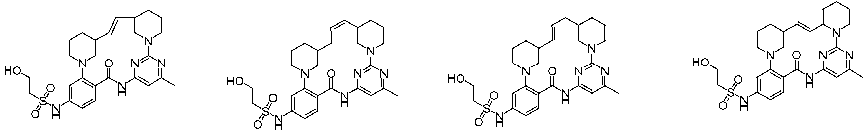

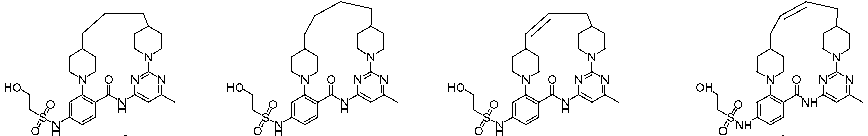

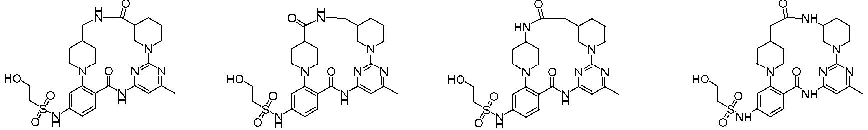

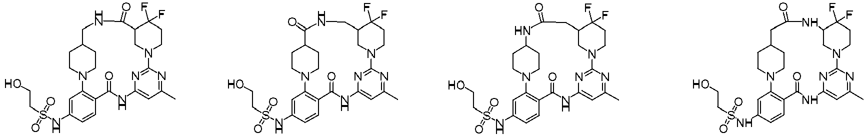

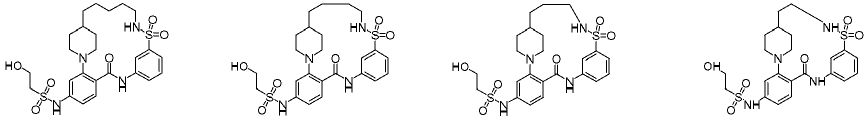

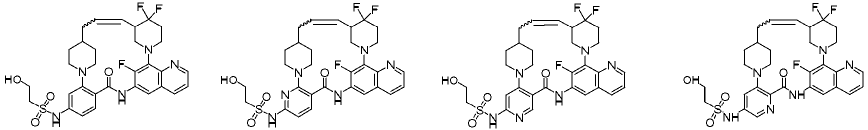

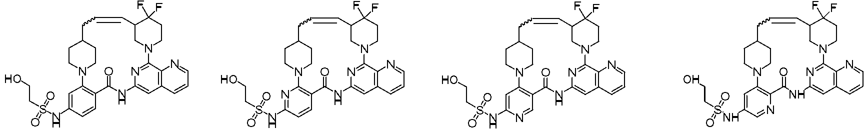

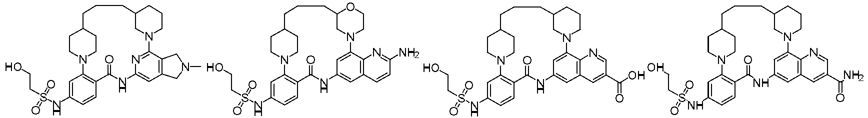

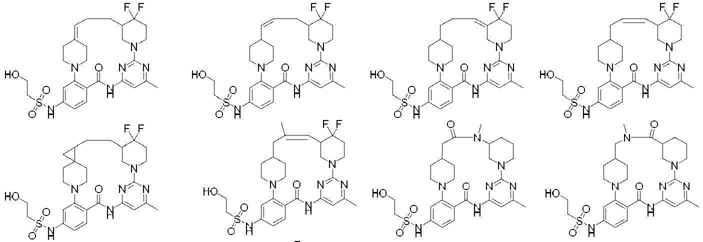

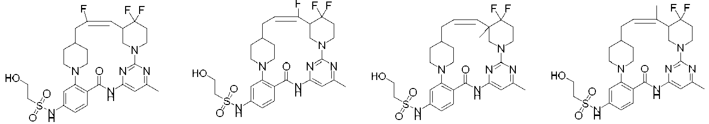

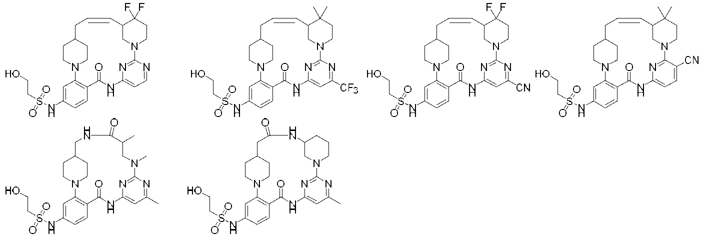

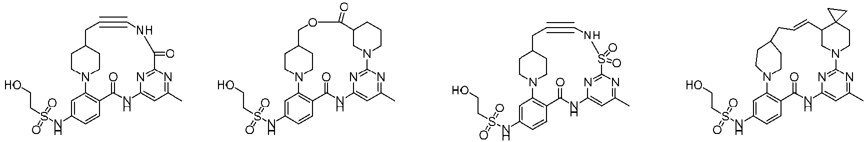

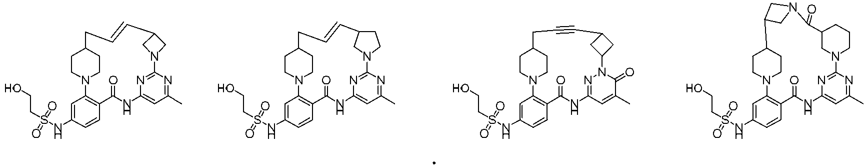

In addition, the present disclosure provides a pharmaceutical composition that contains any one of the compounds in the embodiments of the present disclosure or pharmaceutically acceptable salts, stereoisomers, isotope isomers, prodrugs, hydrates or solvates thereof and pharmaceutically acceptable carriers.

In addition, the present disclosure provides a method for treating tumors by inhibiting KIF18A, comprising administering any one of the compounds of the present disclosure or pharmaceutically acceptable salts, stereoisomers, isotope isomers, prodrugs, hydrates or solvates thereof to an individual in need.

### DEFINITION

Unless otherwise specified, the compounds of the present disclosure may encompass, in addition to their specific structures, pharmaceutically acceptable salts, stereoisomers, isotope isomers (e.g., deuterates), solvates, hydrates, prodrugs and metabolites thereof. Thus, the pharmaceutically acceptable salts, stereoisomers, isotope isomers, solvates, hydrates, prodrugs, and metabolites of these compounds are also considered within the scope of protection.

Unless otherwise specified, the terms used in this specification of patent and claims are defined as follows. Furthermore, many of the groups defined herein may be optionally substituted. A list of typical substituents is presented in Definition by way of example and is not intended to limit the substituents defined elsewhere in this specification of patent and claims.

The term "alkyl" refers to a linear or branched saturated aliphatic hydrocarbyl or linker, comprising 1-20 carbon atoms, preferably 1-12 carbon atoms, more preferably 1-8 carbon atoms, 1-6 carbon atoms or 1-4 carbon atoms. "Lower alkyl" specifically refers to an alkyl comprising 1-4 carbon atoms. Examples of alkyl include -(CH₂)₃-, methyl, trifluoromethyl, ethyl, propyl, isopropyl, n-butyl, isobutyl, tert-butyl and pentyl. Alkyl may be either substituted or unsubstituted. Typical substituents include cycloalkyl, aryl, heteroaryl, heterocycloalkyl, hydroxyl, alkoxy, aryloxy, mercapto, alkylthio, arylthio, cyano, halo, carbonyl, thiocarbonyl, O-carbamoyl, N-carbamoyl, O-thiocarbamoyl, N-thiocarbamoyl, C-acylamino, N-amido, C-carboxyl, O-carboxyl, nitro, silyl, amino and -NR^{x}R^{y}; Wherein, R^{x} and R^{y} are independently selected from hydrogen, alkyl, cycloalkyl, aryl, carbonyl, acetyl, sulfonyl, trifluoromethanesulfonyl and a fused 5- or 6-membered heterocyclyl ring.

The term "alkenyl" refers to a linear or branched hydrocarbyl containing one or more double bonds and typically comprising 2-20 carbon atoms. For instance, "C₂-C₆ alkenyl" comprises 2-6 carbon atoms. Examples of alkenyl include, but are not limited to, vinyl, propenyl, butenyl and 1-methyl-2-buten-1-yl.

The term "alkynyl" refers to a linear or branched hydrocarbyl containing one or more triple bonds and typically comprising 2-20 carbon atoms. For example, "C₂-C₆ alkynyl" comprises 2-6 carbon atoms. Examples of alkynyl include, but are not limited to, ethynyl, 1-propynyl and 1-butynyl.

The term "alkoxy" or "alkyloxy" refers to -O-alkyl. "C₁-C₆ alkoxy" (or alkyloxy) is intended to include C₁, C₂, C₃, C₄, C₅ and C₆ alkoxy. Examples of alkoxy include, but are not limited to, methoxy, ethoxy, propoxy (e.g., n-propoxy and isopropoxy) and tert-butoxy. Similarly, "alkylthio" or "thioalkoxy" refers to sulfur-bridged alkyl comprising a specified number of carbon atoms as defined above; for example, methyl-S- and ethyl-S-.

The term "cycloalkyl" refers to a 3- to 8-membered all-carbon monocyclic or bicyclic ring, a 5-/6- or 6-/6-membered fused all-carbon bicyclic ring, or a fused polycyclic ring (in a "fused" ring system, each ring shares at least one adjacent carbon atom with other rings) group, in which one or more of the rings may contain one or more double bonds but none of such rings has an intact conjugated π-electron system, or two rings forms a spiro by sharing one carbon. Examples of cycloalkyl include, but are not limited to, cyclopropane, cyclobutane, cyclopentane, cyclopentene, cyclohexane, cyclohexadiene, adamantane, cycloheptane and cycloheptatriene. Bicyclic alkyl includes bridged, spiro or fused-ring cycloalkyl. Illustrative examples of cycloalkyl are derived from but not limited to the following:

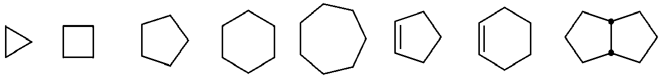

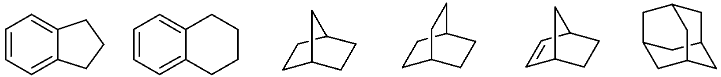

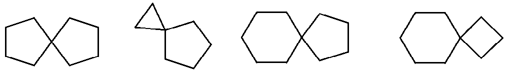

The term "aryl" refers to an all-carbon monocyclic or fused-ring polycyclic group comprising 6-12 carbon atoms, with an intact conjugated π-electron system. Examples of aryl include, but are not limited to, phenyl, naphthyl and anthracenyl. Aryl may be either substituted or unsubstituted. Typical substituents include halo, trihalomethyl, alkyl, hydroxy, alkoxy, aryloxy, mercapto, alkylthio, arylthio, cyano, nitro, carbonyl, thiocarbonyl, C-carboxyl, O-carbamoyl, N-carbamoyl, O-thiocarbamoyl, N-thiocarbamoyl, C-acylamino, N-amido, sulfinyl, sulfonyl, amino and -NR^{a}R^{b}, wherein R^{a} and R^{b} are as defined above. An aryl-fused saturated or unsaturated cycloalkyl/saturated or unsaturated heterocycloalkyl can be regarded as a special substituent of aryl, with typical examples including but not limited to:

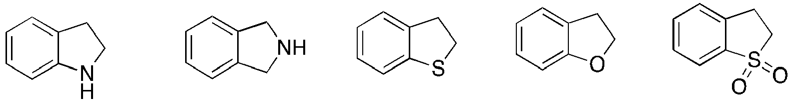

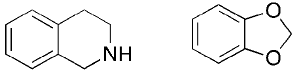

The term "heteroaryl" refers to a monocyclic or fused ring comprising 5-12 ring atoms, with one, two, three or four ring heteroatoms selected from N, O and S and the remaining ring atoms being C, having an intact conjugated π-electron system. Typical examples of heteroaryl include, but are not limited to, acridinyl, azetidinyl, azocinyl, benzimidazolyl, benzofuryl, benzothiofuranyl, benzothienyl, benzoxazolyl, benzoxazolinyl, benzothiazolyl, benzotriazolyl, benzotetrazolyl, benzoisoxazolyl, benzisothiazolyl, benzimidazolinyl, carbazolyl, 4aH-carbazolyl, carbolinyl, chromanyl, chromenyl, cinnolinyl, decahydroquinolyl, 2H,6H-1,5,2-dithiazinyl, dihydrofuro[2,3-b]tetrahydrofuranyl, furanyl, furazanyl, imidazolidinyl, imidazolinyl, imidazolyl, 1H-indazolyl, imidazopyridinyl, indolenyl, dihydroindolyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridyl, isoxazolyl, isoxazolopyridyl, methylenedioxyphenyl, morpholinyl, diazanaphthyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinyl, perimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridoxazolyl, pyridinoimidazolyl, pyridothiazolyl, pyridyl, pyrimidinyl, pyrrolidinyl, pyrrolinyl, 2-pyrrolidonyl, 2H-pyrrolyl, pyrrolyl, quinazolinyl, quinolyl, 4H-quinolizinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridyl, thienothiazolyl, thienoxazolyl, thienoimidazolyl, thienyl, triazinyl, 1,2,3-triazolyl, 1,2,4-triazolyl, 1,2,5-triazolyl, 1,3,4-triazolyl and xanthyl, quinolyl, isoquinolinyl, phthalazinyl, quinazolinyl, indolyl, isoindolyl, dihydroindolyl, 1H-indazolyl, benzimidazolyl, 1,2,3,4-tetrahydroquinolyl, 1,2,3,4-tetrahydroisoquinolyl, 5,6,7,8-tetrahydro-quinolyl, 2,3-dihydro-benzofuranyl, chromanyl, 1,2,3,4-tetrahydro-quinoxalinyl and 1,2,3,4-tetrahydro-quinazolinyl. The term "heteroaryl" may also include biaryl structures formed by the above-defined "aryl" and monocyclic "heteroaryl", including but not limited to "-phenylbipyridyl-", "-phenylbipyrimidinyl", "-pyridylbiphenyl", "-pyridylbipyrimidinyl-" and "-pyrimidinylbiphenyl-"; wherein the present disclosure also includes fused ring and spiro ring compounds containing, for example, heterocyclic rings mentioned above.

A pharmaceutically acceptable heteroaryl is sufficiently stable to be linked to a compound of the present disclosure for formulation into a pharmaceutical composition and subsequent administration to patients in need.

Unless otherwise defined, the substituents in the present disclosure are defined as being independently assigned rather than interdependently related. For example, for a substituent R^{a} (or R^{b}), its definition in different substituents is independently assigned. Specifically, when R^{a} (or R^{b}) is selected as one definition in one substituent, this does not imply that R^{a} (or R^{b}) in other substituents must share the same definition. More specifically, when R^{a} (or R^{b}) is defined as hydrogen in NR^{a}R^{b}, for example (non-exhaustive), it does not imply that R^{a} (or R^{b}) in -C(O)-NR^{a}R^{b} must be hydrogen.

"Halo" or "halogen" includes fluorine, chlorine, bromine and iodine. "Haloalkyl" is intended to include branched and linear saturated aliphatic hydrocarbyl having a specified number of carbon atoms substituted with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Examples of haloalkyl also include "fluoroalkyl" which is intended to include branched and linear saturated aliphatic hydrocarbyl having a specified number of carbon atoms and substituted with 1 or more fluorine atoms.

"Haloalkoxy" or "haloalkyloxy" refers to oxygen-bridged haloalkyl having a specified number of carbon atoms as defined above. For example, "C₁-C₆ haloalkoxy" is intended to include C₁, C₂, C₃, C₄, C₅ and C₆ haloalkoxy. Examples of haloalkoxy include, but are not limited to, trifluoromethoxy, 2,2,2-trifluoroethoxy and pentafluoroethoxy. Similarly, "haloalkylthio" or "thiohaloalkoxy" refers to sulfur-bridged haloalkyl comprising a specified number of carbon atoms as defined above; for example, trifluoromethyl-S- and pentafluoroethyl-S-.

In the present disclosure, the expression Cx1-Cx2 is used when referring to some substituents, which means that the number of carbon atoms in the substituent may be x1 to x2. For example, C₀-C₈ means that the group contains 0, 1, 2, 3, 4, 5, 6, 7 or 8 carbon atoms, C₁-C₈ means that the group contains 1, 2, 3, 4, 5, 6, 7 or 8 carbon atoms, C₂-C₈ means that the group contains 2, 3, 4, 5, 6, 7 or 8 carbon atoms, C₃-C₈ means that the group contains 3, 4, 5, 6, 7 or 8 carbon atoms, C₄-C₈ means that the group contains 4, 5, 6, 7 or 8 carbon atoms, C₀-C₆ means that the group contains 0, 1, 2, 3, 4, 5 or 6 carbon atoms, C₁-C₆ means that the group contains 1, 2, 3, 4, 5 or 6 carbon atoms, C₂-C₆ means that the group contains 2, 3, 4, 5 or 6 carbon atoms, and C₃-C₆ means that the group contains 3, 4, 5 or 6 carbon atoms.

In the present disclosure, the expression "x1-x2-membered ring" is used when referring to cyclic groups (e.g., aryl, heteroaryl, cycloalkyl and heterocycloalkyl), which means that the number of ring atoms in the group may be x1 to x2. For example, 3-12-membered cyclic group may be a 3, 4, 5, 6, 7, 8, 9, 10, 11 or 12-membered ring comprising 3, 4, 5, 6, 7, 8, 9, 10, 11 or 12 ring atoms; 3-6-membered cyclic group may be a 3, 4, 5 or 6-membered ring comprising 3, 4, 5 or 6 ring atoms; 3-8-membered cyclic group may be a 3, 4, 5, 6, 7 or 8-membered ring comprising 3, 4, 5, 6, 7 or 8 ring atoms; 3-9-membered cyclic group may be a 3, 4, 5, 6, 7, 8 or 9-membered ring comprising 3, 4, 5, 6, 7, 8 or 9 ring atoms; 4-7-membered cyclic group may be a 4, 5, 6 or 7-membered ring comprising 4, 5, 6 or 7 ring atoms; 5-8-membered cyclic group may be a 5, 6, 7 or 8-membered ring comprising 5, 6, 7 or 8 ring atoms; 5-12-membered cyclic group may be a 5, 6, 7, 8, 9, 10, 11 or 12-membered ring comprising 5, 6, 7, 8, 9, 10, 11 or 12 ring atoms; 6-12-membered cyclic group may be a 6, 7, 8, 9, 10, 11 or 12-membered ring comprising 6, 7, 8, 9, 10, 11 or 12 ring atoms. The ring atoms may be carbon atoms or heteroatoms, such as heteroatoms selected from N, O and S. The heterocyclic rings may contain 1, 2, 3, 4, 5, 6, 7, 8, 9, 10 or more ring heteroatoms, such as heteroatoms selected from N, O and S.

In the present disclosure, one or more halogens may independently be selected from fluorine, chlorine, bromine, and iodine.

The term "substituted" as used herein means that at least one hydrogen atom is substituted by a non-hydrogen group, provided that normal valence is maintained and the substitution results in a stable compound. The term "cyclic double bond" as used herein refers to a double bond formed between two adjacent ring atoms (e.g. C=C, C=N or N=N).

Where nitrogen atoms (e.g., amines) are present on the compounds of the present disclosure, these nitrogen atoms may be converted to N-oxides by treatment with an oxidizing agent (e.g., mCPBA and/or hydrogen peroxide) to obtain other compounds of the present disclosure. Therefore, the nitrogen atoms shown and claimed are deemed to encompass the nitrogen atoms shown and their N-oxides to obtain derivatives of the present disclosure.

When any variable occurs more than once in any composition or formula of a compound, its definition in each occurrence is independent of its definition in every other occurrence. Therefore, for example, if a group is shown substituted with 0-3 Rs, the group may be optionally substituted with up to three R groups, with R defined independently in each occurrence. Furthermore, combinations of substituents and/or variables are permitted only if such combinations result in stable compounds.

The term "patient" as used herein refers to an organism to be treated by a method of the present disclosure. Such organisms preferably include, but are not limited to, mammals (e.g. rodents, apes/monkeys, horses, cattle, pigs, dogs and cats) and most preferably humans.

The term "effective amount" as used herein refers to an amount of a drug or agent (i.e., a compound of the present disclosure) that will elicit, for example, a biological or medical response in a tissue, a system, an animal or a human sought by a researcher or clinician. Furthermore, the term "therapeutically effective amount" refers to an amount that results in improved treatment, cure, prevention or alleviation of a disease, condition or side effect, or reduction in the progression rate of a disease or condition, as compared to a corresponding subject who does not receive such amount. An effective amount may be administered in one or more doses and is not intended to be limited to a particular formulation or route of administration. The term also includes an amount effective to enhance normal physiological functions within its range.

The term "treatment" as used herein includes its broad sense and encompasses both therapeutic and/or prophylactic treatment of an object. Specifically, "treatment" includes any treatment that results in the alleviation, inhibition, elimination and relief and/or prevention of a condition, disease and disorder, such as alleviation, reduction, regulation, relief, elimination, prophylaxis, prevention or remission of symptoms thereof. The therapeutic treatment includes alleviating, inhibiting or relieving symptoms or conditions of a disease; inhibiting the occurrence of complications; alleviating the underlying metabolic syndrome; inhibiting the occurrence of a disease or condition such as controlling the progression of the disease or condition; alleviating a disease or condition; reducing a disease or condition; alleviating complications caused by a disease or condition, or treating signs caused by a disease or condition. The prophylactic treatment includes a prior treatment to prevent, block or delay, slow the onset or progression of a disease or condition or reduce the severity of the disease or condition.

Similarly, the term "therapeutic agent" also includes agents or reagents used in the therapeutic and/or prophylactic treatment of an object.

The terms "pharmaceutical" or "pharmaceutically acceptable" as used herein refer to compounds, substances, compositions and/or dosage forms that, within the scope of sound medical judgment, are suitable for contact with human and animal tissues without excessive toxicity, irritation, allergic reactions and/or other issues or complications, and commensurate with a reasonable benefit/risk ratio.

Specific Pharmaceutical and Medical Terms

The term "cancer" as used herein refers to an abnormal growth of cells that is uncontrollable and, under certain conditions, capable of metastasis (spread). This type of cancer includes, but is not limited to, solid tumors [such as bladder, intestine, brain, chest, uterus, heart, kidney, lung, lymphoid tissue (lymphoma), ovary, pancreas or other endocrine organs (e.g., thyroid gland), prostate and skin (melanoma)] or blood tumors (e.g., aleukemic leukemia).

The term "combined administration" or similar terms thereof, as used herein, refers to the administration of several selected therapeutic agents to a patient in the same or different modes of administration and at the same or different times.

The term "enhancement" or "capable of enhancing" as used herein refers to an expected outcome of an increase or extension in either potency or duration. Thus, in the context of enhancing the therapeutic effect of a drug, the term "capable of enhancing" refers to the ability of the drug to increase or extend its potency or duration within the system. The term "enhancement value" as used herein refers to the ability to maximize the efficacy of another therapeutic agent in an ideal system.

The term "immune disease" refers to a disease or condition resulting from an adverse or harmful response to endogenous or exogenous antigens, typically leading to cellular dysfunction, or functional damage and impairment, or damage to organs or tissues that may produce immune symptoms.

The term "kit" is synonymous with "product packaging."

The terms "object", "subject" or "patient" include both mammals and non-mammals. Mammals include, but are not limited to, mammals including humans and non-human primates (such as orangutans, apes and monkeys), agricultural animals (such as cattle, horses, goats, sheep and pigs), domestic animals (such as rabbits and dogs), and laboratory animals including rodents (such as rats, mice and guinea pigs). Non-mammals include, but are not limited to, birds and fish. In a preferred embodiment, the selected mammal is human.

As used herein, a compound or pharmaceutical composition, after administered, can relieve a disease, symptom or condition, particularly to reduce severity, delay onset, slow down progression or shorten duration. This applies whether the administration is fixed or temporary, continuous or intermittent, and can be attributed to or associated with the administration.

### Route of Administration

Suitable routes of administration include, but are not limited to, oral, intravenous, rectal, aerosol, parenteral, ocular, pulmonary, transdermal, vaginal, ear canal, nasal and topical. In addition, by way of example only, routes of parenteral administration include intramuscular, subcutaneous, intravenous, intramedullary, ventricular, intraperitoneal, intralymphatic and intranasal.

The mode of administration of the compounds of the present disclosure invention may be topical. In certain Examples, long-acting formulations are administered by (subcutaneous or intramuscular) implantation or intramuscular injection. In another Example, administration is achieved by a targeted drug delivery system. For example, liposomes coated with organ-specific antibodies. In this Example, the liposomes are selectively directed to and absorbed by specific organs.

### Pharmaceutical Composition and Dosage

The term "pharmaceutical carrier" as used herein refers to a pharmaceutical substance, composition or vehicle, such as a liquid or solid filler, a diluent, an excipient, a manufacturing aid (e.g., lubricant, talc, magnesium stearate, calcium stearate or zinc stearate or stearic acid) or a solvent-encapsulated substance, used to carry or deliver the target compound from one organ or part of the body to another. Each carrier must be "acceptable" in the sense of being compatible with other ingredients of the formulation and harmless to patients.

The term "pharmaceutical composition" refers to a composition comprising a compound of the present disclosure together with other optional pharmaceutically acceptable carriers. The "pharmaceutical carrier" refers to a medium generally accepted in the art for delivering a biologically active agent to animals (particularly mammals), including (i.e.) adjuvants, excipients or vehicles such as diluents, preservatives, fillers, flow regulators, disintegrants, wetting agents, emulsifiers, suspending agents, sweeteners, corrigents, perfuming agents, antibacterial agents, antifungal agents, lubricants and dispersants, depending on the mode of administration and nature of the dosage form.

The pharmaceutical composition of the present disclosure may comprise a therapeutically effective amount of one or more compounds described in the present disclosure formulated with optionally one or more pharmaceutical carriers (additives) and/or diluents, and optionally one or more other therapeutic agents. The compounds of the present disclosure may be administered by any suitable means for any of the above uses, for example orally, such as in the form of tablets, pills, powders, granules, elixirs, tinctures, suspensions (including nanosuspensions, microsuspensions and spray-dried dispersions), syrups and emulsions; sublingually; buccally; parenterally, such as by subcutaneous, intravenous, intramuscular or intrasternal injection or infusion techniques (e.g. in the form of sterile injectable aqueous or non-aqueous solutions or suspensions); nasally, including administering to the nasal membrane, such as by inhalation spray; topically, such as in the form of creams or ointments; rectally, such as in the form of suppositories; or intratumorally. They may be administered alone; however, they are typically administered via a drug carrier selected based on the chosen route of administration and standard pharmaceutical practice.

Pharmaceutical carriers are formulated based on various factors known to those skilled in the art. These factors include, but are not limited to: the type and nature of the active agent formulated; the subject to whom the composition containing the active agent is to be administered; the intended route of administration of the composition; and the targeted therapeutic indication. Pharmaceutical carriers include aqueous and non-aqueous liquid media and various solid and semisolid dosage forms.

The above-mentioned carriers may comprise many different ingredients and additives in addition to the active agent. These ingredients are included in the formulation for various reasons known to those skilled in the art, such as stabilizing active agents and binding agents. Descriptions of suitable pharmaceutical carriers and factors involved in carrier selection can be obtained from several readily available sources, such as Allen L. V. Jr. et al. Remington: The Science and Practice of Pharmacy (2 Volumes), 22nd Edition (2012), Pharmaceutical Press.

Dosage regimens of the compounds of the present disclosure will, of course, vary depending on known factors such as the pharmacodynamic properties of the particular agent and its mode and route of administration; the species, age, sex, health status, medical condition and weight of the recipient; the nature and severity of symptoms; the type of concurrent treatment; the frequency of treatment; the route of administration, renal and hepatic function of the patient and expected effects. According to general guidance, the daily oral dose of each active ingredient when used for a given effect should range from about 0.001 mg/day to 10-5000 mg/day, preferably from about 0.01 mg/day to 1000 mg/day, and most preferably from about 0.1 mg/day to 250 mg/day. During constant-rate infusion, the most preferred intravenous dose should range from about 0.01 mg/kg/min to 10 mg/kg/min. The compounds of the present disclosure may be administered in a single daily dose, or divided into two, three or four doses daily.

The compounds are typically administered in the form of mixtures with suitable pharmaceutical diluents, excipients or carriers (collectively referred to herein as pharmaceutical carriers) appropriately selected according to the intended form of administration (e.g., oral tablets, capsules, elixirs and syrups) and conventional pharmaceutical practice.

A dosage form (pharmaceutical composition) suitable for administration may contain about 1 mg-2000 mg of active ingredient per dosage unit. In any of these pharmaceutical compositions, the active ingredient will generally be present in an amount of about 0.1-95% by weight based on the total weight of the composition.

The scope of the present disclosure includes pharmaceutical compositions comprising a therapeutically effective amount of at least one compound of the invention as an active ingredient, alone or in combination with a pharmaceutical carrier. Optionally, the compounds of the present disclosure may be used alone, in combination with other compounds of the present disclosure or in combination with one or more other therapeutic agents (e.g. anticancer agents or other pharmaceutically active substances).

The compounds of the present disclosure (which may be used in a suitable hydrated form) and/or the pharmaceutical compositions of the present disclosure are formulated into pharmaceutical dosage forms by conventional methods known to those skilled in the art, regardless of the selected route of administration.

The actual dose level of the active ingredient in a pharmaceutical composition of the present disclosure may be adjusted to reach an amount effective for achieving the desired therapeutic response, composition and mode of administration and ensuring nontoxicity for a particular patient.

The dosage level will be selected depending on a variety of factors, including the activity of the particular compound of the present disclosure or its ester, salt or amide; route of administration; duration of administration; the excretion rate of the particular compound; the rate and extent of absorption; duration of treatment; other medications, compounds and/or substances used in combination with the particular compound; factors well known in the medical art such as age, sex, weight, condition, general health and previous medical history of the patient to be treated.

A physician or veterinarian of ordinary skill in the art can readily determine and prescribe an effective amount of a desired pharmaceutical composition. For example, to achieve a desired therapeutic effect, a physician or veterinarian may start with a dose level lower than the desired level for any compound of the present disclosure incorporated in a pharmaceutical composition, and then gradually increase the dose level until the desired effect is achieved. Typically, a suitable daily dose for a compound of the present disclosure will be the lowest dose of the compound effective to produce a therapeutic effect. This effective dose usually depends on the factors mentioned above. Typically, oral, intravenous, intracerebroventricular and subcutaneous doses for compounds of the present disclosure range from about 0.01 to 50 mg/kg body weight per day for use in patients. If desired, an effective daily dose of the active compound may be administered in two, three, four, five, six or more sub-doses at a suitable interval throughout the day, optionally in unit dosage form. In certain Examples of the present disclosure, administration is once daily.

Although the compounds of the present disclosure may be administered alone, they are preferably administered in the form of pharmaceutical formulations (compositions).

### Kit/Product Packaging

For use in the treatment of the above indications, kit/product packaging is also described herein. A kit may consist of a delivery device, a drug pack, or a container box which may be divided into several compartments to accommodate one or more containers such as vials, tubes and the like, each containing a separate ingredient involved in a method described herein. Suitable containers include bottles, vials, syringes and test tubes. These containers are made of acceptable materials such as glass or plastic.

For example, a container may contain one or more compounds described herein, either as a pharmaceutical ingredient or in a mixture with other ingredients described herein. The container may have a sterile outlet (e.g. the container may be an intravenous infusion bag or bottle, and its stopper can be pierced by a hypodermic needle). A kit may contain a compound and a description, label or instructions for use for a method described herein.

A typical kit may comprise one or more containers, each containing one or more materials (e.g. reagents, concentrated stock solutions and/or devices) to accommodate commercial promotion and user needs for the use of the compound. These materials include, but are not limited to, buffers, diluents, filters, needles, syringes, delivery devices, bags, containers, bottles and/or test tubes, accompanied by a list of contents and/or instructions for use, and a description of built-in packaging (if any). The entire set of instructions should be included in the kit.

A label may appear on or closely associated with a container. The presence of a label on a container means that letters, numbers or other features are pasted, molded and engraved onto the container; the label may also appear inside a container box or transport box containing multiple containers, such as in a product insert. A label may be used to indicate a specific therapeutic use of the contents. The label may also carry instructions for the use of the contents, such as described in the methods above.

All features described in the specification (including any stated claim, abstract and figure) and/or all steps involved in any method or process may exist in any combination unless some features or steps are mutually exclusive in the same combination.

The above-mentioned features in the present disclosure or the features mentioned in Examples may be combined arbitrarily. All features disclosed in the specification may be used in conjunction with any composition form. Each feature disclosed in the specification may be replaced by any alternative feature that provides the same, equivalent or similar purpose. Therefore, unless otherwise specified, the features disclosed are only general examples of equivalent or similar features.

The present disclosure will be further described below in conjunction with specific Examples. It should be noted that these Examples are not intended to define the scope of the present disclosure but merely to describe the present disclosure. The experimental methods with no specific conditions indicated in the following Examples usually follow conventional conditions or the conditions recommended by manufacturers. Unless otherwise stated, all percentages, ratios, proportions or parts are measured by weight.

The unit of weight-to-volume percentage in the present disclosure is well known to those skilled in the art, for example, it refers to the weight of solute in 100 mL of solution. Unless otherwise defined, all technical and scientific terms used herein have the same meanings as those familiar to those skilled in the art. In addition, any methods and materials similar or equivalent to those described herein may be used in the methods of the present disclosure. The preferred methods and materials described herein are for exemplary purposes only.

### SPECIFIC IMPLEMENTATIONS

The present disclosure is further described through the following specific Examples, which are not intended to limit the scope of the present disclosure to these Examples. Experimental methods with no specific conditions indicated in the following Examples should be selected according to conventional methods and conditions, or commercial specifications.

NMR was measured using Bruker AVANCE-400 NMR spectrometer. Solvents used for measurement were indicated in the spectrum interpretation.

MS measurement was conducted using Agilent 1200-G1956A/1200-6110A/1200-6140A/1260-6125B/Prime-6125B/1260-6120 liquid chromatograph-mass spectrometer (LC/MS), SHIMADZU 20A-2010/20A-2020 LC/MS and Waters ACQ-QDA LC/MS.

HPLC analysis was performed using a SHIMADZU 20A high performance liquid chromatograph.

SFC analysis and determination were conducted using Waters UPCC with PDA Detector and QDa Detector ultra-high performance convergence chromatograph, Waters UPC² with PDA detector ultra-high performance convergence chromatograph, Agilent 1260 with DAD detector high-performance liquid chromatograph, Shimadzu LC-20AB with PDA detector high-performance liquid chromatograph and Shimadzu LC-20AD with PDA detector high-performance liquid chromatograph.

Preparative HPLC separation was conducted using Shimadzu LC-20AP pump, Shimadzu LH-40 Liquid Handler, Shimadzu SPD-20A Detector, Gilson GX-281 Liquid Handler, Gilson 322 pump and Gilson 156 UV Detector preparative chromatographs.

SFC separations were conducted using The Berger MG II, MG III, Sepiatec's Prep SFC 100 System, Waters Prep 80Q SFC SYSTEM, Prep 150 AP SFC SYSTEM, Prep 200 SFC SYSTEM, and Prep 350 SFC SYSTEM.

Flash column chromatography separation was conducted using Biotage IsoleraOne flash-preparative chromatograph.

TLC plates used were GF254 acrylic adhesive silica gel plates provided by Anhui Liangchen Silica Source Materials Co., Ltd. The TLC silica gel plates used for analysis were 0.25 mm in thickness, while those used for product purification were 0.5 mm in thickness.

Pressurized hydrogenation reactions utilized hydrogenation bottles and hydrogen gas cylinders.

Microwave reactions were performed using a Biotage Initiator+ microwave synthesizer.

### A custom glove box from DELLIX was used.

The present disclosure is further described through the following Examples, which are not intended to impose any limitations on the present disclosure. The compounds of the present disclosure can be prepared by a variety of synthetic methods known to those skilled in the art, including the specific Examples listed below, Examples formed by their combination with other chemical synthesis methods and equivalent alternatives well known to those skilled in the art. Preferred Examples include, but are not limited to, the Examples of the present disclosure. It will be apparent to those skilled in the art that various changes and improvements can be made to specific Examples of the present disclosure without departing from the spirit and scope of the present disclosure.

### Example 1:

### 2-Hydroxy-N-(2⁶-methyl-4-oxo-3-aza-2(2,4)-pyrimidin-1(1,3),6(1,4)-dipiperidin-5 (1,2)-benzeneheterocyclonon-8-en-5⁴-yl)ethane-1-sulfonamide

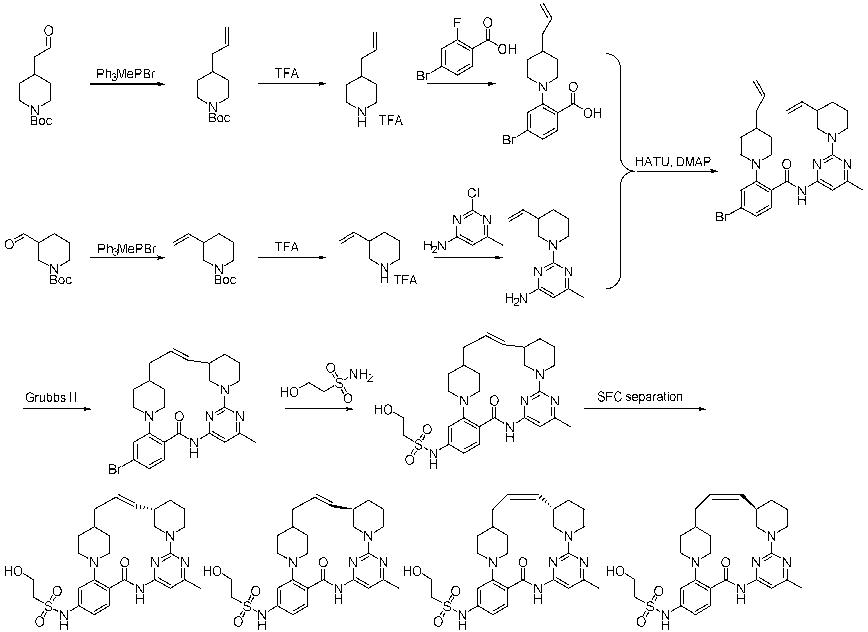

**Step 1:** Under nitrogen atmosphere at 20°C, methyltriphenylphosphonium bromide (24.06 g, 66.00 mmol) was dissolved in dry tetrahydrofuran (350.00 mL), the mixture was cooled to 0°C, and then a 1.0 M lithium bis(trimethylsilyl)amide solution in tetrahydrofuran (66.00 mL, 66.00 mmol) was slowly added and stirred for 1 hour. To the reaction system was added a solution of N-Boc-4-piperidineacetaldehyde (10.00 g, 44.00 mmol) in tetrahydrofuran (20.00 mL). The temperature was raised to 20°C and the mixture was stirred for 16 hours. After the reaction was complete, the reaction was quenched with a saturated sodium bicarbonate solution (100 mL) and the mixture was extracted with ethyl acetate (200 mL × 3). The combined organic layer was dried, filtered and concentrated to give a crude product, which was purified by flash column chromatography (silica, 0-10% ethyl acetate/petroleum ether) to give tert-butyl 4-allylpiperidine-1-carboxylate (10.00 g, 66.00 mmol, yield: 100.0%) as a colorless liquid.

**Step 2:** At 20°C, tert-butyl 4-allylpiperidine-1-carboxylate (10.00 g, 66.00 mmol) was dissolved in dichloromethane (200.00 mL), then trifluoroacetic acid (10.02 mL, 131.81 mmol) was added and the mixture was stirred at 20°C for 16 hours. After the reaction was complete, the solvent and excess trifluoroacetic acid were removed to give 4-allylpiperidine trifluoroacetate (about 15.50 g, crude). The crude product was then dissolved in dimethyl sulfoxide (200.00 mL), then potassium carbonate (30.98 g, 219.68 mmol) and 4-bromo-2-fluorobenzoic acid (9.62 g, 43.94 mmol) were added and the mixture was heated to 140°C and stirred for 48 hours. After the reaction was complete, the mixture was cooled naturally, then the reaction was quenched with cold water (100 mL), and 1*N* hydrochloric acid was added to adjust the pH of the solution to 6-7. The mixture was extracted with ethyl acetate (200 mL × 3). The combined organic layer was washed with saturated sodium chloride solution (100 mL), then dried, filtered and concentrated to give a crude product, which was purified by flash column chromatography (silica, 0-20% ethyl acetate/petroleum ether) to give 4-bromo-2-(4-allylpiperidinyl)benzoic acid (11.50 g, 35.15 mmol, yield: 80.3%) as an orange solid. ¹H NMR (400 MHz, Chloroform-d) δ 8.14 (d, *J* = 8.3 Hz, 1H), 7.61 - 7.48 (m, 2H), 5.85 - 5.68 (m, 1H), 5.14 - 4.98 (m, 2H), 3.19 - 3.05 (m, 2H), 2.91 (td, *J=* 11.7, 2.5 Hz, 2H), 2.11 (t, *J=* 6.8 Hz, 2H), 2.07 - 1.99 (m, 1H), 2.00 - 1.86 (m, 2H), 1.67 - 1.44 (m, 3H) ppm; LCMS (ESI): [M+H]⁺ =324.1.

**Step 3:** Under nitrogen atmosphere at 20°C, methyltriphenylphosphonium bromide (25.64 g, 70.33 mmol) was dissolved in dry tetrahydrofuran (350.00 mL), the mixture was cooled to 0°C, and then a 1.0 M lithium bis(trimethylsilyl)amide solution in tetrahydrofuran (70.33 mL, 70.33 mmol) was slowly added and stirred for 1 hour. To the reaction system was added a solution of tert-butyl 3-formylpiperidine-1-carboxylate (10.00 g, 46.89 mmol) in tetrahydrofuran (20.00 mL). The temperature was raised to 20°C and the mixture was stirred for 16 hours. After the reaction was complete, the reaction was quenched with a saturated sodium bicarbonate solution (100 mL) and the mixture was extracted with ethyl acetate (200 mL × 3). The combined organic layer was dried, filtered and concentrated to give a crude product, which was purified by flash column chromatography (silica, 0-10% ethyl acetate/petroleum ether) to give 3-vinylpiperidin-1-carboxylate (10.00 g, 70.33 mmol, yield: 100.0%) as a colorless liquid.

**Step 4:** At 20°C, 3-vinylpiperidin-1-carboxylate (2.94 g, 13.93 mmol) was dissolved in dichloromethane (100.00 mL), then trifluoroacetic acid (5.01 mL, 65.70 mmol) was added and the mixture was stirred at 20°C for 16 hours. After the reaction was complete, the solvent and excess trifluoroacetic acid were removed to give 3-vinylpiperidin trifluoroacetate (about 5.30 g, crude). The crude product was then dissolved in *N*,*N*-dimethylformamide (100.00 mL), then potassium carbonate (9.82 g, 69.65 mmol) and 2-chloro-6-methylpyrimidin-4-amine (9.62 g, 43.94 mmol) were added and the mixture was heated to 130°C and stirred for 48 hours. After the reaction was complete, the mixture was cooled naturally, and the reaction was quenched with cold water (200 mL). The mixture was extracted with ethyl acetate (200 mL × 3). The combined organic layer was washed with saturated sodium chloride solution (100 mL), then dried, filtered and concentrated to give a crude product, which was purified by flash column chromatography (silica, 0-20% ethyl acetate/petroleum ether) to give 6-methyl-2-(3-vinylpiperidin)pyrimidin-4-amine (2.31 g, 10.73 mmol, yield: 77.0%) as an orange liquid. ¹H NMR (400 MHz, Chloroform-d) δ 5.79 (ddd, *J =* 17.2, 10.6, 6.5 Hz, 1H), 5.60 (s, 1H), δ 5.09 (dt, *J =* 17.4, 1.6 Hz, 1H), 5.02 (dt, *J* = 10.5, 1.5 Hz, 1H), 4.75 - 4.59 (m, 2H), 4.53 (brs, 2H), 2.78 (ddd, *J* = 13.0, 11.8, 3.0 Hz, 1H), 2.62 (dd, *J* = 12.9, 10.6 Hz, 1H), 2.25 - 2.12 (m, 1H), 2.19 (s, 3H), 1.95 - 1.83 (m, 1H), 1.77 - 1.67 (m, 1H), 1.61 - 1.44 (m, 1H), 1.42 - 1.28 (m, 1H); LCMS (ESI): [M+H]⁺ =219.2.

**Step 5:** At 20°C, 6-methyl-2-(3-vinylpiperidin)pyrimidin-4-amine (0.67 g, 3.08 mmol), 4-bromo-2-(4-allylpiperidinyl)benzoic acid (1.00 g, 3.08 mmol), N,N,N',N'-tetramethyl-O-(7-azabenzotriazol-1-yl)uronium hexafluorophosphate (2.99 g, 7.71 mmol) and 4-dimethylaminopyridine (1.88 g, 15.42 mmol) were dissolved in dry *N*,*N*-dimethylformamide (20.00 mL). The mixture was heated to 60°C and stirred for 24 hours. After the reaction was complete, the mixture was cooled naturally, and the reaction was quenched with cold water (50 mL). The mixture was extracted with ethyl acetate (50 mL × 3). The combined organic layer was washed with saturated sodium chloride solution (20 mL), then dried, filtered and concentrated to give a crude product, which was purified by flash column chromatography (silica, 0-10% ethyl acetate/petroleum ether) to give 2-(4-allylpiperidin-1-yl)-4-bromo-*N*-(6-methyl-2-(3-vinylpiperidin-1-yl)pyrimidin-4-y l)benzamide (0.88 g, 1.67 mmol, yield: 54.4%) as a yellow liquid, LCMS (ESI): [M+H]⁺ =524.3.

**Step 6:** At 20°C, 2-(4-allylpiperidin-1-yl)-4-bromo-*N*-(6-methyl-2-(3-vinylpiperidin-1-yl)pyrimidin-4-y l)benzamide (500 mg, 0.76 mmol) was dissolved in dry dichloromethane (40.00 mL), the mixture was purged with nitrogen for 15 minutes, Grubbs catalyst 2^{nd} generation (33 mg, 0.038 mmol) was added and the reaction was heated to reflux for 16 hours. After the reaction was complete, the concentrated reaction solution was purified by flash column chromatography (silica, 0-10% ethyl acetate/petroleum ether) to give 5⁴-bromo-2⁶-methyl-3-aza-2(2,4)-pyrimidin-1(1,3),6(1,4)-dipiperidin-5(1,2)-benzeneh eterocyclonon-8-en-4-one (350 mg, 0.56 mmol, yield: 74.0%) as a white solid, LCMS (ESI): [M+H]⁺ =496.3.

**Step 7:** At 20°C, 5⁴-bromo-2⁶-methyl-3-aza-2(2,4)-pyrimidin-1(1,3),6(1,4)-dipiperidin-5(1,2)-benzeneh eterocyclonon-8-en-4-one (200 mg, 0.40 mmol), 2-hydroxyethylsulfonamide (103 mg, 0.81 mmol), potassium phosphate (262 mg, 1.21 mmol), tris(dibenzylideneacetone)dipalladium (38 mg, 0.04 mmol) and 2-di-tert-butylphosphino-2',4',6'-triisopropylbiphenyl (17 mg, 0.04 mmol) were dissolved in 1,4-dioxane (5.00 mL). The resulting reaction mixture was purged with nitrogen for 15 minutes and stirred at 100°C for 5 hours. After the reaction was complete, the reaction was quenched with cold water (20 mL). The mixture was extracted with ethyl acetate (20 mL × 3). The combined organic layer was dried, filtered and concentrated to give a crude product, which was purified by flash column chromatography (silica, 30-95% ethyl acetate/petroleum ether) to give 170 mg of the product. The product was further purified by preparative HPLC (C18, 70-95% gradient of acetonitrile/water) to give 2-hydroxy-*N*-(2⁶-methyl-4-oxo-3-aza-2(2,4)-pyrimidin-1(1,3),6(1,4)-dipiperidin-5(1,2) -benzeneheterocyclonon-8-en-5⁴-yl)ethane-1-sulfonamide (149 mg, 0.27 mmol, yield: 68.4%), Z/E=3:7, as a white solid. ¹H NMR (400 MHz, DMSO-*d*₆) δ 12.90 (s, 0.3H), 10.73 (s, 0.7H), 10.19 (brs, 1H), 8.05 (d, *J* = 8.6 Hz, 0.3H), 7.81 (d, *J =* 8.5 Hz, 0.7H), 7.32 (s, 0.7H), 7.22 (d, *J =* 2.2 Hz, 0.3H), 7.17 - 7.11 (m, 0.6H), 7.05 (d, *J =* 1.4 Hz, 0.7H), 7.00 (dd, *J* = 8.6, 2.0 Hz, 0.7H), 5.73 - 5.53 (m, 1.4H), 5.48 (ddd, *J* = 11.2, 11.1, 5.3 Hz, 0.3H), 5.35 (dd, *J* = 10.1, 10.1 Hz, 0.3H), 4.94 (brs, 1H), 4.85 (d, *J* = 13.0 Hz, 0.7H), 4.68 (d, *J =* 12.7 Hz, 0.7H), 4.60 (d, *J =* 13.1 Hz, 0.3H), 4.45 (d, *J =* 12.6 Hz, 0.3H), 3.76 (t, *J* = 6.5 Hz, 2H), 3.38 - 3.34 (m, 1.7H), 3.27 (d, *J* = 10.9 Hz, 0.7H), 3.16 (d, *J* = 11.0 Hz, 0.3H), 3.09 - 2.87 (m, 2H), 2.84 - 2.62 (m, 2.3H), 2.56 - 2.44 (m, 2H), 2.31 - 2.20 (m, 4H), 2.19 - 2.08 (m, 0.6H), 1.91 - 1.36 (m, 8.7H), 1.24 (d, *J =* 11.7 Hz, 0.7H) ppm; LCMS (ESI): [M+H]⁺ =541.4.

### Step 8: Example 1

(E)-2-hydroxy-N-(2⁶-methyl-4-oxo-3-aza-2(2,4)-pyrimidin-1(1,3),6(1,4)-dipiperidin-5 (1,2)-benzoaza-8-en--4-yl)ethane-1-sulfonamide (650 mg, 1.20 mmol) was separated by SFC (AD, carbon dioxide-ethanol (0.1% aqueous ammonia)) to give four corresponding isomers: **Example 1A to Example 1D.**

### Example 1A:

**2-Hydroxy-N-((1³S,E)-2⁶-methyl-4-oxo-3-aza-2(2,4)-pyrimidin-1(1,3),6(1,4)-dipip eridin-5(1,2)-benzoaza-8-en-5⁴-yl)ethane-1-sulfonamide.** LCMS (ESI): [M+H]⁺ = 541.2. ¹H NMR (400 MHz, DMSO-*d*₆) δ ppm 10.74 (s, 1H), 10.49 - 9.58 (m, 1H), 7.81 (d, *J =* 8.4 Hz, 1H), 7.32 (s, 1H), 7.14 - 6.89 (m, 2H), 5.76 - 5.51 (m, 2H), 4.85 (br d, *J =* 12.4 Hz, 2H), 4.68 (br d, *J =* 12.8 Hz, 1H), 3.75 (t, *J* = 6.4 Hz, 2H), 3.41 - 3.34 (m, 2H), 3.28 (br d, *J =* 10.8 Hz, 1H), 3.11 - 2.92 (m, 2H), 2.87 - 2.66 (m, 2H), 2.58 - 2.52 (m, 1H), 2.48 - 2.45 (m, 1H), 2.32 - 2.17 (m, 4H), 1.95 - 1.82 (m, 1H), 1.81 - 1.50 (m, 8H), 1.32 - 1.18 (m, 1H)

### Example 1B:

**2-Hydroxy-N-((1³R,E)-2⁶-methyl-4-oxo-3-aza-2(2,4)-pyrimidin-1(1,3),6(1,4)-dipip eridin-5(1,2)-benzoaza-8-en-5⁴-yl)ethane-1-sulfonamide.** LCMS (ESI): [M+H]⁺ = 541.2. ¹H NMR (400 MHz, DMSO-*d*₆) δ ppm 10.74 (s, 1H), 10.17 (br d, *J =* 1.0 Hz, 1H), 7.81 (d, *J* = 8.4 Hz, 1H), 7.32 (s, 1H), 7.14 - 6.92 (m, 2H), 5.75 - 5.51 (m, 2H), 5.05 - 4.89 (m, 1H), 4.85 (br d, *J* = 13.2 Hz, 1H), 4.68 (br d, *J* = 12.4 Hz, 1H), 3.75 (br t, *J =* 6.4 Hz, 2H), 3.42 - 3.34 (m, 2H), 3.28 (br d, *J* = 9.2 Hz, 1H), 3.11 - 2.94 (m, 2H), 2.87 - 2.66 (m, 2H), 2.54 (br s, 1H), 2.48 - 2.43 (m, 1H), 2.31 - 2.20 (m, 4H), 1.95 - 1.82 (m, 1H), 1.82 - 1.50 (m, 8H), 1.29 - 1.18 (m, 1H)

### Example 1C:

**2-Hydroxy-N-((1³S,Z)-2⁶-methyl-4-oxo-3-aza-2(2,4)-pyrimidin-1(1,3),6(1,4)-dipip eridin-5(1,2)-benzoaza-8-en-5⁴-yl)ethane-1-sulfonamide.** LCMS (ESI): [M+H]⁺ = 541.2. ¹H NMR (400 MHz, DMSO-*d*₆) δ ppm 13.55 - 13.15 (m, 1H), 10.33 (s, 1H), 8.06 (d, *J =* 8.8 Hz, 1H), 7.31 (br s, 1H), 7.25 (d, *J* = 2.0 Hz, 1H), 7.17 (dd, *J* = 2.0, 8.8 Hz, 1H), 5.60 - 5.43 (m, 1H), 5.42 - 5.28 (m, 1H), 4.62 - 4.40 (m, 2H), 3.76 (t, *J* = 6.4 Hz, 2H), 3.37 (br s, 2H), 3.18 (br s, 1H), 3.07 - 2.96 (m, 2H), 2.88 - 2.71 (m, 3H), 2.39 (s, 3H), 2.32 - 2.23 (m, 1H), 2.19 - 1.98 (m, 2H), 1.91 - 1.69 (m, 6H), 1.62 - 1.42 (m, 3H)

### Example 1D:

**2-Hydroxy-N-((1³R,Z)-2⁶-methyl-4-oxo-3-aza-2(2,4)-pyrimidin-1(1,3),6(1,4)-dipip eridin-5(1,2)-benzoaza-8-en-5⁴-yl)ethane-1-sulfonamide.** LCMS (ESI): [M+H]⁺ = 541.2. ¹H NMR (400 MHz, DMSO-*d*₆) δ ppm 12.90 (br s, 1H), 10.58 - 9.89 (m, 1H), 8.05 (d, *J* = 8.4 Hz, 1H), 7.22 (d, *J* = 2.0 Hz, 1H), 7.18 - 7.08 (m, 2H), 5.48 (dt, *J* = 5.2, 11.2 Hz, 1H), 5.41 - 5.30 (m, 1H), 5.08 - 4.82 (m, 1H), 4.60 (br d, *J* = 12.8 Hz, 1H), 4.45 (br d, *J* = 12.4 Hz, 1H), 3.76 (t, *J* = 6.4 Hz, 2H), 3.42 - 3.34 (m, 2H), 3.16 (br d, *J* = 10.8 Hz, 1H), 3.03 - 2.87 (m, 2H), 2.77 (q, *J =* 12.4 Hz, 2H), 2.70 - 2.61 (m, 1H), 2.36 - 2.20 (m, 4H), 2.19 - 2.07 (m, 2H), 1.91 - 1.37 (m, 9H)

### Example 2:

### (Z)-2-Hydroxy-N-(2⁶-methyl-4-oxo-3-aza-2(2,4)-pyrimidin-5(2,3)-pyridin-1(1,3),6 (1,4)-dipiperidinylcyclononane-8-en-55-yl)ethane-1-sulfonamide

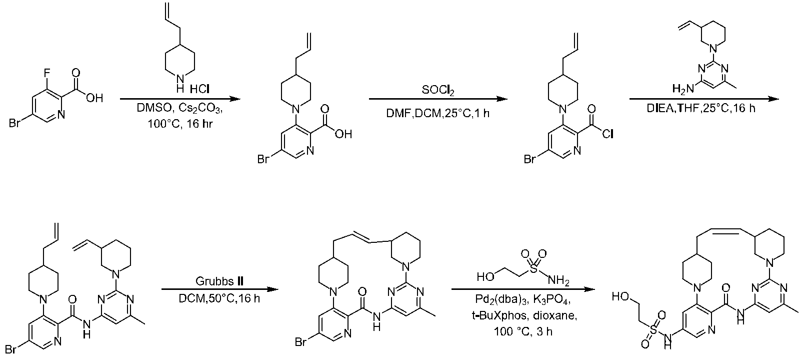

**Step 1:** To a solution of 5-bromo-3-fluoropicolinic acid (2.00 g, 9.09 mmol) and cesium carbonate (9.07 g, 27.3 mmol) in dimethyl sulfoxide (40 mL) was added 4-(prop-2-en-1-yl)piperidine hydrochloride (1.91 g, 11.8 mmol). The mixture was stirred at 100°C for 16 hours. The reaction mixture was poured into water (400 mL) and the pH was adjusted to 2-3 with 1N dilute hydrochloric acid. The mixture was extracted with ethyl acetate (400 mL × 2), and the combined organic layer was washed with saturated saline (200 mL), dried over anhydrous magnesium sulfate, filtered, and concentrated under reduced pressure to give the residue. The residue was purified by flash column chromatography (silica gel, 0-5% gradient of dichloromethane/methanol) to give 5-bromo-3-[4-(prop-2-en-1-yl)piperidin-1-yl]pyridine-2-carboxylic acid (2.90 g, 26.8 mmol, yield: 98%) as a brown solid compound. LCMS (ESI): [M+H]⁺ = 327.2. ¹H NMR (400 MHz, DMSO-d6) δ ppm 8.66 (s, 1H), 7.90 (d, *J =* 1.2 Hz, 1H), 5.85 - 5.69 (m, 1H), 5.07 (br d, *J* = 5.2 Hz, 1H), 5.03 (s, 1H), 3.21 (br d, *J* = 11.6 Hz, 2H), 2.90 (br t, *J =* 11.6 Hz, 2H), 2.10 (br t, *J* = 6.4 Hz, 2H), 1.94 (br d, *J* = 11.2 Hz, 2H), 1.64 - 1.41 (m, 3H)

**Step 2:** At 25°C, to a solution of 5-bromo-3-[4-(prop-2-en-1-yl)piperidin-1-yl]pyridine-2-carboxylic acid (1.00 g, 3.08 mmol) in dichloromethane (20.0 mL) was added thionyl chloride (0.55 g, 4.61 mmol). The reaction mixture was stirred at 25°C for 1 hour. The reaction solution was used directly in the next step without further workup or purification. LCMS (ESI): [M+H]⁺ = 341.2.

**Step 3:** At 25°C, to a solution of 5-bromo-3-[4-(prop-2-en-1-yl)piperidin-1-yl]pyridine-2-carbonyl chloride (1.00 g, 2.91 mmol) in dichloromethane (20 mL) was added 2-(3-vinylpiperidin-1-yl)-6-methylpyrimidin-4-amine (640 mg, 2.91 mmol). N,N-diisopropylethylamine (2.05 g, 15.9 mmol) and a solution of potassium tert-butoxide in tetrahydrofuran (11.6 mL, 11.6 mmol) were added. The reaction mixture was stirred at 25°C for 16 hours. The reaction mixture was diluted with water (100 mL) and extracted with ethyl acetate (100 mL × 2). The combined organic layer was dried over anhydrous magnesium sulfate, filtered and concentrated under reduced pressure to give a residue. The residue was purified by flash column chromatography (silica gel, 0-9% gradient of tetrahydrofuran/petroleum ether) to give 5-bromo-N-[2-(3-vinylpiperidin-1-yl)-6-methylpyrimidin-4-yl]-3-[4-(prop-2-en-1-yl) piperidin-1-yl]pyridine-2-carboxamide (0.59 g, 1.13 mmol, yield: 39%) as a brown solid compound. LCMS (ESI): [M+H]⁺ = 525.3

**Step 4:** At 20°C, 5-bromo-N-[2-(3-vinylpiperidin-1-yl)-6-methylpyrimidin-4-yl]-3-[4-(prop-2-en-1-yl) piperidin-1-yl]pyridine-2-carboxamide (560 mg, 1.07 mmol) was dissolved in dry dichloromethane (40.0 mL), the mixture was purged with nitrogen for 15 minutes, Grubbs catalyst 2^{nd} generation(462 mg, 0.53 mmol) was added and the reaction was heated to reflux at 50°C for 16 hours. After the reaction was complete, the concentrated reaction solution was purified by flash column chromatography (silica, 0-10% tetrahydrofuran/petroleum ether) to give (Z)-5⁵-bromo-2⁶-methyl-3-aza-2(2,4)-pyrimidin-5(2,3)-pyridin-1(1,3),6(1,4)-dipiperid inylcyclononane-8-en-4-one as a yellow solid. LCMS (ESI): [M+H]⁺ = 497.0.

**Step 5:** To a solution of (Z)-5⁵-bromo-2⁶-methyl-3-aza-2(2,4)-pyrimidin-5(2,3)-pyridin-1(1,3),6(1,4)-dipiperid inylcyclononane-8-en-4-one (20 mg, 0.04 mmol), 2-hydroxyethanesulfonamide (5.03 mg, 0.04 mmol), di-tert-butyl-[2-(2,4,6-triisopropylphenyl)phenyl]phosphane (3.41 mg, 0.008 mmol), and potassium phosphate (26.1 mg, 0.12 mmol) in 1,4-dioxane (0.50 mL) was added tris(dibenzylideneacetone)dipalladium (3.76 mg, 0.004 mmol). The nitrogen replacement was performed for 3 times. The mixture was heated to 100°C and stirred for 3 h. The reaction mixture was diluted with water (5 mL) and extracted with ethyl acetate (10 mL × 2). The organic phases were combined, dried over anhydrous sodium sulfate, and filtered, and the filtrate was concentrated to give the residue. The residue was purified by preparative HPLC (C18, 12-52% gradient of water (formic acid)/acetonitrile) to give (Z)-2-hydroxy-N-(26-methyl-4-oxo-3-aza-2(2,4)-pyrimidin-5(2,3)-pyridin-1(1,3),6(1, 4)-dipiperidinylcyclononane-8-en-55-yl)ethane-1-sulfonamide (2.13 mg, 0.004 mmol, yield: 10%) as a white solid compound. LCMS (ESI): [M+H]⁺ = 542.3. ¹H NMR (400 MHz, DMSO-d6) δ ppm 10.28 (s, 1H), 8.09 (d, *J* = 2.0 Hz, 1H), 7.36 - 7.26 (m, 2H), 5.71 - 5.48 (m, 2H), 5.24 - 4.95 (m, 1H), 4.88 (br d, *J* = 13.2 Hz, 1H), 4.75 (br d, *J* = 12.8 Hz, 1H), 3.76 (t, *J =* 6.4 Hz, 2H), 3.28 - 3.21 (m, 2H), 3.05 - 3.00 (m, 2H), 2.81 - 2.71 (m, 2H), 2.63 - 2.55 (m, 1H), 2.49 - 2.39 (m, 2H), 2.28 (s, 3H), 2.24 - 2.17 (m, 1H), 1.92 - 1.45 (m, 10H), 1.19 (br d, *J =* 11.6 Hz, 1H)

### Example 3:

### 2-Hydroxy-N-(2⁶-methyl-4-oxo-3-aza-2(2,4)-pyrimidin-1(1,3),6(1,4)-dipiperidin-5 (1,2)-benzeneheterocyclononane-5⁴-yl)ethane-1-sulfonamide

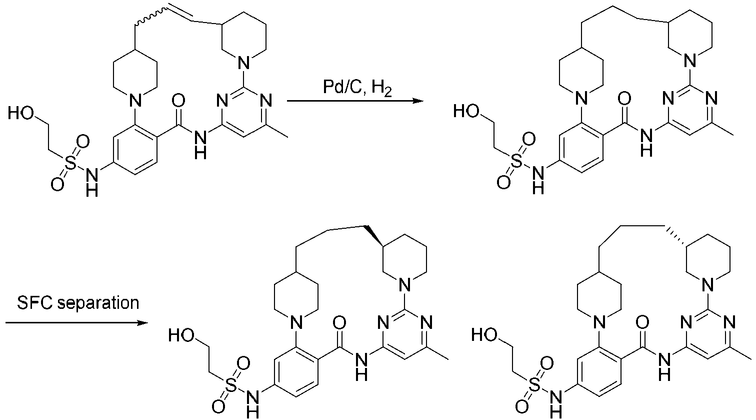

**Step 1:** At 20°C, 2-hydroxy-N-(2⁶-methyl-4-oxo-3-aza-2(2,4)-pyrimidin-1(1,3),6(1,4)-dipiperidin-5(1,2) -benzeneheterocyclonon-8-en-5⁴-yl)ethane-1-sulfonamide (70 mg, 0.13 mmol) and 10% palladium/carbon (70 mg, 100% w/w) were dissolved in methanol (5.00 mL), and acetic acid (0.10 g, 1.67 mmol) was added. The mixture was purged with hydrogen and stirred at 20°C for 24 hours to complete the reaction. The mixture was filtered through celite, rinsed with dichloromethane (50 mL), then concentrated and purified by flash column chromatography (silica, 30-95% ethyl acetate/petroleum ether) to give 70 mg of the product. The product was further purified by preparative HPLC (C18, 50-70% gradient of acetonitrile/water) to give 2-hydroxy-*N*-(2⁶-methyl-4-oxo-3-aza-2(2,4)-pyrimidin-1(1,3),6(1,4)-dipiperidin-5(1,2) -benzeneheterocyclononane-5⁴-yl)ethane-1-sulfonamide (55 mg, 0.10 mmol, yield: 78.6%) as a white solid. LCMS (ESI): [M+H]⁺ =543.4. ¹H NMR (400 MHz, DMSO-*d*₆) δ 11.69 (s, 1H), 10.19 (s, 1H), 7.96 (d, *J =* 8.6 Hz, 1H), 7.33 (s, 1H), 7.11 (d, *J* = 2.2 Hz, 1H), 7.05 (dd, *J =* 8.6, 2.1 Hz, 1H), 4.93 (brs, 1H), 4.13 (d, *J =* 12.8 Hz, 1H), 3.95 (d, *J* = 12.7 Hz, 1H), 3.74 (t, *J* = 6.5 Hz, 2H), 3.52 - 3.43 (m, 1H), 3.24 - 3.00 (m, 3H), 2.77 - 2.61 (m, 2H), 2.24 (s, 3H), 2.07 - 1.82 (m, 2H), 1.75 - 1.15 (m, 16H) ppm.

### Step 2:

2-Hydroxy-N-(2⁶-methyl-4-oxo-3-aza-2(2,4)-pyrimidin-1(1,3),6(1,4)-dipiperidin-5(1, 2)-benzoazacyclononyl-5⁴-yl)ethane-1-sulfonamide (173 mg, 0.32 mmol) was separated by SFC (AD, carbon dioxide-ethanol (0.1% aqueous ammonia)) to give 2 corresponding isomers: **Example 3A and Example 3B.**

### Example 3A:

**2-Hydroxy-N-((1³R)-2⁶-methyl-4-oxo-3-aza-2(2,4)-pyrimidin-1(1,3),6(1,4)-dipiper idin-5(1,2)-benzeneheterocyclononane-5⁴-yl)ethane-1-sulfonamide.** LCMS (ESI): [M+H]⁺ = 543.3. ¹H NMR (400 MHz, DMSO-*d*₆) δ ppm 11.72 (s, 1H), 10.22 (s, 1H), 7.98 (d, *J =* 8.4 Hz, 1H), 7.36 (s, 1H), 7.13 (d, *J* = 2.0 Hz, 1H), 7.07 (dd, *J =* 2.0, 8.8 Hz, 1H), 5.08 - 4.75 (m, 1H), 4.15 (br d, *J* = 11.2 Hz, 1H), 4.04 - 3.88 (m, 1H), 3.76 (br t, *J =* 6.4 Hz, 2H), 3.59 - 3.47 (m, 1H), 3.41 - 3.35 (m, 2H), 3.28 - 3.20 (m, 1H), 3.19 - 3.06 (m, 2H), 2.80 - 2.65 (m, 2H), 2.27 (s, 3H), 2.12 - 1.81 (m, 2H), 1.78 - 1.22 (m, 14H)

### Example 3B:

**2-Hydroxy-N-((1³S)-2⁶-methyl-4-oxo-3-aza-2(2,4)-pyrimidin-1(1,3),6(1,4)-dipiperi din-5(1,2)-benzeneheterocyclononane-5⁴-yl)ethane-1-sulfonamide.** LCMS (ESI): [M+H]⁺ = 543.3. ¹H NMR (400 MHz, DMSO-*d*₆) δ ppm 11.71 (s, 1H), 10.21 (br s, 1H), 7.97 (d, *J =* 8.8 Hz, 1H), 7.34 (s, 1H), 7.12 (d, *J* = 2.0 Hz, 1H), 7.06 (dd, *J* = 2.0, 8.8 Hz, 1H), 4.93 (br t, *J =* 5.6 Hz, 1H), 4.14 (br d, *J =* 10.4 Hz, 1H), 4.03 - 3.91 (m, 1H), 3.75 (q, *J =* 6.0 Hz, 2H), 3.56 - 3.46 (m, 1H), 3.39 - 3.34 (m, 2H), 3.27 - 3.19 (m, 1H), 3.16 - 3.05 (m, 2H), 2.77 - 2.65 (m, 2H), 2.26 (s, 3H), 2.08 - 1.82 (m, 2H), 1.78 - 1.31 (m, 14H)

### Example 4:

### 2-Hydroxy-N-(1¹-methyl-7-oxo-8-aza-1(2,4)-pyrimidin-2(1,3),5(4,1)-dipiperidin-6 (1,2)-benzeneheterocyclooct-3-en-6⁵-yl)ethane-1-sulfonamide

### Example 5:

### 2-Hydroxy-N-(1¹-methyl-7-oxo-8-aza-1(2,4)-pyrimidin-2(1,3),5(4,1)-dipiperidin-6 (1,2)-benzeneheterocyclooct-6⁵-yl)ethane-1-sulfonamide

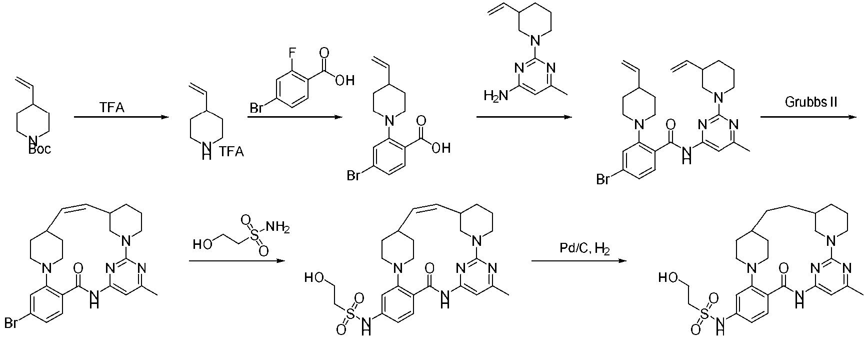

**Step 1:** At 20°C, tert-butyl 4-vinylpiperidin-1-carboxylate (5.00 g, 23.66 mmol) was dissolved in dichloromethane (50.00 mL), then trifluoroacetic acid (10.02 mL, 131.81 mmol) was added and the mixture was stirred at 20°C for 16 hours. After the reaction was complete, the solvent and excess trifluoroacetic acid were removed to give 4-allylpiperidine trifluoroacetate (about 10.50 g, crude). The crude product was then dissolved in dimethyl sulfoxide (50.00 mL), then potassium carbonate (16.69 g, 118.32 mmol) and 4-bromo-2-fluorobenzoic acid (5.18 g, 23.66 mmol) were added and the mixture was heated to 140°C and stirred for 48 hours. After the reaction was complete, the mixture was cooled naturally, then the reaction was quenched with cold water (100 mL), and 1*N* hydrochloric acid was added to adjust the pH of the solution to 6-7. The mixture was extracted with ethyl acetate (100 mL × 3). The combined organic layer was washed with sodium bicarbonate solution (100 mL × 2), then dried, filtered and concentrated to give a crude product, which was purified by flash column chromatography (silica, 0-20% ethyl acetate/petroleum ether) to give 4-bromo-2-(4-vinylpiperidin)benzoic acid (4.77 g, 15.42 mmol, yield: 65.2%) as an orange solid. LCMS (ESI): [M+H]⁺ =310.1. ¹H NMR (400 MHz, Chloroform-d) δ 8.16 (d, *J* = 8.2 Hz, 1H), 7.59 - 7.50 (m, 2H), 5.84 (ddd, *J* = 17.0, 10.5, 6.2 Hz, 1H), 5.10 (dt, *J =* 17.3, 2.2 Hz, 1H), 5.07 (d, *J =* 9.7 Hz, 1H), 3.16 (dt, *J =* 12.0, 3.7 Hz, 2H), 2.96 (td, *J =* 11.8, 2.7 Hz, 2H), 2.26 (s, 1H), 2.08 - 1.89 (m, 2H), 1.80 - 1.60 (m, 2H) ppm.

**Step 2:** At 20°C, 6-methyl-2-(3-vinylpiperidin)pyrimidin-4-amine (0.70 g, 3.22 mmol), 4-bromo-2-(4-vinylpiperidinyl)benzoic acid (1.00 g, 3.22 mmol), N,N,N',N' -tetramethyl-O-(7-azabenzotriazol-1-yl)uronium hexafluorophosphate (3.13 g, 8.06 mmol) and 4-dimethylaminopyridine (1.21 g, 9.67 mmol) were dissolved in dry *N*,*N*-dimethylformamide (20.00 mL). The mixture was heated to 60°C and stirred for 24 hours. After the reaction was complete, the mixture was cooled naturally, and the reaction was quenched with cold water (50 mL). The mixture was extracted with ethyl acetate (50 mL × 3). The combined organic layer was washed with saturated sodium chloride solution (20 mL), then dried, filtered and concentrated to give a crude product, which was purified by flash column chromatography (silica, 0-10% ethyl acetate/petroleum ether) to give 4-bromo-*N*-(6-methyl-2-(3-vinylpiperidin-1-yl)pyrimidin-4-yl)-2-(4-vinylpiperidin-1-yl)benzamide (0.75 g, 1.47 mmol, yield: 45.5%) as a yellow liquid, LCMS (ESI): [M+H]⁺ =510.3.

**Step 3:** Under nitrogen atmosphere at 20°C, 4-bromo-*N*-(6-methyl-2-(3-vinylpiperidin-1-yl)pyrimidin-4-yl)-2-(4-vinylpiperidin-1-yl)benzamide (748 mg, 1.47 mmol) was dissolved in dry dichloromethane (80.00 mL), then Grubbs catalyst 2^{nd} generation (100 mg, 0.11 mmol) was added and the reaction was heated to reflux for 36 hours. After the reaction was complete, the concentrated reaction solution was purified by flash column chromatography (silica, 0-10% ethyl acetate/petroleum ether) to give 6⁵-bromo-1⁶-methyl-8-aza-1(2,4)-pyrimidin-2(1,3),6(4,1)-dipiperidin-6(1,2)-benzeneh eterocyclooct-3-en-7-one (432 mg, 0.90 mmol, yield: 60.9%) as a white solid, LCMS (ESI): [M+H]⁺ = 482.3.

**Step 4:** Under nitrogen atmosphere at 20°C, 6⁵-bromo-1⁶-methyl-8-aza-1(2,4)-pyrimidin-2(1,3),6(4,1)-dipiperidin-6(1,2)-benzeneh eterocyclooct-3-en-7-one (432 mg, 0.90 mmol), 2-hydroxyethylsulfonamide (343 mg, 2.69 mmol), potassium phosphate (776 mg, 3.58 mmol), tris(dibenzylideneacetone)dipalladium (84 mg, 0.09 mmol) and 2-di-tert-butylphosphino-2',4',6'-triisopropylbiphenyl (38 mg, 0.09 mmol) were dissolved in 1,4-dioxane (15.00 mL). The resulting reaction mixture was stirred at 100°C for 5 hours. After the reaction was complete, the reaction was quenched with cold water (20 mL). The mixture was extracted with ethyl acetate (50 mL × 3). The combined organic layer was dried, filtered and concentrated to give a crude product, which was purified by flash column chromatography (silica, 30-95% ethyl acetate/petroleum ether) to give 2-hydroxy-*N*-(1⁶-methyl-7-oxo-8-aza-1(2,4)-pyrimidin-2(1,3),5(4,1)-dipiperidin-6(1,2) -benzeneheterocyclooct-3-en-6⁵-yl)ethane-1-sulfonamide (282 mg, 0.54 mmol, yield: 59.8%), Z/E=94:6, LCMS (ESI): [M+H]⁺ =527.4. ¹H NMR (400 MHz, DMSO-*d*₆) δ 12.89 (s, 1H), 10.27 (brs, 1H), 8.02 (d, *J* = 8.5 Hz, 1H), 7.24 (s, 1H), 7.20 (d, *J* = 2.2 Hz, 1H), 7.13 (dd, *J* = 8.6, 2.0 Hz, 1H), 5.58 (dd, *J* = 12.3, 6.7 Hz, 1H), 5.15 (dd, *J =* 12.5, 7.4 Hz, 1H), 4.95 (brs, 1H), 4.60 (d, *J =* 13.0 Hz, 1H), 4.39 (d, *J =* 8.6 Hz, 1H), 3.76 (t, *J =* 6.5 Hz, 2H), 3.37 (t, *J =* 6.5 Hz, 2H), 3.29 - 3.03 (m, 3H), 2.97 (dd, *J =* 12.3, 9.8 Hz, 1H), 2.88 (t, *J =* 12.6 Hz, 1H), 2.66 - 2.46 (m, 3H), 2.31 (s, 3H), 2.30 - 2.19 (m, 1H), 2.00 (d, *J =* 12.9 Hz, 1H), 1.91 - 1.71 (m, 2H), 1.65 (d, *J =* 12.4 Hz, 1H), 1.55 (d, *J =* 12.5 Hz, 1H), 1.48 - 1.22 (m, 2H) ppm.

**Step 5:** At 20°C, 2-hydroxy-*N*-(1⁶-methyl-7-oxo-8-aza-1(2,4)-pyrimidin-2(1,3),5(4,1)-dipiperidin-6(1,2) -benzeneheterocyclooct-3-en-6⁵-yl)ethane-1-sulfonamide (88 mg, 0.18 mmol) and 10% palladium/carbon (88 mg, 100% w/w) were dissolved in methanol (10.00 mL), and acetic acid (0.10 g, 1.67 mmol) was added. The mixture was purged with hydrogen and stirred at 20°C for 24 hours to basically complete the reaction. The mixture was filtered through celite and washed with dichloromethane/methanol (50 mL), then concentrated and purified by flash column chromatography (silica, 50-70% ethyl acetate/petroleum ether) to give 2-hydroxy-*N*-(1⁶-methyl-7-oxo-8-aza-1(2,4)-pyrimidin-2(1,3),5(4,1)-dipiperidin-6(1,2) -benzeneheterocyclooct-6⁵-yl)ethane-1-sulfonamide (25 mg, 0.05 mmol, yield: 28.3%, crude). LCMS (ESI): [M+H]⁺=529.4. ¹H NMR (400 MHz, DMSO-*d*₆) δ 12.36 (s, 1H), 7.95 (d, *J* = 8.6 Hz, 1H), 7.23 (s, 1H), 7.16 (d, *J* = 2.1 Hz, 1H), 7.09 (dd, *J* = 8.6, 2.1 Hz, 1H), 4.54 (d, *J* = 13.2 Hz, 1H), 4.40 (d, *J* = 12.7 Hz, 1H), 3.76 (t, *J* = 6.4 Hz, 2H), 3.36 (t, *J =* 6.4 Hz, 2H), 3.31 - 2.80 (m, 5H), 2.57 - 2.41 (m, 1H), 2.30 (s, 3H), 1.95 - 1.00 (m, 14H) ppm.

### Example 6:

### 2-Hydroxy-N-(2¹-methyl-4-oxo-3-aza-2(2,4)-pyrimidin-1(1,3),6(1,4)-dipiperidin-5 (1,2)-benzenecyclodec-9-en-5⁴-yl)ethane-1-sulfonamide

### Example 7:

### 2-Hydroxy-N-(2⁶-methyl-4-oxo-3-aza-2(2,4)-pyrimidin-1(1,3),6(1,4)-dipiperidin-5 (1,2)-benzenecyclodecane-5⁴-yl)ethane-1-sulfonamide

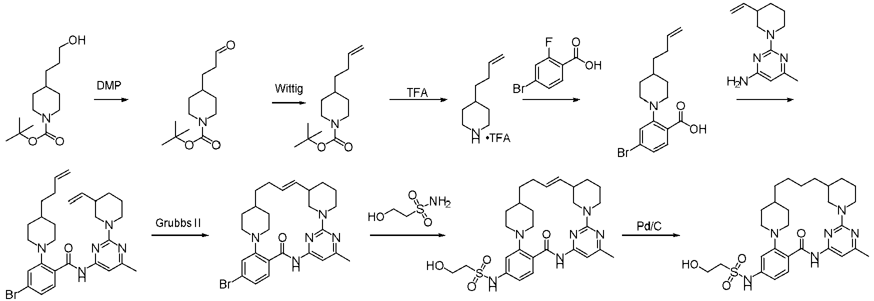

**Step 1:** At 20°C, N-tert-butoxycarbonyl-4-(3-hydroxypropyl)piperidine (5.0 g, 20.55 mmol) was dissolved in dry dichloromethane (200.00 mL). The solution was cooled to 0°C, then Dess-Martin periodinane (13.21 g, 30.82 mmol) was added portionwise, the temperature was raised to 20°C and the mixture was stirred for 1 hour. After the reaction was complete, the reaction was quenched with saturated sodium bicarbonate solution (100 mL) and sodium sulfite solution (100 mL) and the mixture was extracted with dichloromethane (200 mL × 3). The combined organic layer was dried, filtered and concentrated to give N-tert-butoxycarbonyl-4-(3-oxoalkenyl)piperidine (3.00 g, 12.43 mmol, yield: 60.5%, crude) as a colorless liquid.

**Step 2:** Under nitrogen atmosphere at 20°C, methyltriphenylphosphonium bromide (6.80 g, 18.65 mmol) was dissolved in dry tetrahydrofuran (100.00 mL), the mixture was cooled to 0°C, and then a 1.0 M lithium bis(trimethylsilyl)amide solution in tetrahydrofuran (18.65 mL, 18.65 mmol) was slowly added and stirred for 1 hour. To the reaction system was added a solution of crude N-tert-butoxycarbonyl-4-(3-oxoalkenyl)piperidine (3.00 g, 12.43 mmol) in tetrahydrofuran (10.00 mL). The temperature was raised to 20°C and the mixture was stirred for 16 hours. After the reaction was complete, the reaction was quenched with a saturated sodium bicarbonate solution (100 mL) and the mixture was extracted with ethyl acetate (100 mL × 3). The combined organic layer was dried, filtered and concentrated to give a crude product, which was purified by flash column chromatography (silica, 0-10% ethyl acetate/petroleum ether) to give N-tert-butoxycarbonyl-4-(3-buten-1-yl)piperidine (2.60 g, 10.86 mmol, yield: 87.4%) as a colorless liquid.

**Step 3:** At 20°C, N-tert-butoxycarbonyl-4-(3-buten-1-yl)piperidine (2.60 g, 10.86 mmol) was dissolved in dichloromethane (50.00 mL), then trifluoroacetic acid (6.20 mL, 80.98 mmol) was added and the mixture was stirred at 20°C for 16 hours. After the reaction was complete, the solvent and excess trifluoroacetic acid were removed to give 4-(3-buten-1-yl)piperidin trifluoroacetate (about 5.50 g, crude). The crude product was then dissolved in dimethyl sulfoxide (50.00 mL), then potassium carbonate (7.66 g, 54.31 mmol) and 4-bromo-2-fluorobenzoic acid (2.38 g, 10.86 mmol) were added and the mixture was heated to 140°C and stirred for 48 hours. After the reaction was complete, the mixture was cooled naturally, then the reaction was quenched with cold water (100 mL), and 1*N* hydrochloric acid was added to adjust the pH of the solution to 7-8. The mixture was extracted with ethyl acetate (200 mL × 3). The combined organic layer was washed with sodium bicarbonate solution (100 mL), then dried, filtered and concentrated to give a crude product, which was purified by flash column chromatography (silica, 0-20% ethyl acetate/petroleum ether) to give 4-bromo-2-(4-(3-buten-1-yl)piperidin-1-yl)benzoic acid (2.44 g, 7.21 mmol, yield: 66.4%) as an orange solid. LCMS (ESI): [M+H]⁺ =338.2. ¹H NMR (400 MHz, Chloroform-d) δ 8.15 (d, *J* = 8.3 Hz, 1H), 7.54 (s, 1H), 7.57 - 7.51 (m, 1H), 5.90 - 5.70 (m, 1H), 5.07 - 4.95 (m, 2H), 3.16 - 3.07 (m, 2H), 3.04 - 2.79 (m, 2H), 2.17 - 2.07 (m, 2H), 2.00 - 1.91 (m, 2H), 1.61 - 1.40 (m, 4H) ppm.

**Step 4:** At 20°C, 6-methyl-2-(3-vinylpiperidin)pyrimidin-4-amine (0.65 g, 2.96 mmol), 4-bromo-2-(4-(3-buten-1-yl)piperidin-1-yl)benzoic acid (1.00 g, 2.96 mmol), N,N,N',N'-tetramethyl-O-(7-azabenzotriazol-1-yl)uronium hexafluorophosphate (2.87 g, 7.39 mmol) and 4-dimethylaminopyridine (1.11 g, 8.87 mmol) were dissolved in dry N,N-dimethylformamide (20.00 mL). The mixture was heated to 60°C and stirred for 24 hours. After the reaction was complete, the mixture was cooled naturally, and the reaction was quenched with cold water (50 mL). The mixture was extracted with ethyl acetate (50 mL × 3). The combined organic layer was washed with saturated sodium chloride solution (20 mL), then dried, filtered and concentrated to give a crude product, which was purified by flash column chromatography (silica, 0-10% ethyl acetate/petroleum ether) to give 4-bromo-2-(4-(3-buten-1-yl)piperidin-1-yl)-*N*-(6-methyl-2-(3-vinylpiperidin-1-yl)pyri midin-4-yl)benzamide (0.71 g, 1.32 mmol, yield: 44.6%) as a yellow liquid, LCMS (ESI): [M+H]⁺ = 538.3.

**Step 5:** At 20°C, 4-bromo-2-(4-(3-buten-1-yl)piperidin-1-yl)-*N*-(6-methyl-2-(3-vinylpiperidin-1-yl)pyri midin-4-yl)benzamide (710 mg, 1.32 mmol) was dissolved in dry dichloromethane (80.00 mL), the mixture was purged with nitrogen for 15 minutes, Grubbs catalyst 2^{nd} generation (114 mg, 0.066 mmol) was added and the reaction was heated to reflux for 36 hours. After the reaction was complete, the concentrated reaction solution was purified by flash column chromatography (silica, 0-10% ethyl acetate/petroleum ether) to give 5⁴-bromo-2⁶-methyl-3-aza-2(2,4)-pyrimidin-1(1,3),6(1,4)-dipiperidin-5(1,2)-benzenec yclodec-9-en-4-one (580 mg, 1.14 mmol, yield: 86.2%) as a white solid, LCMS (ESI): [M+H]⁺ = 510.3.

**Step 6:** Under nitrogen atmosphere at 20°C, 5⁴-bromo-2⁶-methyl-3-aza-2(2,4)-pyrimidin-1(1,3),6(1,4)-dipiperidin-5(1,2)-benzenec yclodec-9-en-4-one (580 mg, 1.14 mmol), 2-hydroxyethylsulfonamide (290 mg, 2.27 mmol), potassium phosphate (738 mg, 3.41 mmol), tris(dibenzylideneacetone)dipalladium (106 mg, 0.11 mmol) and 2-di-tert-butylphosphino-2',4',6'-triisopropylbiphenyl (48 mg, 0.11 mmol) were dissolved in 1,4-dioxane (10.00 mL). The resulting mixture was stirred at 100°C for 5 hours. After the reaction was complete, the reaction was quenched with cold water (20 mL). The mixture was extracted with ethyl acetate (50 mL × 3). The combined organic layer was dried, filtered and concentrated to give a crude product, which was purified by flash column chromatography (silica, 50-70% ethyl acetate/petroleum ether). The product was further purified by preparative HPLC (C18, 70-95% gradient of acetonitrile/water) to give 2-hydroxy-*N*-(2⁶-methyl-4-oxo-3-aza-2(2,4)-pyrimidin-1(1,3),6(1,4)-dipiperidin-5(1,2) -benzenecyclodec-9-en-5⁴-yl)ethane-1-sulfonamide (178 mg, 0.32 mmol, yield: 28.3%), Z/E=3:7. LCMS (ESI): [M+H]⁺ =555.4. ¹H NMR (400 MHz, DMSO-*d*₆) δ 12.00 (s, 0.7H), 11.88 (s, 0.3H), 10.20 (brs, 1H), 7.99 (d, *J* = 8.6 Hz, 0.7H), 7.87 (d, *J* = 8.6 Hz, 0.3H), 7.33 (s, 0.7H), 7.20 (s, 1H), 7.33 (s, 0.3H), 7.11 - 7.02 (m, 1H), 5.75 - 5.58 (m, 0.3H), 5.50 (dd, J = 7.9, 7.5 Hz, 0.3H), 5.43 (dd, *J* = 11.5, 9.8 Hz, 0.7H), 5.14 (dd, *J =* 9.7 Hz, 0.7H), 4.93 (brs, 1H), 4.80 (d, *J =* 13.3 Hz, 0.3H), 4.71 (d, *J =* 13.2 Hz, 0.3H), 4.53 (d, *J* = 12.9 Hz, 0.7H), 4.41 (d, *J* = 12.5 Hz, 0.7H), 3.74 (t, *J* = 6.6 Hz, 2H), 3.26 - 3.02 (m, 2H), 3.02 - 2.90 (m, 1H), 2.87 - 2.64 (m, 2H), 2.59 (t, *J* = 11.6 Hz, 0.7H), 2.48 - 2.33 (m, 2.3H), 2.26 (s, 3H), 1.98 - 1.30 (m, 14H) ppm.

**Step 7:** At 20°C, 2-hydroxy-*N*-(2⁶-methyl-4-oxo-3-aza-2(2,4)-pyrimidin-1(1,3),6(1,4)-dipiperidin-5(1,2) -benzenecyclodec-9-en-5⁴-yl)ethane-1-sulfonamide (100 mg, 0.18 mmol) and 10% palladium/carbon (100 mg, 100% w/w) were dissolved in methanol (10.00 mL), and acetic acid (0.20 g, 3.34 mmol) was added. The mixture was purged with hydrogen and stirred at 20°C for 24 hours to complete the reaction. The mixture was filtered through celite and washed with dichloromethane/methanol (50 mL), then concentrated and purified by flash column chromatography (silica, 30-95% ethyl acetate/petroleum ether) to give 2-hydroxy-*N*-(2⁶-methyl-4-oxo-3-aza-2(2,4)-pyrimidin-1(1,3),6(1,4)-dipiperidin-5(1,2) -benzeneheterocyclononane-5⁴-yl)ethane-1-sulfonamide (17 mg, 0.03 mmol, yield: 17.0%). LCMS (ESI): [M+H]⁺ =557.4. ¹H NMR (400 MHz, DMSO-*d*₆) δ 12.25 (s, 1H), 8.02 (d, *J* = 8.7 Hz, 1H), 7.39 (s, 1H), 7.23 (d, *J* = 2.1 Hz, 1H), 7.10 (dd,*J* = 8.7, 2.1 Hz, 1H), 4.59 (dd, *J =* 31.7, 12.9 Hz, 2H), 3.75 (t, *J =* 6.5 Hz, 2H), 3.21 - 3.05 (m, 2H), 2.95 - 2.75 (m, 2H), 2.67 - 2.52 (m, 2H), 2.25 (s, 3H), 1.97 - 1.78 (m, 2H), 1.77 - 1.05 (m, 18H) ppm.

### Example 8:

### (Z)-2-hydroxy-N-(1⁶-methyl-7-oxo-8-aza-1(2,4)-pyrimidin-2(1,3),5(3,1)-dipiperidi n-6(1,2)-benzeneheterocyclooct-3-en-6⁵-yl)ethane-1-sulfonamide

### Example 9:

### 2-Hydroxy-N-(1⁶-methyl-7-oxo-8-aza-1(2,4)-pyrimidin-2(1,3),5(3,1)-dipiperidin-6 (1,2)-benzeneheterocyclooctan-6⁵-yl)ethane-1-sulfonamide

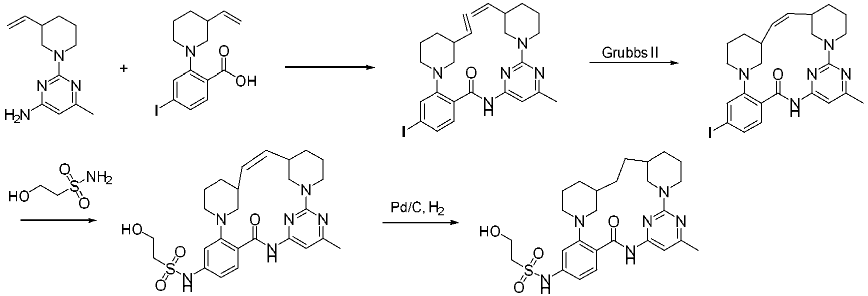

**Step 1:** At 20°C, 6-methyl-2-(3-vinylpiperidin-1-yl)pyrimidin-4-amine (0.61 g, 2.80 mmol), 4-iodo-2-(3-vinylpiperidin-1-yl)benzoic acid (1.00 g, 2.80 mmol), N,N,N',N'-tetramethyl-O-(7-azabenzotriazol-1-yl)uronium hexafluorophosphate (2.72 g, 7.00 mmol) and 4-dimethylaminopyridine (1.05 g , 8.40 mmol) were dissolved in dry *N*,*N*-dimethylacetamide (10.00 mL). The mixture was heated to 60°C and stirred for 24 hours. After the reaction was complete, the mixture was cooled naturally, and the reaction was quenched with cold water (50 mL). The mixture was extracted with ethyl acetate (50 mL × 3). The combined organic layer was washed with saturated sodium chloride solution (20 mL), then dried, filtered and concentrated to give a crude product, which was purified by flash column chromatography (silica, 0-10% ethyl acetate/petroleum ether) to give 4-iodo-*N*-(6-methyl-2-(3-vinylpiperidin-1-yl)pyrimidin-4-yl)-2-(3-vinylpiperidin-1-yl) benzamide (1.30 g, 2.33 mmol, yield: 83.3%) as a yellow liquid, LCMS (ESI): [M+H]⁺ = 558.3.

**Step 2:** Under nitrogen atmosphere at 20°C, 4-iodo-*N*-(6-methyl-2-(3-vinylpiperidin-1-yl)pyrimidin-4-yl)-2-(3-vinylpiperidin-1-yl) benzamide (1.30 g, 2.33 mmol) was dissolved in dry toluene (120.00 mL), Grubbs catalyst 2^{nd} generation (108 mg, 0.12 mmol) was added and additional Grubbs catalyst 2^{nd} generation (108 mg, 0.12 mmol) was added every 24 hours for a total reaction time of 72 h. Then the concentrated reaction solution was purified by flash column chromatography (silica, 0-10% ethyl acetate/petroleum ether) to give (Z)-6⁵-iodo-1⁶-methyl-8-aza-1(2,4)-pyrimidin-2(1,3),5(3,1)-dipiperidin-6(1,2)-benzen eheterocyclooct-3-en-7-one (281 mg, 0.53 mmol, yield: 22.8%) as a white solid, LCMS (ESI): [M+H]⁺ = 530.3.

**Step 3:** Under nitrogen atmosphere at 20°C, (Z)-6⁵-iodo-1⁶-methyl-8-aza-1(2,4)-pyrimidin-2(1,3),5(3,1)-dipiperidin-6(1,2)-benzen eheterocyclooct-3-en-7-one (281 mg, 0.53 mmol), 2-hydroxyethylsulfonamide (135 mg, 1.06 mmol), potassium phosphate (460 mg, 2.12 mmol) and cuprous iodide (103 mg, 0.53 mmol) were placed in a dry reaction flask. After the mixture was purged with nitrogen, trans-N,N'-dimethyl-1,2-cyclohexanediamine (153 mg, 1.06 mmol) and 1,4-dioxane (20.00 mL) were added. After the solid was dissolved, the mixture was stirred at 100°C for 12 hours. After the reaction was complete, the reaction was quenched with a saturated ammonium chloride solution (20 mL). The mixture was extracted with ethyl acetate (50 mL × 3). The combined organic layer was washed with saturated sodium chloride solution, then dried, filtered and concentrated to give a crude product, which was purified by flash column chromatography (silica, 50-70% ethyl acetate/petroleum ether) and preparative HPLC (C18, 50-60% acetonitrile/water) to give Example 8: (Z)-2-hydroxy-N-(1⁶-methyl-7-oxo-8-aza-1(2,4)-pyrimidin-2(1,3),5(3,1)-dipiperidin-6 (1,2)-benzeneheterocyclooct-3-en-6⁵-yl)ethane-1-sulfonamide (43 mg, 0.08 mmol, yield: 15.4%) as a white solid. LCMS (ESI): [M+H]⁺ =527.4. ¹H NMR (400 MHz, DMSO-*d*₆) δ 14.38 (s, 1H), 10.31 (s, 1H), 8.15 (d, *J* = 8.7 Hz, 1H), 7.31 (d, *J =* 2.2 Hz, 1H), 7.25 - 7.15 (m, 2H), 5.34 (t, *J =* 10.5 Hz, 1H), 5.15 (dd, *J =* 10.1, 4.8 Hz, 1H), 4.94 (s, 1H), 4.48 (d, *J =* 12.9 Hz, 1H), 4.21 (dd, *J* = 30.7, 12.0 Hz, 2H), 3.76 (t, *J* = 6.4 Hz, 2H), 3.36 (t, *J* = 6.5 Hz, 2H), 3.07 - 2.88 (m, 3H), 2.83 (td, *J* = 11.1, 4.3 Hz, 1H), 2.77 - 2.54 (m, 3H), 2.32 (s, 3H), 1.96 - 1.80 (m, 2H), 1.73 (t, *J* = 13.5 Hz, 2H), 1.62 - 1.33 (m, 3H), 1.32 - 1.14 (m, 1H).

**Step 4:** At 20°C, (Z)-2-hydroxy-N-(1⁶-methyl-7-oxo-8-aza-1(2,4)-pyrimidin-2(1,3),5(3,1)-dipiperidin-6 (1,2)-benzeneheterocyclooct-3-en-6⁵-yl)ethane-1-sulfonamide (30 mg, 0.06 mmol) and 10% palladium/carbon (30 mg, 100% w/w) were dissolved in methanol (5.00 mL), and formic acid (0.10 g, 2.17 mmol) was added. The mixture was purged with hydrogen and stirred at 20°C for 24 hours to complete the reaction. The mixture was concentrated and purified by flash column chromatography (silica, 30-95% ethyl acetate/petroleum ether) to give 2-hydroxy-N-(1⁶-methyl-7-oxo-8-aza-1(2,4)-pyrimidin-2(1,3),5(3,1)-dipiperidin-6(1,2) -benzeneheterocyclooctan-6⁵-yl)ethane-1-sulfonamide (18.6 mg, 0.04 mmol, yield: 62.0%). LCMS (ESI): [M+H]⁺ =529.4. ¹H NMR (400 MHz, DMSO-*d*₆) δ 13.87 (s, 1H), 10.28 (s, 1H), 8.15 (d, *J* = 8.7 Hz, 1H), 7.30 (d, *J* = 2.2 Hz, 1H), 7.24 - 7.14 (m, 2H), 4.95 (t, *J =* 5.7 Hz, 1H), 4.52 (d, *J =* 13.1 Hz, 1H), 4.29 (d, *J* = 12.9 Hz, 1H), 3.77 (q, *J =* 6.1 Hz, 2H), 3.37 (t, *J =* 6.5 Hz, 2H), 3.07 (d, *J =* 10.9 Hz, 1H), 2.97 - 2.83 (m, 3H), 2.59 (t, *J* = 11.6 Hz, 1H), 2.39 (t, *J* = 10.9 Hz, 1H), 2.31 (s, 3H), 2.10 (d, *J* = 12.8 Hz, 1H), 1.95 - 1.71 (m, 2H), 1.66 (d, *J =* 11.8 Hz, 1H), 1.52 (d, *J =* 12.4 Hz, 1H), 1.45 - 1.14 (m, 7H), 1.13 - 0.91 (m, 2H) ppm.

### Example 10:

### 2-Hydroxy-N-(2⁶-methyl-4-oxo-3-aza-2(2,4)-pyrimidin-1,6(1,3)-dipiperidin-5(1,2) -benzeneheterocyclononane-8-en-5⁴-yl)ethane-1-sulfonamide

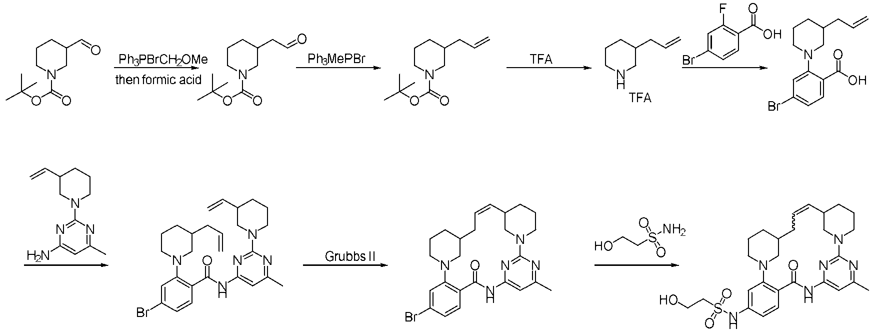

**Step 1:** Under nitrogen atmosphere at 20°C, (methoxymethyl)triphenylphosphonium bromide (12.30 g, 35.17 mmol) was dissolved in dry tetrahydrofuran (150.00 mL), the mixture was cooled to 0°C, and then a 1.0 M lithium bis(trimethylsilyl)amide solution in tetrahydrofuran (35.17 mL, 35.17 mmol) was slowly added and stirred for 1 hour. To the reaction system was added a solution of N-tert-butoxycarbonyl-3-formylpiperidine (5.00 g, 23.44 mmol) in tetrahydrofuran (10.00 mL). The temperature was raised to 20°C and the mixture was stirred for 16 hours. After the reaction was complete, the reaction was quenched with a saturated sodium bicarbonate solution (100 mL) and the mixture was extracted with ethyl acetate (100 mL × 3). The combined organic layer was dried, filtered and concentrated to give a crude product (about 19 g).

The crude product was dissolved in formic acid (10.00 mL) and the mixture was stirred for 7 hours. A saturated sodium bicarbonate solution (200 mL) was then added to quench the reaction and the mixture was extracted with ethyl acetate (100 mL × 3). The combined organic layer was dried, filtered and concentrated to give a crude product, which was purified by flash column chromatography (silica, 8-12% ethyl acetate/petroleum ether) to give N-tert-butoxycarbonyl-3-(2-oxoethyl)piperidine (3.90 g, 17.15 mmol, yield: 73.2%) as a colorless liquid.

**Step 2:** Under nitrogen atmosphere at 20°C, methyltriphenylphosphonium bromide (9.38 g, 25.74 mmol) was dissolved in dry tetrahydrofuran (100.00 mL), the mixture was cooled to 0°C, and then a 1.0 M lithium bis(trimethylsilyl)amide solution in tetrahydrofuran (25.74 mL, 25.74 mmol) was slowly added and stirred for 1 hour. To the reaction system was added a solution of N-tert-butoxycarbonyl-3-(2-oxoethyl)piperidine(3.90 g, 17.15 mmol) in tetrahydrofuran (10.00 mL). The temperature was raised to 20°C and the mixture was stirred for 16 hours. After the reaction was complete, the reaction was quenched with a saturated sodium bicarbonate solution (100 mL) and the mixture was extracted with ethyl acetate (100 mL × 3). The combined organic layer was dried, filtered and concentrated to give a crude product, which was purified by flash column chromatography (silica, 0-10% ethyl acetate/petroleum ether) to give N-tert-butoxycarbonyl-3-allylpiperidin (3.10 g, 13.76 mmol, yield: 80.1%) as a colorless liquid.

**Step 3:** At 20°C, N-tert-butoxycarbonyl-3-allylpiperidin (3.10 g, 13.76 mmol) was dissolved in dichloromethane (50.00 mL), then trifluoroacetic acid (5.26 mL, 68.79 mmol) was added and the mixture was stirred at 20°C for 16 hours. After the reaction was complete, the solvent and excess trifluoroacetic acid were removed to give 3-(prop-2-en-1-yl)piperidin trifluoroacetate (about 6.50 g, crude). The crude product was then dissolved in dimethyl sulfoxide (50.00 mL), then potassium carbonate (9.70 g, 68.79 mmol) and 4-bromo-2-fluorobenzoic acid (3.01 g, 13.76 mmol) were added and the mixture was heated to 140°C and stirred for 48 hours. After the reaction was complete, the mixture was cooled naturally, then the reaction was quenched with cold water (100 mL), and 1*N* hydrochloric acid was added to adjust the pH of the solution to 7-8. The mixture was extracted with ethyl acetate (200 mL × 3). The combined organic layer was washed with sodium bicarbonate solution (100 mL), then dried, filtered and concentrated to give a crude product, which was purified by flash column chromatography (silica, 0-20% ethyl acetate/petroleum ether) to give 4-bromo-2-(3-allylpiperidin-1-yl)benzoic acid (3.77 g, 11.63 mmol, yield: 84.5%) as an orange solid. LCMS (ESI): [M+H]⁺ =324.1. ¹H NMR (400 MHz, Chloroform-d) δ 8.15 (d, *J* = 8.9 Hz, 1H), 7.57 - 7.50 (m, 2H), 5.83 - 5.65 (m, 1H), 5.10 - 4.98 (m, 2H), 3.19 - 3.00 (m, 2H), 2.83 (t, *J* = 11.5 Hz, 1H), 2.53 (t, *J =* 9.1 Hz, 1H), 2.22 - 1.66 (m, 6H), 1.22 - 1.03 (m, 1H) ppm .

**Step 4:** At 20°C, 6-methyl-2-(3-vinylpiperidin)pyrimidin-4-amine (0.96 g, 4.41 mmol), 4-bromo-2-(3-allylpiperidin-1-yl)benzoic acid (1.30 g, 4.10 mmol), N,N,N',N' -tetramethyl-O-(7-azabenzotriazol-1-yl)uronium hexafluorophosphate (5.38 g, 13.88 mmol) and 4-dimethylaminopyridine (2.88 g , 23.13 mmol) were dissolved in dry N,N-dimethylformamide (20.00 mL). The mixture was heated to 60°C and stirred for 48 hours. After the reaction was complete, the mixture was cooled naturally, and the reaction was quenched with cold water (50 mL). The mixture was extracted with ethyl acetate (50 mL × 3). The combined organic layer was washed with saturated sodium chloride solution (20 mL), then dried, filtered and concentrated to give a crude product, which was purified by flash column chromatography (silica, 0-10% ethyl acetate/petroleum ether) to give 2-(3-allylpiperidin-1-yl)-4-bromo-N-(6-methyl-2-(3-vinylpiperidin-1-yl)pyrimidin-4-y l)benzamide (1.15 g, 2.24 mmol, yield: 54.8%) as a yellow liquid, LCMS (ESI): [M+H]⁺ =524.3.

**Step 5:** Under nitrogen atmosphere at 20°C, 2-(3-allylpiperidin-1-yl)-4-bromo-*N*-(6-methyl-2-(3-vinylpiperidin-1-yl)pyrimidin-4-y l)benzamide (1.60 g, 3.05 mmol) was dissolved in dry dichloromethane (120.00 mL), Grubbs catalyst 2^{nd} generation (132 mg, 0.15 mmol) was added and the reaction was heated to reflux for 24 hours. After the reaction was complete, the concentrated reaction solution was purified by flash column chromatography (silica, 0-10% ethyl acetate/petroleum ether) to give 5⁴-bromo-2⁶-methyl-3-aza-2(2,4)-pyrimidin-1,6(1,3)-dipiperidin-5(1,2)-benzeneheter ocyclonon-8-en-4-one (1.31 g, 2.64 mmol, yield: 86.6%) as a white solid, LCMS (ESI): [M+H]⁺ = 496.3.

**Step 6:** Under nitrogen atmosphere at 20°C, 5⁴-bromo-2⁶-methyl-3-aza-2(2,4)-pyrimidin-1,6(1,3)-dipiperidin-5(1,2)-benzeneheter ocyclonon-8-en-4-one (1.31 g, 2.64 mmol), 2-hydroxyethylsulfonamide (1.01 g, 7.92 mmol), potassium phosphate (2.29 g, 10.56 mmol), tris(dibenzylideneacetone)dipalladium (247 mg, 0.26 mmol) and 2-di-tert-butylphosphino-2',4',6'-triisopropylbiphenyl (112 mg, 0.26 mmol) were dissolved in 1,4-dioxane (25.00 mL). The resulting reaction mixture was stirred at 100°C for 12 hours. After the reaction was complete, the reaction was quenched with cold water (20 mL). The mixture was extracted with ethyl acetate (50 mL × 3). The combined organic layer was dried, filtered and concentrated to give a crude product, which was purified by flash column chromatography (silica, 50-70% ethyl acetate/petroleum ether) to give 2-hydroxy-N-(2⁶-methyl-4-oxo-3-aza-2(2,4)-pyrimidin-1,6(1,3)-dipiperidin-5(1,2)-be nzeneheterocyclononane-8-en-5⁴-yl)ethane-1-sulfonamide (1.15 g, 2.13 mmol, yield: 80.6%). LCMS (ESI): [M+H]⁺ =541.4. ¹H NMR (400 MHz, DMSO-*d*₆) δ 13.28 (s, 0.18H), 12.69 (s, 0.55H), 11.24 (s, 0.27H), 10.24 (brs, 1H), 8.23 - 7.76 (m, 1H), 7.52 - 7.00 (m, 3H), 5.68 - 5.21 (m, 2H), 4.94 (brs, 1H), 4.74 - 4.55 (m, 1H), 4.35 - 4.08 (m, 1H), 3.78 (t, *J* = 6.5 Hz, 2H), 3.67 - 3.41 (m, 1H), 3.37 (td, *J* = 6.6, 2.5 Hz, 2H), 3.29 - 2.84 (m, 4H), 2.71 - 2.37 (m, 3H), 2.32 (s, 3H), 2.18 - 1.23 (m, 10H) ppm.

### Example 11:

### 2-Hydroxy-N-(2⁶-methyl-4-oxo-3-aza-2(2,4)-pyrimidin-1,6(1,3)-dipiperidin-5(1,2) -benzoylcyclononyl-5⁴-yl)ethane-1-sulfonamide

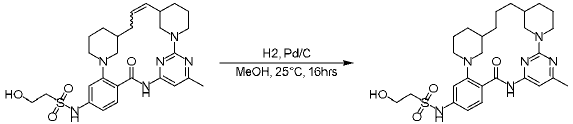

**Step** 1: At 25°C, to a solution of 2-hydroxy-N-(2⁶-methyl-4-oxo-3-aza-2(2,4)-pyrimidin-1,6(1,3)-dipiperidin-5(1,2)-be nzylcyclononane-8-en-5⁴yl)ethane-1-sulfonamide (200 mg, 0.37 mmol) in methanol (4 mL) was added wet palladium on carbon (20 mg). The reaction mixture was purged with argon three times and then hydrogen three times, and stirred for 16 hours under hydrogen atmosphere (50 Psi). The reaction mixture was filtered under reduced pressure to remove the palladium on carbon, and then concentrated under vacuum to give
2-hydroxy-N-(2⁶-methyl-4-oxo-3-aza-2(2,4)-pyrimidin-1,6(1,3)-dipiperidin-5(1,2)-be nzoylcyclononyl-5⁴-yl)ethane-1-sulfonamide (190 mg, 0.35 mmol, yield: 95%) as a white solid compound. LCMS (ESI): [M+H]⁺ = 543.2. ¹H NMR (400 MHz, DMSO-d6) δ ppm 12.60 - 11.32 (m, 1H), 11.01 - 8.79 (m, 1H), 8.32 - 7.57 (m, 1H), 7.52 - 6.58 (m, 3H), 6.00 - 4.86 (m, 1H), 4.70 - 4.00 (m, 2H), 3.83 - 3.55 (m, 2H), 3.23 - 2.67 (m, 6H), 2.38 - 2.10 (m, 5H), 2.05 - 0.98 (m, 16H)

### Example 12:

### N-(6,6-dioxo-3-oxo-6-thia-4,7-diaza-1(1,4)-piperidin-2(1,2),5(1,3)-diphenylhetero cyclododec-9-ene-2⁵-yl)-2-hydroxy-1-ethanesulfonamide

### Example 13:

### N-(6,6-dioxo-3-oxo-6-thia-4,7-diaza-1(1,4)-piperidin-2(1,2),5(1,3)-diphenylhetero cyclododecane-2⁵-yl)-2-hydroxy-1-ethanesulfonamide

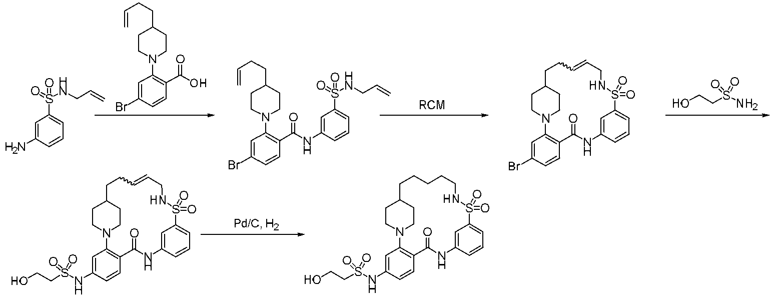

**Step 1:** At 20°C, N-allyl-3-aminobenzenesulfonamide (628 mg, 2.96 mmol), 4-bromo-2-(4-(3-buten-1-yl)piperidin-1-yl)benzoic acid (1.00 g, 2.96 mmol), N,N,N',N'-tetramethyl-O-(7-azabenzotriazol-1-yl)uronium hexafluorophosphate (2.87 g, 7.39 mmol) and 4-dimethylaminopyridine (1.11 g, 8.87 mmol) were dissolved in dry N,N-dimethylformamide (20.00 mL). The resulting reaction mixture was stirred at 25°C for 24 hours. After the reaction was complete, the reaction was quenched with water (50 mL). The mixture was extracted with ethyl acetate (50 mL × 3). The combined organic layer was washed with saturated sodium chloride solution (20 mL), then dried, filtered and concentrated to give a crude product, which was purified by flash column chromatography (silica, 20-25% ethyl acetate/petroleum ether) to give N-(3-(N-allylsulfamoyl)phenyl)-4-bromo-2-(4-(3-buten-1-yl)piperidin-1-yl)benzamid e (1.30 g, 1.89 mmol, yield: 63.7%) as a yellow liquid, LCMS (ESI): [M+H]⁺ =532.2.

**Step 2:** At 20°C, N-(3-(N-allylsulfamoyl)phenyl)-4-bromo-2-(4-(3-buten-1-yl)piperidin-1-yl)benzamid e (1.30 g, 1.89 mmol) was dissolved in dry toluene (80.00 mL), Grubbs catalyst 2^{nd} generation (180 mg, 0.20 mmol) was added and the reaction was heated to reflux for 16 hours. After the reaction was complete, the concentrated reaction solution was purified by flash column chromatography (silica, 25-30% ethyl acetate/petroleum ether) to give 2⁵-bromo-6,6-dioxo-6-thia-4,7-diaza-1(1,4)-piperidin-2(1,2),5(1,3)-diphenylheterocyc lododec-9-en-3-one (410 mg, 0.81 mmol, yield: 43.0%) as a white solid, LCMS (ESI): [M+H]⁺ =504.2.

**Step 3:** Under nitrogen atmosphere at 20°C, 2⁵-bromo-6,6-dioxo-6-thia-4,7-diaza-1(1,4)-piperidin-2(1,2),5(1,3)-diphenylheterocyc lododec-9-en-3-one (410 mg, 0.81 mmol), 2-hydroxyethylsulfonamide (311 mg, 2.44 mmol), potassium phosphate (704 mg, 3.25 mmol), cuprous iodide (158 mg, 0.81 mmol) and trans-N,N'-dimethyl-1,2-cyclohexanediamine (234 mg, 1.63 mmol) were dissolved in N,N-dimethylformamide (10.00 mL). The resulting reaction mixture was stirred at 100°C for 15 hours. After the reaction was complete, the mixture was cooled to 25°C, and the reaction was quenched with ammonium chloride solution (20 mL). The mixture was extracted with ethyl acetate (20 mL × 3). The combined organic layer was washed with saturated sodium chloride solution, then dried, filtered and concentrated to give a crude product. The product was purified by flash column chromatography (silica, 50-70% ethyl acetate/petroleum ether) and separated by preparative HPLC (C18, 40-50% acetonitrile/water) to give **Example 12** N-(6,6-dioxo-3-oxo-6-thia-4,7-diaza-1(1,4)-piperidin-2(1,2),5(1,3)-diphenylheterocyc lododec-9-ene-2⁵-yl)-2-hydroxy-1-ethanesulfonamide (189 mg, 0.34 mmol, yield: 42.4%) as a white solid. LCMS (ESI): [M+H]⁺ =549.3. ¹H NMR (400 MHz, DMSO-*d*₆) δ 11.44 (s, 0.18H), 11.18 (s, 0.50H), 11.10 (s, 0.11H), 10.51 (s, 0.20H), 8.73 (dd, *J* = 8.3, 2.0 Hz, 0.50H), 8.62 (dd, *J* = 8.1, 2.1 Hz, 0.18H), 8.57 (dd, *J* = 8.3, 2.1 Hz, 0.11H), 8.17 (t, *J =* 1.9 Hz, 0.20H), 7.95 - 7.41 (m, 5H), 7.13 - 6.85 (m, 2H), 5.62 - 4.66 (m, 2H), 3.76 (t, *J =* 6.6 Hz, 2H), 3.54 - 3.27 (m, 2H), 3.25 - 3.12 (m, 2H), 2.84 - 2.52 (m, 2H), 2.29 - 2.06 (m, 1H), 1.98 - 1.02 (m, 10H) ppm.

**Step 4:** At 20°C, N-(6,6-dioxo-3-oxo-6-thia-4,7-diaza-1(1,4)-piperidin-2(1,2),5(1,3)-diphenylheterocyc lododec-9-ene-2⁵-yl)-2-hydroxy-1-ethanesulfonamide (12 mg, 0.023 mmol) and 10% palladium/carbon (100 mg, 100% w/w) were dissolved in methanol (10.00 mL), and formic acid (0.20 g, 3.34 mmol) was added. The mixture was purged with hydrogen and stirred at 20°C for 24 hours to complete the reaction. The reaction solution was concentrated and purified by flash column chromatography (silica, 30-95% ethyl acetate/petroleum ether) to give **Example 13** N-(6,6-dioxo-3-oxo-6-thia-4,7-diaza-1(1,4)-piperidin-2(1,2),5(1,3)-diphenylheterocyc lododecane-2⁵-yl)-2-hydroxy-1-ethanesulfonamide (17 mg, 0.03 mmol, yield: 17.0%) as a white solid. LCMS (ESI): [M+H]⁺ =551.3. ¹H NMR (400 MHz, DMSO-*d*₆) δ 11.09 (s, 1H), 10.04 (s, 1H), 8.64 (dd, *J =* 8.1, 2.1 Hz, 1H), 7.73 (t, *J =* 5.2 Hz, 1H), 7.66 (d, *J =* 8.3 Hz, 1H), 7.63 - 7.54 (m, 2H), 7.44 (d, *J =* 8.1 Hz, 1H), 7.00 - 6.90 (m, 2H), 4.94 (t, *J* = 5.6 Hz, 1H), 3.72 (q, *J =* 6.2 Hz, 2H), 3.29 (t, *J =* 6.6 Hz, 2H), 3.13 (d, *J =* 11.1 Hz, 2H), 2.64 (t, *J* = 10.8 Hz, 2H), 2.58 - 2.48 (m, 2H), 1.64 (d, *J* = 10.7 Hz, 2H), 1.60 - 1.48 (m, 2H), 1.44 - 1.32 (m, 2H), 1.30 - 1.17 (m, 5H), 1.15 - 1.02 (m, 2H) ppm.

### Example 14:

### N-(6,6-dioxo-3-oxo-6-thia-1(1,4)-piperidin-2(1,2),5(1,3)-diphenylheterocyclounde cane-2⁵-yl)-2-hydroxy-1-ethanesulfonamide

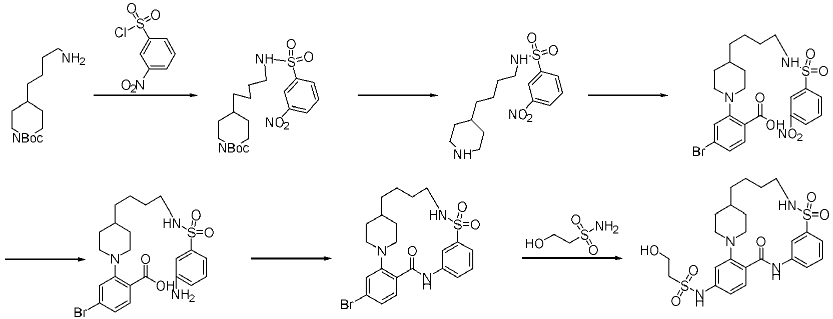

**Step 1:** A mixture containing tert-butyl 4-(4-aminobutyl)piperidinecarboxylate (7.00 g, 27.30 mmol), 3-nitrobenzenesulfonyl chloride (6.70 g, 30.03 mmol), N,N-diisopropylethylamine (18.00 g, 136.51 mmol) and tetrahydrofuran (70.00 mL) was stirred at 20°C for 12 hours. The reaction solution was poured into water (50 mL) and extracted with ethyl acetate (50 mL × 3). The organic layer was dried over anhydrous sodium sulfate, then filtered and concentrated, and purified by column chromatography (silica gel, 0-50% gradient of ethyl acetate/petroleum ether) to give tert-butyl 4-(4-((3-nitrophenyl)sulfonamido)butyl)piperidine-1-carboxylate (10.30 g, 23.33 mmol, yield: 85.4%) as a yellow oil. LCMS (ESI): [M-100]⁺ = 342.2.

**Step 2:** A mixture containing tert-butyl 4-(4-((3-nitrophenyl)sulfonamido)butyl)piperidine-1-carboxylate (5.00 g, 11.32 mmol) and hydrochloric acid/ethyl acetate solution (20.00 mL, 4.0 M) was stirred at 20°C for 1 hour. The reaction solution was concentrated to give 3-nitro-N-(4-(piperidin-4-yl)butyl)benzenesulfonamide (3.90 g, crude) as a white solid. LCMS (ESI): [M+H]⁺ = 342.2.

**Step 3:** A mixture containing 3-nitro-N-(4-(piperidin-4-yl)butyl)benzenesulfonamide (3.50 g, 10.25 mmol), 4-bromo-2-fluorobenzoic acid (2.90 g, 12.30 mmol), potassium carbonate (4.30 g, 30.75 mmol) and dimethyl sulfoxide (50.00 mL) was stirred at 120°C for 12 hours. The reaction solution was poured into water (50 mL), then 4.0 M hydrochloric acid/ethyl acetate solution (20 mL) was added to adjust the pH to nearly 5, and the solution was extracted with ethyl acetate (50 mL × 3). The organic layer was dried over anhydrous sodium sulfate, then filtered and concentrated, and purified by column chromatography (silica gel, 0-50% gradient of ethyl acetate/petroleum ether) to give 4-bromo-2-(4-(4-((3-nitrophenyl)sulfonamido)butyl)piperidin-1-yl)benzoic acid (7.20 g, crude) as a black oil. LCMS (ESI): [M+H]⁺ = 540.2.

**Step 4:** A solution containing 4-bromo-2-(4-(4-((3-nitrophenyl)sulfonamido)butyl)piperidin-1-yl)benzoic acid (7.00 g, 12.95 mmol), iron powder (3.70 g, 64.76 mmol), ammonium chloride (3.50 g, 64.76 mmol), ethanol (50.00 mL) and water (10.00 mL) was stirred at 60°C for 1 hour. The reaction solution was filtered while still hot, then concentrated to remove ethanol, and extracted with ethyl acetate (30 mL × 3). The organic layer was dried over anhydrous sodium sulfate, then filtered and concentrated, and purified by column chromatography (C18, 0-50% gradient of acetonitrile/water) to give 2-(4-(4-((3-aminophenyl)sulfonamido)butyl)piperidin-1-yl)-4-bromobenzoic acid (1.10 g, 2.15 mmol, yield: 16.6%) as a yellow solid. LCMS (ESI): [M+H]⁺ = 510.2.

**Step 5:** 2-(4-(4-(3-aminophenyl)sulfonamido)butyl)piperidin-1-yl)-4-bromobenzoic acid (1.10 g, 2.16 mmol) was dissolved in dichloromethane (100.00 mL). The mixture was added dropwise to a solution containing 2-chloro-1-methylpyridinium iodide (843 mg, 3.23 mmol), N,N-diisopropylethylamine (853 mg, 6.46 mmol) and dichloromethane (500.00 mL). The resulting mixture was stirred at 20°C for 2 hours. The reaction solution was poured into water (100 mL) and extracted with dichloromethane (50 mL × 3). The organic layer was dried over anhydrous sodium sulfate, then filtered and concentrated, and purified by column chromatography (silica gel, 0-50% gradient of ethyl acetate/petroleum ether) to give 2⁵-bromo-6,6-dioxo-6-thia-4,7-diaza-1(1,4)-piperidin-2(1,2),5(1,3)-diphenylheterocyc loundec-3-one (700 mg, 1.42 mmol, yield: 66.0%) as a white solid. LCMS (ESI): [M+H]⁺ = 492.2.

**Step 6:** A mixture containing 2⁵-bromo-6,6-dioxo-6-thia-4,7-diaza-1(1,4)-piperidin-2(1,2),5(1,3)-diphenylheterocyc loundec-3-one (500 mg, 1.02 mmol), 2-hydroxyethanesulfonamide (259 mg, 2.03 mmol), potassium phosphate (660 mg, 3.05 mmol), 2-di-tert-butylphosphino-2',4',6'-triisopropylbiphenyl (43 mg, 0.10 mmol), tris(dibenzylideneacetone)dipalladium (95 mg, 0.10 mol) and 1,4-dioxane (5.00 mL) was stirred at 100°C for 8 hours under nitrogen atmosphere. The reaction solution was poured into water (30 mL) and extracted with ethyl acetate (30 mL × 3). The organic layer was dried over anhydrous sodium sulfate, then filtered and concentrated, and purified by column chromatography (silica gel, 0-50% gradient of ethyl acetate/petroleum ether) and (C18, 0-60% gradient of acetonitrile/water) to give *N*-(6,6-dioxo-3-oxo-6-thia-1(1,4)-piperidin-2(1,2),5(1,3)-diphenylheterocycloundecan e-2⁵-yl)-2-hydroxy-1-ethanesulfonamide (120 mg, 0.22 mmol, yield: 22.0%) as a white solid. LCMS (ESI): [M+H]⁺ =537.3. ¹H NMR (400 MHz, DMSO) δ 10.86 (s, 1H), 10.05 (brs, 1H), 8.77 - 8.70 (m, 1H), 7.67 - 7.61 (m, 3H), 7.60 - 7.49 (m, 2H), 7.00 - 6.93 (m, 2H), 4.99 (brs, 1H), 3.78 (t, J = 6.8 Hz, 2H), 3.34-3.28 (m, 2H), 3.17 (d, J = 11.2 Hz, 2H), 3.02 (q, J = 6.0 Hz, 2H), 2.66 (t, J = 10.8 Hz, 2H), 1.66-1.51 (m, 3H), 1.43 - 1.30 (m, 4H), 1.28 - 1.18 (m, 2H), 1.14-0.99 (m, 2H) ppm.

### Example 15:

### (E)-N-(6,6-dioxo-3-oxo-6-thia-4,7-diaza-1(1,4)-piperidin-2(1,2),5(1,3)-diphenylhet erocyclodec-9-en-2⁵-yl)-2-hydroxy-1-ethanesulfonamide

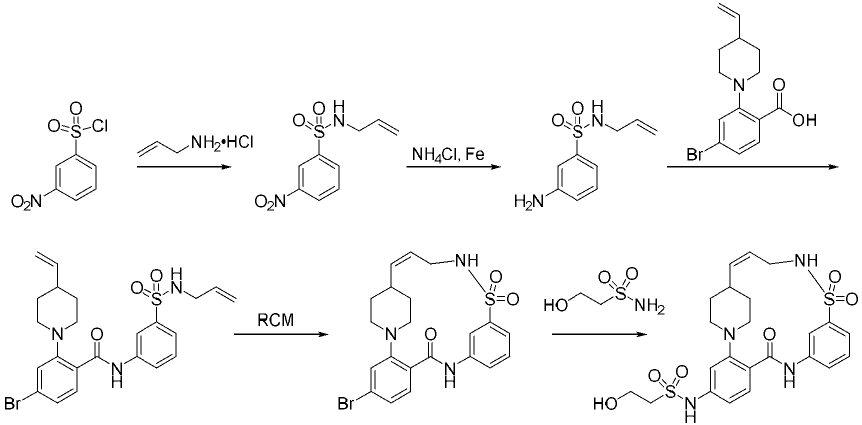

**Step 1:** At 20°C, 3-nitrobenzenesulfonyl chloride (5.00 g, 22.56 mmol) and allylamine hydrochloride (2.56 g, 27.07 mmol) were dissolved in dry dichloromethane (50.00 mL). The solution was cooled to 0°C, then triethylamine (9.60 mL, 67.68 mmol) was added and the mixture was stirred for 2 hours. After the reaction was complete, saturated sodium bicarbonate solution (100 mL) and sodium sulfite solution (100 mL) were added to quench the reaction and the mixture was extracted with dichloromethane (50 mL × 3). The combined organic layer was dried, filtered and concentrated to give N-allyl-3-nitrobenzenesulfonamide (5.47 g, 22.56 mmol, 100%). LCMS (ESI): [M+H]⁺ = 243.1.

**Step 2:** At 20°C, N-allyl-3-nitrobenzenesulfonamide (5.47 g, 22.56 mmol) was dissolved in ethanol (100.00 mL) and water (20.00 mL), and ammonium chloride (12.19 g, 225.60 mmol) and iron powder (12.73 g, 225.60 mmol) were added. The mixture was heated to 85°C and stirred for 4 hours. After the reaction was complete, the mixture was filtered and washed with ethyl acetate (100 mL), concentrated under reduced pressure to remove ethanol, and extracted with ethyl acetate (100 mL × 3). The combined organic layer was dried, filtered and concentrated to give a crude product, which was purified by flash column chromatography (silica, 30-40% ethyl acetate/petroleum ether) to give N-allyl-3-aminobenzenesulfonamide (3.50 g, 16.49 mmol, yield: 73.1%) as a white solid. LCMS (ESI): [M+H]⁺ =213.1. ¹H NMR (400 MHz, Chloroform-d) δ 7.29 - 7.12 (m, 3H), 6.82 (ddd, *J* = 7.8, 2.4, 1.2 Hz, 1H), 5.76 - 5.62 (m, 1H), 5.18 - 5.10 (m, 2H), 5.05 (dq, *J* = 10.3, 1.4 Hz, 1H), 4.15 (brs, 2H), 3.54 (ddt, *J* = 6.0, 6.0, 1.6 Hz, 2H) ppm.

**Step 3:** At 20°C, N-allyl-3-aminobenzenesulfonamide (684 mg, 3.22 mmol), 4-bromo-2-(4-vinylpiperidin-1-yl)benzoic acid (1.00 g, 3.22 mmol), N,N,N ' ,N ' -tetramethyl-O-(7-azabenzotriazol-1-yl)uronium hexafluorophosphate (3.13 g, 8.06 mmol) and 4-dimethylaminopyridine (1.20 g , 9.67 mmol) were dissolved in dry N,N-dimethylformamide (20.00 mL). The resulting reaction mixture was stirred at 25°C for 24 hours. After the reaction was complete, the reaction was quenched with water (50 mL). The mixture was extracted with ethyl acetate (50 mL × 3). The combined organic layer was washed with saturated sodium chloride solution (20 mL), then dried, filtered and concentrated to give a crude product, which was purified by flash column chromatography (silica, 20-25% ethyl acetate/petroleum ether) to give N-(3-(N-allylsulfamoyl)phenyl)-4-bromo-2-(4-vinylpiperidin-1-yl)benzamide (970 mg, 1.92 mmol, yield: 59.6%) as a yellow liquid, LCMS (ESI): [M+H]⁺ = 504.2.

**Step 4:** At 20°C, N-(3-(N-allylsulfamoyl)phenyl)-4-bromo-2-(4-vinylpiperidin-1-yl)benzamide (970 mg, 1.92 mmol) was dissolved in dry toluene (80.00 mL), Grubbs catalyst 2^{nd} generation (180 mg, 0.20 mmol) was added and the reaction was heated to reflux for 16 hours. After the reaction was complete, the concentrated reaction solution was purified by flash column chromatography (silica, 25-30% ethyl acetate/petroleum ether) to give (*E*)-2⁵-bromo-6,6-dioxo-6-thia-4,7-diaza-1(1,4)-piperidin-2(1,2),5(1,3)-diphenylheter ocyclodec-9-en-3-one (280 mg, 0.59 mmol, yield: 30.6%) as a white solid, LCMS (ESI): [M+H]⁺ = 476.3.

**Step 5:** Under nitrogen atmosphere at 20°C, (*E*)-2⁵-bromo-6,6-dioxo-6-thia-4,7-diaza-1(1,4)-piperidin-2(1,2),5(1,3)-diphenylheter ocyclodec-9-en-3-one (145 mg, 0.30 mmol), 2-hydroxyethylsulfonamide (233 mg, 1.83 mmol), potassium phosphate (659 mg, 3.04 mmol), cuprous iodide (118 mg, 0.61 mmol) and trans-N,N'-dimethyl-1,2-cyclohexanediamine (175 mg, 1.22 mmol) were dissolved in N,N-dimethylformamide (10.00 mL). The resulting reaction mixture was stirred at 100°C for 15 hours. After the reaction was complete, the mixture was cooled to 25°C, and the reaction was quenched with ammonium chloride solution (20 mL). The mixture was extracted with ethyl acetate (20 mL × 3). The combined organic layer was washed with saturated sodium chloride solution, then dried, filtered and concentrated to give a crude product, which was purified by flash column chromatography (silica, 50-70% ethyl acetate/petroleum ether) to give a product. The product was further recrystallized (anhydrous acetonitrile) to give (*E*)-N-(6,6-dioxo-3-oxo-6-thia-4,7-diaza-1(1,4)-piperidin-2(1,2),5(1,3)-diphenylheter ocyclodec-9-en-2⁵-yl)-2-hydroxy-1-ethanesulfonamide (12 mg, 0.023 mmol, yield: 7.6%) as a white solid. LCMS (ESI): [M+H]⁺ =521.3. ¹H NMR (400 MHz, DMSO-*d*₆) δ 11.18 (s, 1H), 10.07 (s, 1H), 8.64 (d, *J=* 8.0 Hz, 1H), 7.84 (t, *J=* 5.4 Hz, 1H), 7.67 (d, *J* = 8.9 Hz, 1H), 7.57 (t, *J=* 8.0 Hz, 1H), 7.49 (d, *J=* 7.9 Hz, 1H), 7.31 (t, *J=* 2.1 Hz, 1H), 7.01 - 6.94 (m, 2H), 5.76 (d, *J* = 15.9 Hz, 1H), 4.96 (d, *J* = 5.9 Hz, 1H), 4.82 (dt, *J* = 14.3, 6.5 Hz, 1H), 3.76 (q, *J* = 6.2 Hz, 2H), 3.66 (t, *J* = 5.6 Hz, 2H), 3.32 - 3.28 (m, 2H), 3.15 (d, *J* = 11.4 Hz, 2H), 2.87 - 2.70 (m, 2H), 2.21 - 2.09 (m, 1H), 1.82 - 1.66 (m, 2H), 1.66 - 1.47 (m, 2H) ppm.

### Example 16:

### N-(6,6-dioxo-3-oxo-6-thia-4,7-diaza-1(1,4)-piperidin-2(1,2),5(1,3)-diphenylhetero cyclodecane-2⁵-yl)-2-hydroxy-1-ethanesulfonamide

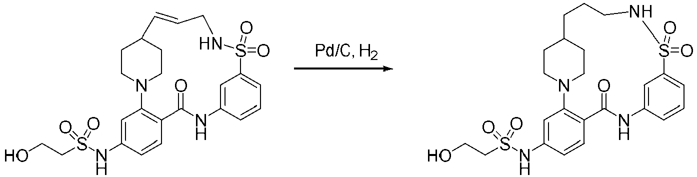

**Step 1:** At 20°C, (*E*)-N-(6,6-dioxo-3-oxo-6-thia-4,7-diaza-1(1,4)-piperidin-2(1,2),5(1,3)-diphenylheter ocyclodec-9-en-2⁵-yl)-2-hydroxy-1-ethanesulfonamide (30 mg, 0.58 mmol) and 10% palladium/carbon (30 mg, 100% w/w) were dissolved in methanol (10.00 mL), and formic acid (0.20 g, 3.34 mmol) was added. The mixture was purged with hydrogen and stirred at 20°C for 24 hours to complete the reaction. The reaction solution was concentrated and purified by flash column chromatography (silica, 70-95% ethyl acetate/petroleum ether) and preparative HPLC (C18, 4-44% gradient of water (aqueous ammonia + ammonium bicarbonate)/acetonitrile) to give N-(6,6-dioxo-3-oxo-6-thia-4,7-diaza-1(1,4)-piperidin-2(1,2),5(1,3)-diphenylheterocyc lodecane-2⁵-yl)-2-hydroxy-1-ethanesulfonamide (17 mg. 0.33 mmol, yield: 57.4%) as a white solid. LCMS (ESI): [M+H]⁺ =523.1. ¹H NMR (400 MHz, DMSO-*d₆*) δ ppm 10.77 (s, 1H), 10.31 - 9.66 (m, 1H), 8.60 (br d, *J=* 8.4 Hz, 1H), 7.70 (s, 1H), 7.63 (t, *J* = 8.0 Hz, 1H), 7.58 (d, *J =* 8.8 Hz, 1H), 7.49 - 7.38 (m, 2H), 7.03 - 6.92 (m, 2H), 4.98 (br s, 1H), 3.76 (t, *J* = 6.4 Hz, 2H), 3.32 - 3.29 (m, 2H), 3.11 (br d, *J* = 10.4 Hz, 2H), 2.79 (br d, *J* = 4.4 Hz, 2H), 2.59 (br t, *J* = 10.4 Hz, 2H), 1.61 - 1.28 (m, 9H) ppm.

### Example 17:

### N-(6,6-dioxo-9-oxo-6-thia-8-aza-5(1,4)-piperazin-2(1,4)-piperidin-1(1,2),7(1,3)-di phenylheterocyclononane-1³-yl)-2-hydroxyethane-1-sulfonamide

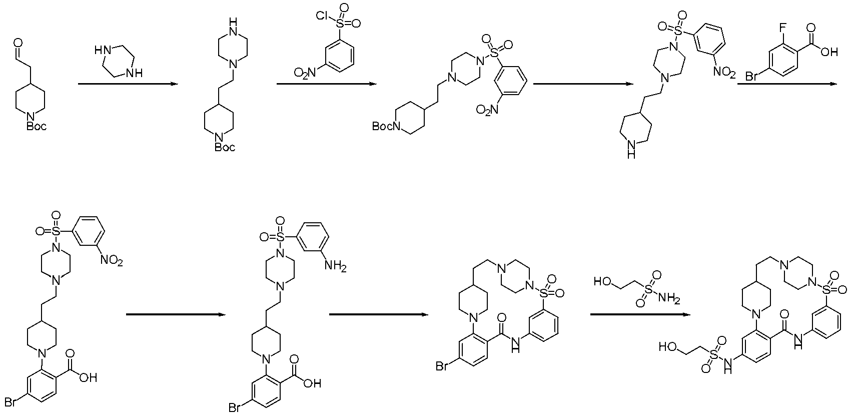

**Step 1:** N-Boc-4-piperidineacetaldehyde (9.00 g, 39.60 mmol) was dissolved in 1,2-dichloroethane (100.00 mL). The mixture was added dropwise to a solution containing piperazine (17.10 g, 197.98 mmol), sodium cyanoborohydride (3.70 g, 59.39 mmol), N,N-diisopropylethylamine (15.70 g, 118.79 mmol) and dichloroethane (100.00 mL). The resulting mixture was stirred at 20°C for 12 hours. The reaction solution was poured into water (200 mL) and extracted with ethyl acetate (200 mL × 3). The organic layer was dried over anhydrous sodium sulfate, then filtered and concentrated, and purified by column chromatography (silica gel, 0-30% gradient of methanol/dichloromethane) to give tert-butyl 4-(2-(piperazin-1-yl)ethyl)piperidine-1-carboxylate (2.30 g, 7.73 mmol, yield: 19.5%) as a white solid. LCMS (ESI): [M-55]⁺ = 298.3.

**Step 2:** A solution containing tert-butyl 4-(2-piperazinylethyl)piperidinecarboxylate (2.30 g, 7.73 mmol), 3-nitrobenzenesulfonyl chloride (2.10 g, 9.28 mmol), N,N-diisopropylethylamine (5.51 g, 38.66 mmol) and tetrahydrofuran (30.00 mL) was stirred at 20°C for 1 hour. The reaction solution was poured into water (30 mL) and extracted with ethyl acetate (30 mL × 3). The organic layer was dried over anhydrous sodium sulfate, then filtered and concentrated, and purified by column chromatography (silica gel, 0-50% gradient of ethyl acetate/petroleum ether) to give tert-butyl 4-(2-(4-(3-nitrophenyl)sulfonyl)piperazin-1-yl)ethyl)piperidine-1-carboxylate (1.80 g, 3.73 mmol, yield: 48.2%) as a white solid. LCMS (ESI): [M+Na]⁺ = 505.3.

**Step 3:** A reaction solution containing tert-butyl 4-(2-(4-(3-nitrophenyl)sulfonyl)piperazin-1-yl)ethyl)piperidine-1-carboxylate (1.80 g, 3.73 mmol) and a hydrochloric acid/ethyl acetate solution (10.00 mL, 4.0 M) was stirred at 20°C for 1 hour and concentrated to give 1-(3-nitrophenyl)sulfonyl)-4-(2-(piperidin-4-yl)ethyl)piperazine (1.60 g, crude) as a white solid. LCMS (ESI): [M+H]⁺ = 383.3.

**Step 4:** A solution containing 1-(3-nitrophenyl)sulfonyl)-4-(2-(piperidin-4-yl)ethyl)piperazine (1.30 g, 3.40 mmol), 4-bromo-2-fluorobenzoic acid (1.10 g, 5.10 mmol), potassium carbonate (1.40 g, 10.20 mmol) and dimethyl sulfoxide (50.00 mL) was stirred at 100°C for 12 hours. The reaction solution was poured into water (100 mL), a hydrochloric acid/ethyl acetate solution (5 mL) was added to adjust the pH to about 7, and the mixture was extracted with ethyl acetate (30 mL × 3). The organic layer was dried over anhydrous sodium sulfate, then filtered and concentrated, and purified by column chromatography (silica gel, 0-25% gradient of methanol/dichloromethane) to give 4-bromo-2-(4-(2-(4-(3-nitrophenyl)sulfonyl)piperazin-1-yl)ethyl)piperidin-1-yl)benzo ic acid (760 mg, 1.31 mmol, yield: 38.5%) as a yellow oil. LCMS (ESI): [M+H]⁺ = 581.3.

**Step 5:** A solution containing 4-bromo-2-(4-(2-(4-(3-nitrophenyl)sulfonyl)piperazin-1-yl)ethyl)piperidin-1-yl)benzo ic acid (760 mg, 1.31 mmol), iron powder (369 mg, 6.54 mmol), ammonium chloride (353 mg, 6.54 mmol), ethanol (20.00 mL) and water (5.00 mL) was stirred at 60°C for 1 hour. The reaction solution was filtered and extracted with ethyl acetate (30 mL × 3). The organic layer was dried over anhydrous sodium sulfate, then filtered and concentrated to give a crude product, which was purified by column chromatography (C18, 0-100% gradient of acetonitrile/water) to give 2-(4-(2-(4-((3-aminophenyl)sulfonyl)piperazin-1-yl)ethyl)piperidin-1-yl)-4-bromoben zoic acid (150 mg, 0.27 mmol, yield: 20.8%) as a white solid. LCMS (ESI): [M+H]⁺ = 551.2.

**Step 6:** 2-(4-(2-(4-((3-aminophenyl)sulfonyl)piperazin-1-yl)ethyl)piperidin-1-yl)-4-bromoben zoic acid (150 mg, 0.27 mmol) was dissolved in dichloromethane (10.00 mL). The mixture was added dropwise to a solution containing 2-chloro-1-methylpyridinium iodide (106 mg, 0.41 mmol), N,N-diisopropylethylamine (179 mg, 1.36 mmol) and dichloromethane (75.00 mL) and was stirred at 20°C for 12 hours. The reaction mixture was concentrated and purified by column chromatography (silica gel, 0-100% gradient of ethyl acetate/petroleum ether) to give 1⁵-bromo-6-thia-8-aza-5(1,4)-piperazin-2(1,4)-piperidin-1(1,2),7(1,3)-dipheny1hetero cyclonon-9-one-6,6-dioxide (50 mg, 0.094 mmol, yield: 34.5%) as a white solid. LCMS (ESI): [M+H]⁺= 533.2.

**Step 7:** A solution containing 1⁵-bromo-6-thia-8-aza-5(1,4)-piperazin-2(1,4)-piperidin-1(1,2),7(1,3)-diphenylhetero cyclonon-9-one-6,6-dioxide (50 mg, 0.094 mmol), 2-hydroxyethanesulfonamide (24 mg, 0.19 mmol), potassium phosphate (61 mg, 0.28 mmol), 2-di-tert-butylphosphino-2',4',6'-triisopropylbiphenyl (4 mg, 0.0094 mmol), tris(dibenzylideneacetone)dipalladium (9 mg, 0.0094 mmol) and 1,4-dioxane (5.00 mL) was stirred at 100°C for 8 hours under nitrogen atmosphere. The reaction solution was poured into water (30 mL) and extracted with ethyl acetate (30 mL × 3). The organic layer was dried over anhydrous sodium sulfate, then filtered and concentrated, and purified by column chromatography (silica gel, 0-25% gradient of methanol/dichloromethane; C18, 0-100% gradient of acetonitrile/water) to give *N*-(6,6-dioxo-9-oxo-6-thia-8-aza-5(1,4)-piperazin-2(1,4)-piperidin-1(1,2),7(1,3)-diphe nylheterocyclononane-1⁵-yl)-2-hydroxyethane-1-sulfonamide (2.5 mg, 0.0043 mmol, yield: 4.6%). LCMS (ESI): [M+H]⁺ = 578.30.

### Example 18:

### N-(1⁴,1⁴-difluoro-2⁶-methyl-4-oxo-3-aza-2(2,4)-pyrimidin-1(1,3),6(1,4)-dipiperidi n-5(1,2)-benzeneheterocyclonon-8-en-5⁴-yl)-2-hydroxyethane-1-sulfonamide

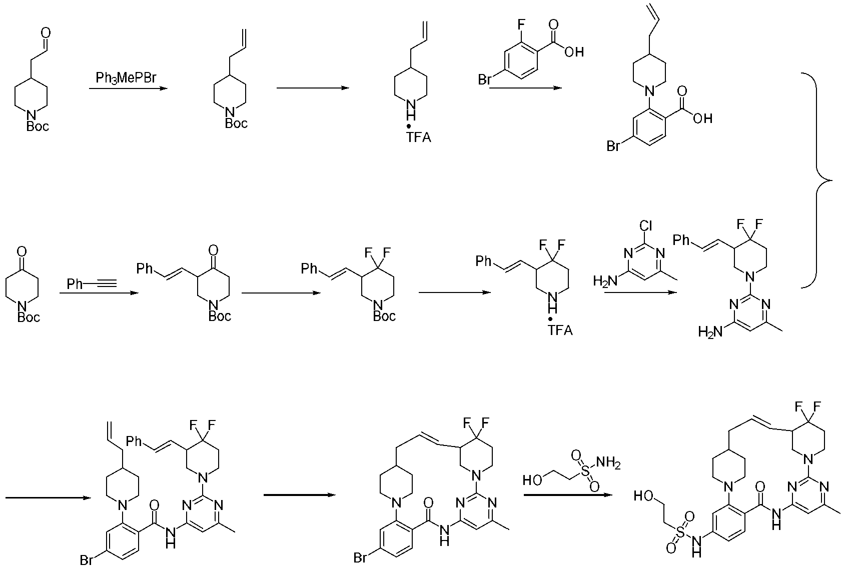

**Step 1:** Under nitrogen atmosphere at 20°C, methyltriphenylphosphonium bromide (24.06 g, 66.00 mmol) was dissolved in dry tetrahydrofuran (350.00 mL), the mixture was cooled to 0°C, and then a 1.0 M lithium bis(trimethylsilyl)amide solution in tetrahydrofuran (66.00 mL, 66.00 mmol) was slowly added and stirred for 1 hour. To the reaction system was added a solution of N-Boc-4-piperidineacetaldehyde (10.00 g, 44.00 mmol) in tetrahydrofuran (20.00 mL). The temperature was raised to 20°C and the mixture was stirred for 16 hours. After the reaction was complete, the reaction was quenched with a saturated sodium bicarbonate solution (100 mL) and the mixture was extracted with ethyl acetate (200 mL × 3). The combined organic layer was dried, filtered and concentrated to give a crude product, which was purified by flash column chromatography (silica, 0-10% ethyl acetate/petroleum ether) to give tert-butyl 4-allylpiperidine-1-carboxylate (10.00 g, 66.00 mmol, yield: 100.0%) as a colorless liquid.

**Step 2:** At 20°C, tert-butyl 4-allylpiperidine-1-carboxylate (10.00 g, 66.00 mmol) was dissolved in dichloromethane (200.00 mL), then trifluoroacetic acid (10.02 mL, 131.81 mmol) was added and the mixture was stirred at 20°C for 16 hours. After the reaction was complete, the solvent and excess trifluoroacetic acid were removed to give 4-allylpiperidine trifluoroacetate (about 15.50 g, crude). The crude product was then dissolved in dimethyl sulfoxide (200.00 mL), then potassium carbonate (30.98 g, 219.68 mmol) and 4-bromo-2-fluorobenzoic acid (9.62 g, 43.94 mmol) were added and the mixture was heated to 140°C and stirred for 48 hours. After the reaction was complete, the mixture was cooled naturally, then the reaction was quenched with cold water (100 mL), and 1*N* hydrochloric acid was added to adjust the pH of the solution to 6-7. The mixture was extracted with ethyl acetate (200 mL × 3). The combined organic layer was washed with saturated sodium chloride solution (100 mL), then dried, filtered and concentrated to give a crude product, which was purified by flash column chromatography (silica, 0-20% ethyl acetate/petroleum ether) to give 4-bromo-2-(4-allylpiperidinyl)benzoic acid (11.50 g, 35.15 mmol, yield: 80.3%) as an orange solid. ¹H NMR (400 MHz, Chloroform-d) δ 8.14 (d, *J=* 8.3 Hz, 1H), 7.61 - 7.48 (m, 2H), 5.85 - 5.68 (m, 1H), 5.14 - 4.98 (m, 2H), 3.19 - 3.05 (m, 2H), 2.91 (td, *J* = 11.7, 2.5 Hz, 2H), 2.11 (t, *J* = 6.8 Hz, 2H), 2.07 - 1.99 (m, 1H), 2.00 - 1.86 (m, 2H), 1.67 - 1.44 (m, 3H) ppm; LCMS (ESI): [M+H]⁺ =324.1.

**Step 3:** At 20°C, to a solution of N-(tert-butoxycarbonyl)-4-piperidone (600.00 g, 3011.29 mmol) and phenylacetylene (307.54 g, 3011.29 mmol) in dimethyl sulfoxide (6.00 L) was added potassium tert-butoxide (341.31 g, 3011.29 mmol). The reaction mixture was stirred in an oil bath at 100°C for 1 hour to complete the reaction. The reaction mixture was poured into cold water (1500 mL) to quench the reaction, and extracted with ethyl acetate (1500 mL × 2). The combined organic layer was concentrated to give a crude product, which was purified by flash column chromatography (silica, 0-10% ethyl acetate/petroleum ether) to give N-tert-butoxycarbonyl-3-((1E)-2-phenylethenyl)-4-piperidone (220.00 g, 729.94 mmol, yield: 24.2%) as a yellow oil.

**Step 4:** At -40°C, to a solution of N-tert-butoxycarbonyl-3-((1E)-2-phenylethenyl)-4-piperidone (100.00 g, 33.81 mmol) in dichloromethane (1800.00 mL) was added diethylaminosulfur trifluoride (160.45 g, 995.41 mmol). The temperature was raised to 0°C and the mixture was stirred for 5 hours to complete the reaction. The reaction was quenched with a sodium bicarbonate solution (1000 mL) and the reaction solution was extracted with dichloromethane (1000 mL × 3). The organic layer was dried, filtered and concentrated to give a crude product, which was purified by flash column chromatography (silica, 0-8% ethyl acetate/petroleum ether) to give N-tert-butoxycarbonyl-3-((1*E*)-2-phenylethenyl)-4,4-difluoropiperidine (33.00 g, 102.03 mmol, yield: 30.8%) as a yellow oil.

**Step 5:** At 20°C, to a solution of N-tert-butoxycarbonyl-3-((1*E*)-2-phenylethenyl)-4,4-difluoropiperidine (10.00 g, 30.92 mmol) in dichloromethane (40.00 mL) was added trifluoroacetic acid (8.00 mL). The reaction mixture was stirred for 16 hours, and concentrated under reduced pressure to give 3-((1E)-2-phenylethenyl)-4,4-difluoropiperidine (8.00 g, crude) as a yellow oil. 3-((1E)-2-phenylethenyl)-4,4-difluoropiperidine (8.00 g, crude) was dissolved in dimethyl sulfoxide (140.00 mL), and potassium carbonate (12.81 g, 92.71 mmol) and 2-chloro-6-methylpyrimidin-4-amine (4.44 g, 30.92 mmol) were added. The reaction mixture was stirred in an oil bath at 100°C for 16 hours to basically complete the reaction. The reaction mixture was poured into cold water (100 mL) to quench the reaction, and extracted with dichloromethane (300 mL × 3). The combined organic layer was washed with a sodium chloride solution (100 mL), dried over magnesium sulfate, filtered and concentrated to give a crude product, which was purified by flash column chromatography (silica, 0-35% ethyl acetate/petroleum ether) to give 2-(3-((1E)-2-phenylethenyl)-4,4-difluoropiperidin)-6-methylpyrimidin-4-amine (4.00 g, 9.55 mmol, yield: 39%) as a yellow solid.

**Step 6:** At 20°C, 2-(3-((1E)-2-phenylethenyl)-4,4-difluoropiperidin)-6-methylpyrimidin-4-amine (2.0 g, 6.05 mmol), 4-bromo-2-(4-allylpiperidinyl)benzoic acid (2.16 g, 6.66 mmol), N,N,N ' ,N ' -tetramethyl-O-(7-azabenzotriazol-1-yl)uronium hexafluorophosphate (11.74 g, 30.27 mmol) and 4-dimethylaminopyridine (3.77 g, 30.27 mmol) were dissolved in dry 1,2-dichloroethane (50.00 mL). The mixture was heated to 80°C and stirred for 48 hours. After the reaction was complete, the mixture was cooled naturally, then the reaction was quenched with cold water (50 mL), and the mixture was extracted with dichloroethane (50 mL × 3). The combined organic layer was dried, filtered and concentrated to give a crude product, which was purified by flash column chromatography (silica, 0-8% ethyl acetate/petroleum ether) to give 2-(4-allylpiperidin-1-yl)-4-bromo-N-(2-(4,4-difluoro-3-styrylpiperidin-1-yl)-6-methyl pyrimidin-4-yl)benzamide (1.05 g, 1.65 mmol, yield: 27.3%) as a white solid, LCMS (ESI): [M+H]⁺ =636.4/[M/2+H]⁺ =319.6.

**Step 7:** Under nitrogen atmosphere at 20°C, 2-(4-allylpiperidin-1-yl)-4-bromo-N-(2-(4,4-difluoro-3-styrylpiperidin-1-yl)-6-methyl pyrimidin-4-yl)benzamide (1.05 g, 1.65 mmol) was dissolved in dry dichloroethane (100.00 mL), Grubbs catalyst 2^{nd} generation (136 mg, 0.15 mmol) was added and the reaction was heated to reflux for 48 hours. After the reaction was complete, the concentrated reaction solution was purified by flash column chromatography (silica, 0-8% ethyl acetate/petroleum ether) to give 5⁴-bromo-1⁴,1⁴-difluoro-2⁶-methyl-3-aza-2(2,4)-pyrimidin-1(1,3),6(1,4)-dipiperidin-5 (1,2)-benzeneheterocyclonon-8-en-4-one (400 mg, 0.75 mmol, yield: 45.4%) as a white solid, LCMS (ESI): [M+H]⁺ = 532.3.

**Step 8:** Under nitrogen atmosphere at 20°C, 5⁴-bromo-1⁴,1⁴-difluoro-2⁶-methyl-3-aza-2(2,4)-pyrimidin-1(1,3),6(1,4)-dipiperidin-5 (1,2)-benzeneheterocyclonon-8-en-4-one (400 mg, 0.75 mmol), 2-hydroxyethylsulfonamide (288 mg, 2.25 mmol), potassium phosphate (488 mg, 2.25 mmol), cuprous iodide (146 mg, 0.75 mmol) and trans-N,N'-dimethyl-1,2-cyclohexanediamine (216 mg, 1.50 mmol) were dissolved in N,N-dimethylformamide (10.00 mL). The resulting reaction mixture was stirred at 100°C for 5 hours. After the reaction was complete, the reaction was quenched with cold water (20 mL). The mixture was extracted with ethyl acetate (20 mL × 3). The combined organic layer was washed with a sodium chloride solution (100 mL), dried over magnesium sulfate, filtered and concentrated to give a crude product, which was purified by flash column chromatography (silica, 50-70% ethyl acetate/petroleum ether) to give 430 mg of the product. The product was further purified by preparative HPLC (C18, 70-95% gradient of acetonitrile/water) to give *N*-(1⁴,1⁴-difluoro-2⁶-methyl-4-oxo-3-aza-2(2,4)-pyrimidin-1(1,3),6(1,4)-dipiperidin-5( 1,2)-benzeneheterocyclonon-8-en-5⁴-yl)-2-hydroxyethane-1-sulfonamide (80 mg, 0.14 mmol, yield: 18.5%) as a white solid. LCMS (ESI): [M+H]⁺ =577.4. ¹H NMR (400 MHz, DMSO-*d₆*) δ 12.98 (s, 0.66H), 10.84 (s, 0.32H), 10.23 (brs, 1H), 8.24 - 6.88 (m, 4H), 5.94 - 5.36 (m, 2H), 5.08 - 4.31 (m, 3H), 3.75 (t, *J* = 6.4 Hz, 2H), 3.36 (d, *J =* 6.3 Hz, 2H), 3.22 - 2.68 (m, 7H), 2.36 - 2.25 (m, 4H), 2.19 - 1.47 (m, 8H) ppm.

### Example 18A:

### N-((1³S,E)-1⁴,1⁴-difluoro-2¹-methyl-4-oxo-3-aza-2(2,4)-pyrimidin-1(1,3),6(1,4)-dip iperidin-5(1,2)-benzeneheterocyclononane-8-en-5⁴-yl)-2-hydroxyethane-1-sulfona mide

### Example 18B:

### N-(1³R,E)-1⁴,1⁴-difluoro-2⁶-methyl-4-oxo-3-aza-2(2,4)-pyrimidin-1(1,3),6(1,4)-dip iperidin-5(1,2)-benzeneheterocyclononane-8-en-5⁴-yl)-2-hydroxyethane-1-sulfona mide

### Example 18C:

### N-((1³S,Z)-1⁴,1⁴-difluoro-2⁶-methyl-4-oxo-3-aza-2(2,4)-pyrimidin-1(1,3),6(1,4)-dip iperidin-5(1,2)-benzeneheterocyclononane-8-en-5⁴-yl)-2-hydroxyethane-1-sulfona mide

### Example 18D:

### N-((1³R,Z)-1⁴,1⁴-difluoro-2⁶-methyl-4-oxo-3-aza-2(2,4)-pyrimidin-1(1,3),6(1,4)-di piperidin-5(1,2)-benzeneheterocyclononane-8-en-5⁴-yl)-2-hydroxyethane-1-sulfon amide

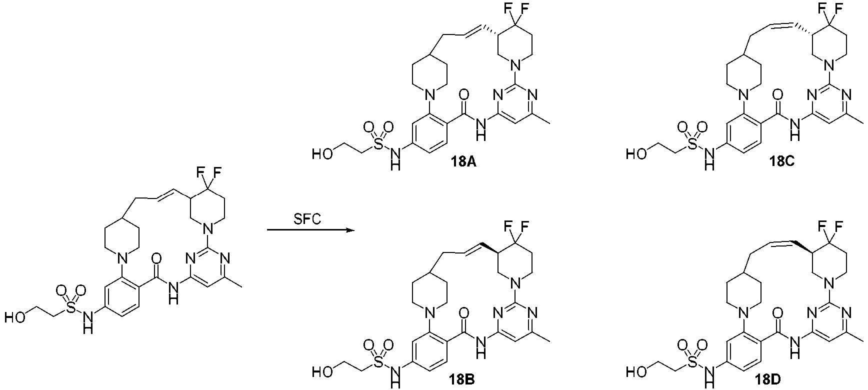

**Step** 1: N-(1⁴,1⁴-difluoro-2⁶-methyl-4-oxo-3-aza-2(2,4)-pyrimidin-1(1,3),(1,4)-dipiperidin-5(1, 2)-benzeneheterocyclononane-8-en-5⁴-yl)-2-hydroxyethane-1-sulfonamide (400 mg, 0.69 mmol) was purified by preparative HPLC (C18, 38%-78% gradient of water (aqueous ammonia + ammonium bicarbonate)/acetonitrile), then lyophilized, and separated by SFC (column: REGIS (s,s) WHELK-O1 (250 mm*30 mm, 5 um); mobile phase: CO₂-EtOH (0.1% NH₃H₂O); gradient: isocratic 45%; flow rate: 80 mL/min; column temperature: 40°C) to give **P1, P2** and **P34.**

**Example 18A: P1** was lyophilized and purified by preparative HPLC (C18, 32%-72% gradient of water (aqueous ammonia + ammonium bicarbonate)/acetonitrile) to give N-((1³S,E)-1⁴,1⁴-difluoro-2⁶-methyl-4-oxo-3-aza-2(2,4)-pyrimidin-1(1,3),6(1,4)-dipip eridin-5(1,2)-benzeneheterocyclononane-8-en-5⁴-yl)-2-hydroxyethane-1-sulfonamide (5.76 mg, 0.01 mmol, yield: 1.00%) as a purple solid. SFC (column: (S,S)-Whelk-0-1.8 50*4.6 mm I.D., 1.8 um; mobile phase: CO₂-EtOH (0.05% DEA); gradient: isocratic 45%; flow rate: 2.2 mL/min; column temperature: 35°C, retention time Rt=0.879); LCMS (ESI): [M+H]⁺ = 577.2; ¹H NMR (400 MHz, DMSO- *d₆*) δ 10.86 (s, 1H), 7.81 (d, *J=* 8.4 Hz, 1H), 7.42 (s, 1H), 7.04 (d, *J=* 1.6 Hz, 1H), 6.99 (dd, *J* = 1.6, 8.4 Hz, 1H), 5.83 (ddd, *J* = 6.4, 9.2, 15.6 Hz, 1H), 5.46 (dd, *J* = 8.8, 15.2 Hz, 1H), 5.00 - 4.93 (m, 1H), 4.79 (br d, *J* = 13.6 Hz, 1H), 3.75 (t, *J* = 6.4 Hz, 2H), 3.27 (br s, 2H), 3.20 (br d, *J=* 12.4 Hz, 1H), 3.02 - 2.78 (m, 4H), 2.56 - 2.53 (m, 1H), 2.48 - 2.41 (m, 1H), 2.32 - 2.19 (m, 4H), 2.18 - 1.99 (m, 2H), 1.89 (br dd, *J= 9.6,* 12.8 Hz, 1H), 1.81 - 1.62 (m, 4H), 1.25 (br d, *J* = 11.2 Hz, 1H) ppm

**Example 18B: P2** was lyophilized to give N-(1³R,E)-1⁴,1⁴-difluoro-2⁶-methyl-4-oxo-3-aza-2(2,4)-pyrimidin-1(1,3),6(1,4)-dipipe ridin-5(1,2)-benzeneheterocyclononane-8-en-5⁴-yl)-2-hydroxyethane-1-sulfonamide (16.45 mg, 0.03 mmol, yield: 4.00%) as a yellow solid. SFC (column: (S,S)-Whelk-0-1.8 50*4.6 mm I.D., 1.8 µm, mobile phase: CO₂-EtOH (0.05% DEA), gradient: isocratic 45%, flow rate: 2.2 mL/min, column temperature: 35°C, retention time Rt=1.016). LCMS (ESI): [M+H]⁺ = 577.2. ¹H NMR (400 MHz, DMSO- *d₆*) δ 10.90 (s, 1H), 10.27 (s, 1H), 7.87 (d, *J=* 8.8 Hz, 1H), 7.48 (s, 1H), 7.12 (d, *J* = 1.6 Hz, 1H), 7.07 (dd, *J* = 1.6, 8.4 Hz, 1H), 5.95 - 5.82 (m, 1H), 5.51 (dd, *J* = 8.8, 15.6 Hz, 1H), 5.02 (br d, *J=* 10.0 Hz, 2H), 4.85 (br d, *J=* 13.2 Hz, 1H), 3.81 (br t, *J* = 6.0 Hz, 2H), 3.37 - 3.30 (m, 2H), 3.25 (br d, *J=* 12.4 Hz, 1H), 3.08 - 2.83 (m, 4H), 2.62 - 2.58 (m, 1H), 2.53 - 2.47 (m, 1H), 2.37 - 2.23 (m, 4H), 2.20 - 2.03 (m, 2H), 1.98 - 1.90 (m, 1H), 1.87 - 1.78 (m, 2H), 1.74 - 1.66 (m, 2H), 1.32 - 1.29 (m, 1H) ppm

P34 was lyophilized and separated by SFC (column: DAICEL CHIRALPAK IG (250 mm*30 mm, 10 um); mobile phase: CO₂-MeCN/EtOH (0.1%NH₃H₂O); gradient: isocratic 60%; flow rate: 80 mL/min; column temperature: 40°C) to give P3 and P4.

**Example 18C:** P3 was lyophilized to give N-((1³S,Z)-1⁴,1⁴-difluoro-2⁶-methyl-4-oxo-3-aza-2(2,4)-pyrimidin-1(1,3),6(1,4)-dipip eridin-5(1,2)-benzeneheterocyclononane-8-en-5⁴-yl)-2-hydroxyethane-1-sulfonamide (23.34 mg, 0.04 mmol, yield: 6.00%) as a white solid. SFC (column: Chiralpak IG 50*4.6 mm I.D., 3 um; mobile phase: CO₂-EtOH (0.05% DEA); gradient: isocratic 40%; flow rate: 4 mL/min; column temperature: 35°C; retention time Rt=2.977); LCMS (ESI): [M+H]⁺ = 577.3. ¹H NMR (400 MHz, DMSO-*d₆*) δ ppm 13.01 (br s, 1H), 8.02 (d, *J* = 8.8 Hz, 1H), 7.26 (s, 1H), 7.18 (d, *J=* 1.6 Hz, 1H), 7.11 (dd, *J* = 2.0, 8.4 Hz, 1H), 5.77 (dt, *J=* 5.2, 11.2 Hz, 1H), 5.52 (br t, *J* = 10.0 Hz, 1H), 4.78 - 4.61 (m, 1H), 4.55 - 4.39 (m, 1H), 3.75 (t, *J=* 6.8 Hz, 2H), 3.22 - 2.92 (m, 6H), 2.78 (q, *J* = 12.4 Hz, 2H), 2.55 - 2.52 (m, 1H), 2.35 - 2.28 (m, 4H), 2.18 - 1.93 (m, 4H), 1.90 - 1.79 (m, 2H), 1.76 - 1.66 (m, 1H), 1.62 - 1.49 (m, 1H) ppm

**Example 18D:** P4 was lyophilized to give N-((1³R,Z)-1⁴,1⁴-difluoro-2⁶-methyl-4-oxo-3-aza-2(2,4)-pyrimidin-1(1,3),6(1,4)-dipip eridin-5(1,2)-benzeneheterocyclononane-8-en-5⁴-yl)-2-hydroxyethane-1-sulfonamide (22.78 mg, 0.03 mmol, yield: 5.00%). SFC (column: Chiralpak IG 50*4.6 mm I.D., 3 um; mobile phase: CO₂-EtOH (0.05% DEA); gradient: isocratic 40%; flow rate: 4 mL/min; column temperature: 35°C; retention time Rt=1.682); LCMS (ESI): [M+H]⁺ = 577.3. ¹H NMR (400 MHz, DMSO-*d₆*) δ ppm 13.01 (br s, 1H), 8.03 (d, *J* = 8.4 Hz, 1H), 7.26 (s, 1H), 7.19 (d, J= 2.0 Hz, 1H), 7.12 (dd, *J* = 2.0, 8.8 Hz, 1H), 5.77 (dt, *J* = 5.2, 11.2 Hz, 1H), 5.52 (br t, *J* = 10.0 Hz, 1H), 4.70 (br d, *J* = 12.0 Hz, 1H), 4.47 (br d, *J= 12.4* Hz, 1H), 3.75 (t, *J* = 6.4 Hz, 2H), 3.24 - 2.92 (m, 5H), 2.78 (q, *J* = 12.4 Hz, 2H), 2.54 - 2.52 (m, 1H), 2.37 - 2.28 (m, 4H), 2.18 - 1.92 (m, 4H), 1.89 - 1.79 (m, 2H), 1.75 - 1.67 (m, 1H), 1.63 - 1.50 (m, 1H)

### Example 19:

### (E)-N-(1⁴,1⁴-difluoro-2⁶-methyl-4-oxo-3-aza-2(2,4)-pyrimidin-1(1,3),6(1,4)-dipipe ridin-5(1,2)-benzeneheterocyclonon-7-en-5⁴-yl)-2-hydroxyethane-1-sulfonamide

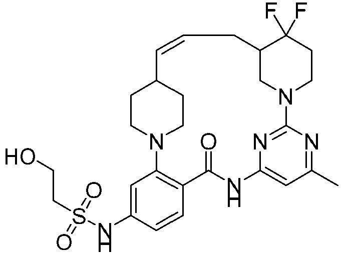

The preparations in Example 19 followed the synthetic route of Example 18. LCMS (ESI): [M+H]⁺ =577.4. ¹H NMR (400 MHz, DMSO-*d₆*) δ 12.78 (s, 1H), 10.28 (s, 1H), 8.07 (d, *J* = 8.7 Hz, 1H), 7.39 (s, 1H), 7.22 (d, *J= 2.2* Hz, 1H), 7.13 (dd, *J* = 8.7, 2.2 Hz, 1H), 5.71 - 5.55 (m, 1H), 5.37 (dt, *J=* 15.4, 7.1 Hz, 1H), 5.05 - 4.82 (m, 2H), 4.73 (d, *J* = 13.6 Hz, 1H), 3.76 (t, *J* = 6.4 Hz, 2H), 3.36 (t, *J* = 6.5 Hz, 2H), 3.20 - 2.94 (m, 3H), 2.93 - 2.73 (m, 2H), 2.50 - 2.39 (m, 2H), 2.33 - 2.22 (m, 1H), 2.27 (s, 3H), 2.18 - 2.07 (m, 1H), 2.02 - 1.62 (m, 7H) ppm.

### Example 20:

### N-(1⁴,1⁴-difluoro-2⁶-methyl-4-oxo-3-aza-2(2,4)-pyrimidin-1(1,3),6(1,4)-dipiperidin-5( 1,2)-benzeneheterocyclononane-5⁴-yl)-2-hydroxyethane-1-sulfonamide

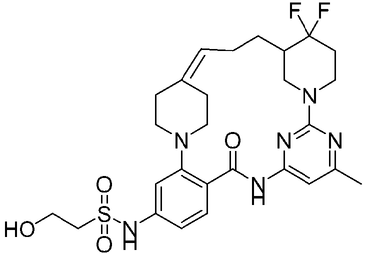

The preparations in **Example 20** followed the synthetic route of **Example 18.** LCMS (ESI): [M+H]⁺ = 577.5. ¹H NMR (400 MHz, DMSO) δ 13.89 (s, 1H), 10.28 (s, 1H), 8.11 (d, *J= 9.2* Hz, 1H), 7.32 (s, 1H), 7.21 - 7.16 (m, 2H), 5.35 (d, *J=* 11.2 Hz, 1H), 5.02-4.86 (m, 2H), 3.77 (t, *J* = 6.4 Hz, 2H), 3.40 - 3.36 (m, 2H), 3.24 - 3.09 (m, 3H), 2.97 - 2.86 (m, 2H), 2.78 - 2.54 (m, 4H), 2.30 (s, 3H), 2.13 - 2.05 (m, 2H), 1.89 - 1.74 (m, 2H), 1.37 (s, 1H), 1.27-1.22 (m, 4H) ppm.

### Example 21:

### N-(1⁴,1⁴-difluoro-2⁶-methyl-4-oxo-3-aza-2(2,4)-pyrimidin-1(1,3),6(1,4)-dipiperidi n-5(1,2)-benzeneheterocyclonon-9-en-5⁴-yl)-2-hydroxyethane-1-sulfonamide

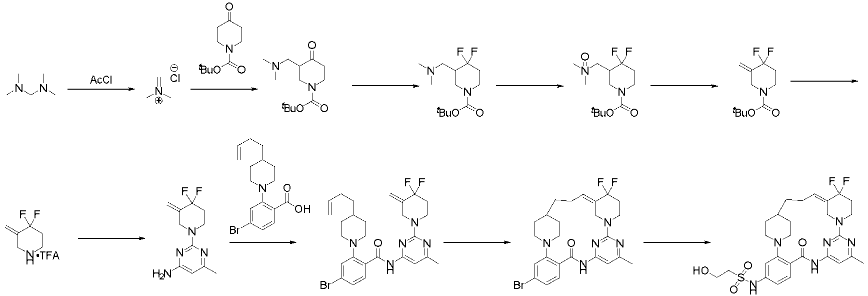

**Step 1:** Under nitrogen atmosphere at 0°C, to a solution of N,N,N',N'-tetramethyldiaminomethane (10.0 g, 97.87 mmol) in methyl tert-butyl ether (250 mL) was added dropwise acetyl chloride (7.84 g, 97.87 mmol). After the addition was completed, the mixture was stirred for 30 minutes and filtered. The filter cake was washed with methyl tert-butyl ether (25 mL) and evaporated to dryness under reduced pressure to give N-methyl-N-methylenemethanaminium iodide (9.0 g, 96.20 mmol, yield: 98.3%) as a white solid (highly hygroscopic).

**Step 2:** At 20°C, to a solution of N-(tert-butoxycarbonyl)-4-piperidone (5.00 g, 25.09 mmol) in acetonitrile (50.00 mL) was added N-methyl-N-methylenemethanaminium iodide (2.82 g, 30.11 mmol). The reaction was complete after 10 hours at 40°C. The reaction solution was concentrated, quenched with a saturated sodium bicarbonate solution (50 mL), and extracted with ethyl acetate (50 mL × 3). The combined organic layer was dried, filtered and concentrated to give N-tert-butoxycarbonyl-3-(dimethylamino)methyl)-4-piperidone (6.50 g, 25.36 mmol, quantitative yield, crude). LCMS (ESI): [M+H]⁺ =257.2.

**Step 3:** Under nitrogen atmosphere at 0°C, N-tert-butoxycarbonyl-3-(dimethylamino)methyl)-4-piperidone (6.50 g, 25.36 mmol) was dissolved in dichloromethane (120.00 mL), diethylaminosulfur trifluoride (12.91 g, 76.07 mmol) was added, and the temperature was raised to 25°C to react for 2 hours. The reaction was quenched with a saturated sodium bicarbonate solution (300 mL) and the mixture was extracted with dichloromethane (100 mL × 3). The combined organic layer was dried, filtered and concentrated to give N-tert-butoxycarbonyl-3-(dimethylamino)methyl)-4,4-difluoropiperidine (5.50 g, 19.76 mmol, yield: 77.9%, crude) as a yellow liquid, LCMS (ESI): [M+H]⁺ =279.2.

**Step 4:** Under nitrogen atmosphere at 20°C, N-tert-butoxycarbonyl-3-(dimethylamino)methyl)-4,4-difluoropiperidine (5.50 g, 19.76 mmol) was dissolved in dichloromethane (150.00 mL), m-chloroperoxybenzoic acid (6.02 g, 29.64 mmol) was added and the mixture was stirred for 2 hours. To the solution was added solid sodium carbonate (10.0 g). The mixture was stirred for 1 hour, filtered and concentrated to give a crude product. The product was purified by flash column chromatography (silica, 0-100% methanol/dichloromethane) to give 1-(1-(tert-butoxycarbonyl)-4,4-difluoropiperidin-3-yl)-N,N-dimethylmethanamine oxide (2.50 g, 8.49 mmol, yield: 43.0%) as a yellow liquid, LCMS (ESI): [M+H]⁺ =295.2.

**Step 5:** At 20°C, 1-(1-(tert-butoxycarbonyl)-4,4-difluoropiperidin-3-yl)-N,N-dimethylmethanamine oxide (2.50 g, 8.49 mmol) was dissolved in dimethyl sulfoxide (50 mL) and the mixture was stirred at 130°C for 2 hours. The reaction was quenched with water (100 mL). The reaction mixture was extracted with ethyl acetate (100 mL × 3) and washed with a saturated sodium chloride solution. The combined organic layer was dried, filtered and concentrated to give a crude product, which was purified by flash column chromatography (silica, 0-10% ethyl acetate/petroleum ether) to give N-tert-butoxycarbonyl-4,4-difluoro-3-methylenepiperidine (1.20 g, 5.14 mmol, yield: 60.5%) as a colorless liquid, LCMS (ESI): [M-55]⁺ =178.1.

**Step 6:** At 20°C, N-tert-butoxycarbonyl-4,4-difluoro-3-methylenepiperidine (1.20 g, 5.14 mmol) was dissolved in dichloromethane (50.00 mL), trifluoroacetic acid (6.00 mL) was added and the mixture was stirred at 20°C for 16 hours. After the reaction was complete, the solvent and excess trifluoroacetic acid were removed to give 4,4-difluoro-3-methylenepiperidine trifluoroacetate (about 2.50 g, crude). The crude product was then dissolved in N,N-dimethylformamide (20.00 mL), then potassium carbonate (3.55 g, 25.72 mmol) and 2-chloro-6-methylpyrimidin-4-amine (1.11 g, 7.72 mmol) were added and the mixture was heated to 130°C and stirred for 24 hours. After the reaction was complete, the mixture was cooled naturally. The reaction was quenched with cold water (100 mL). The mixture was extracted with ethyl acetate (100 mL × 3). The combined organic layer was washed with a saturated sodium chloride solution (100 mL), then dried, filtered and concentrated to give a crude product, which was purified by flash column chromatography (silica, 0-30% ethyl acetate/petroleum ether) to give 2-(4,4-difluoro-3-methylenepiperidin-1-yl)-6-methylpyrimidin-4-amine (450 mg, 1.87 mmol, yield: 36.4%) as a colorless liquid, LCMS (ESI): [M+H]⁺ =241.2.

**Step 7:** At 20°C, 2-(4,4-difluoro-3-methylenepiperidin-1-yl)-6-methylpyrimidin-4-amine (450 mg, 1.87 mmol), 4-bromo-2-(4-(3-buten-1-yl)piperidin-1-yl)benzoic acid (634 mg, 1.87 mmol), N,N,N',N'-tetramethyl-O-(7-azabenzotriazol-1-yl)uronium hexafluorophosphate (2.18 g, 5.62 mmol) and 4-dimethylaminopyridine (934 mg, 7.49 mmol) were dissolved in dry N,N-dimethylacetamide (10.00 mL). The mixture was heated to 60°C and stirred for 24 hours. After the reaction was complete, the mixture was cooled naturally. The reaction was quenched with cold water (50 mL). The mixture was extracted with ethyl acetate (50 mL × 3). The combined organic layer was washed with a saturated sodium chloride solution (100 mL), then dried, filtered and concentrated to give a crude product, which was purified by flash column chromatography (silica, 0-8% ethyl acetate/petroleum ether) to give 4-bromo-2-(4-(3-buten-1-yl)piperidin-1-yl)-*N*-(2-(4,4-difluoro-3-methylenepiperidin-1-yl)-6-methylpyrimidin-4-yl)benzamide (260 mg, 0.46 mmol, yield: 24.8%) as a white solid, LCMS (ESI): [M+H]⁺ =.

**Step 8:** Under nitrogen atmosphere at 20°C, 4-bromo-2-(4-(3-buten-1-yl)piperidin-1-yl)-*N*-(2-(4,4-difluoro-3-methylenepiperidin-1-yl)-6-methylpyrimidin-4-yl)benzamide (260 mg, 0.46 mmol) was dissolved in dry dichloroethane (50.00 mL), Grubbs catalyst 2^{nd} generation (50 mg, 0.058 mmol) was added and the reaction was heated to reflux for 48 hours. After the reaction was complete, the concentrated reaction solution was purified by flash column chromatography (silica, 0-8% ethyl acetate/petroleum ether) to give 5⁴-bromo-1⁴,1⁴-difluoro-2⁶-methyl-3-aza-2(2,4)-pyrimidin-1(1,3),6(1,4)-dipiperidin-5 (1,2)-benzeneheterocyclonon-9-en-4-one (240 mg, 0.45 mmol, yield: 97.2%) as a white solid, LCMS (ESI): [M+H]⁺ =532.3.

**Step 9:** Under nitrogen atmosphere at 20°C, 5⁴-bromo-1⁴,1⁴-difluoro-2⁶-methyl-3-aza-2(2,4)-pyrimidin-1(1,3),6(1,4)-dipiperidin-5 (1,2)-benzeneheterocyclonon-9-en-4-one (240 mg, 0.45 mmol), 2-hydroxyethylsulfonamide (475 mg, 3.72 mmol), potassium phosphate (1.07 g, 1.24 mmol), cuprous iodide (241 mg, 1.24 mmol) and trans-N,N'-dimethyl-1,2-cyclohexanediamine (356 mg, 2.48 mmol) were dissolved in N,N-dimethylformamide (5.00 mL). The resulting reaction mixture was stirred at 100°C for 5 hours. After the reaction was complete, the reaction was quenched with cold water (20 mL). The reaction mixture was extracted with ethyl acetate (20 mL × 3). The combined organic layer was washed with a sodium chloride solution (100 mL), dried over magnesium sulfate, filtered and concentrated to give a crude product. The product was purified by flash column chromatography (silica, 50-70% ethyl acetate/petroleum ether) and preparative HPLC (C18, 40%-80% gradient of water (aqueous ammonia+ ammonium bicarbonate)/acetonitrile) to give *N*-(1⁴,1⁴-difluoro-2⁶-methyl-4-oxo-3-aza-2(2,4)-pyrimidin-1(1,3),6(1,4)-dipiperidin-5( 1,2)-benzeneheterocyclonon-9-en-5⁴-yl)-2-hydroxyethane-1-sulfonamide (1.08 mg, 0.41 mmol, yield: 0.43%) as a white solid. LCMS (ESI): [M+H]⁺ =577.4.

### Example 23:

### N-(1⁴,1⁴-difluoro-2⁶-methyl-4-oxo-3-aza-2(2,4)-pyrimidin-1(1,3),6(1,4)-dipiperidi n-5(1,2)-benzeneheterocyclononane-5⁴-yl)-2-hydroxyethane-1-sulfonamide

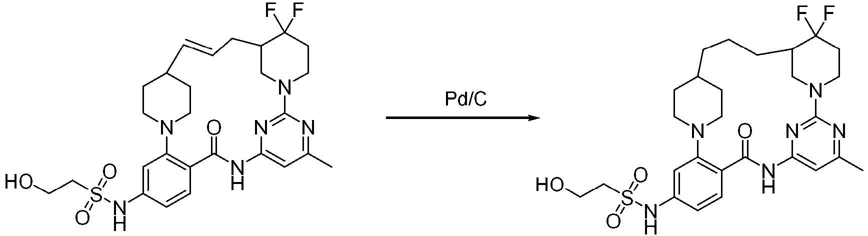

**Step** 1: At 20°C, (E)-*N*-(1⁴,1⁴-difluoro-2⁶-methyl-4-oxo-3-aza-2(2,4)-pyrimidin-l(1,3),6(1,4)-dipiperidi n-5(1,2)-benzeneheterocyclonon-7-en-5⁴-yl)-2-hydroxyethane-1-sulfonamide (120 mg, 0.21 mmol) and 10% palladium/carbon (120 mg, 100% w/w) were dissolved in methanol (10.00 mL), and formic acid (0.20 g, 3.34 mmol) was added. The mixture was purged with hydrogen and stirred at 20°C for 24 hours to complete the reaction. The mixture was concentrated and purified by flash column chromatography (silica, 30-95% ethyl acetate/petroleum ether) to give N-(1⁴,1⁴-difluoro-2⁶-methyl-4-oxo-3-aza-2(2,4)-pyrimidin-1(1,3),6(1,4)-dipiperidin-5( 1,2)-benzeneheterocyclononane-5⁴-yl)-2-hydroxyethane-1-sulfonamide (43 mg, 0.07 mmol, yield: 35.7%) as a white solid. ¹H NMR (400 MHz, DMSO-*d*₆) δ 11.41 (s, 1H), 10.22 (brs, 1H), 7.93 (d, *J* = 8.5 Hz, 1H), 7.50 (s, 1H), 7.10 (d, *J* = 2.1 Hz, 1H), 7.05 (dd, *J* = 8.6, 2.0 Hz, 1H), 4.95 (brs, 1H), 4.27 - 4.10 (m, 1H), 4.08 - 3.91 (m, 1H), 3.85 - 3.67 (m, 3H), 3.67 - 3.51 (m, 1H), 3.36 (t, *J* = 7.3 Hz, 2H), 3.21 (d, *J* = *11.0* Hz, 1H), 3.08 (d, *J* = 11.6 Hz, 1H), 2.77 (t, *J* = 11.3 Hz, 1H), 2.65 (t, *J* = 11.7 Hz, 1H), 2.31 (s, 3H), 2.10 - 1.82 (m, 5H), 1.76 - 1.31 (m, 9H) ppm.

### Example 23A:

### N-((1³S)-1⁴,1⁴-difluoro-2⁶-methyl-4-oxo-3-aza-2(2,4)-pyrimidin-1(1,3),6(1,4)-dipip eridin-5(1,2)-benzeneheterocyclononane-5⁴-yl)-2-hydroxyethane-1-sulfonamide

### Example 23B:

### N-((1³R)-1⁴,1⁴-difluoro-2⁶-methyl-4-oxo-3-aza-2(2,4)-pyrimidin-1(1,3),6(1,4)-dipi peridin-5(1,2)-benzeneheterocyclononane-5⁴-yl)-2-hydroxyethane-1-sulfonamide

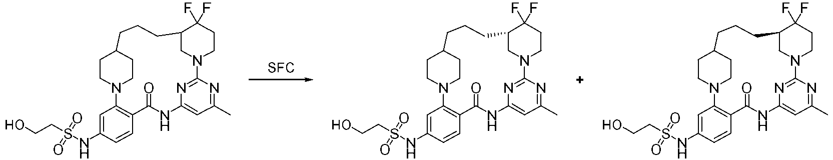

**Step 1:** N-(1⁴,1⁴-difluoro-2⁶-methyl-4-oxo-3-aza-2(2,4)-pyrimidin-1(1,3),6(1,4)-dipiperidin-5( 1,2)-benzeneheterocyclononane-5⁴-yl)-2-hydroxyethane-1-sulfonamide (50 mg, 0.086 mmol) was separated by SFC (column: REGIS (s, s) WHELK-O1 (250 mm*30 mm, 5 um); mobile phase: CO₂-EtOH (0.1% NH₃H₂O); gradient: isocratic 35%; flow rate: 80 mL/min; column temperature: 40°C) to give **Example 23A** and **Example 23B.**

**Example 23A:** SFC (column: (S,S)-Whelk-0-1.8 50¡Á4.6 mm I.D., 1.8 µm, mobile phase: CO₂-EtOH (0.05% DEA), gradient: isocratic 45%, flow rate: 2.2 mL/min, column temperature: 35°C, retention time Rt=1.108). LCMS (ESI): [M+H]⁺ = 579.2. ¹H NMR (400 MHz, DMSO-d6) δ ppm 11.63 - 11.22 (m, 1H), 7.89 (d, *J*= 8.4 Hz, 1H), 7.49 (s, 1H), 7.12 - 6.91 (m, 2H), 4.27 - 4.06 (m, 1H), 3.95 (br dd, *J*= 2.8, 12.0 Hz, 1H), 3.85 - 3.67 (m, 3H), 3.67 - 3.56 (m, 1H), 3.27 - 3.24 (m, 2H), 3.18 (br d, *J=* 9.8 Hz, 1H), 3.11 - 3.05 (m, 1H), 2.81 - 2.57 (m, 2H), 2.37 - 2.21 (m, 3H), 2.11 - 1.80 (m, 5H), 1.77 - 1.62 (m, 3H), 1.60 - 1.26 (m, 6H) ppm

**Example 23B:** SFC (column: (S,S)-Whelk-0-1.8 50¡Á4.6 mm I.D., 1.8 µm, mobile phase: CO₂-EtOH (0.05% DEA), gradient: isocratic 45%, flow rate: 2.2 mL/min, column temperature: 35°C, retention time Rt=1.226). LCMS (ESI): [M+H]⁺ = 579.2. ¹H NMR (400 MHz, DMSO-d6) δ ppm 11.74 - 11.24 (m, 1H), 7.88 (d, *J* = 8.8 Hz, 1H), 7.49 (s, 1H), 7.13 - 6.84 (m, 2H), 4.28 - 4.04 (m, 1H), 3.95 (br d, *J =* 10.0 Hz, 1H), 3.85 - 3.67 (m, 3H), 3.67 - 3.56 (m, 1H), 3.28 (br d, *J* = 6.8 Hz, 2H), 3.18 (br d, *J* = 10.4 Hz, 1H), 3.06 (br d, *J* = 11.6 Hz, 1H), 2.80 - 2.59 (m, 2H), 2.37 - 2.20 (m, 3H), 1.84 (br s, 5H), 1.70 (br s, 9H) ppm

### Example 24:

### (Z)-N-(1⁴,1⁴-difluoro-2⁶-methoxy-4-oxo-3-aza-2(2,4)-pyrimidin-1(1,3),6(1,4)-dipip eridin-5(1,2)-benzeneheterocyclonon-8-en-5⁴-yl)-2-hydroxyethane-1-sulfonamide

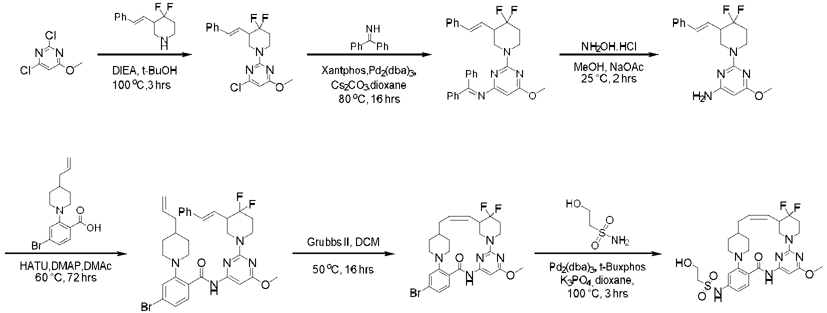

**Step 1:** 2,4-dichloro-6-methoxypyrimidine (1.50 g, 8.38 mmol) and (E)-4,4-difluoro-3-styrylpiperidine (0.88 g, 5.59 mmol) were dissolved in tert-butanol (30.0 mL), and diisopropylethylamine (5.42 g, 41.9 mmol) was added. The mixture was stirred at 100°C for 3 hours. The reaction mixture was diluted with water (100 mL) and extracted with ethyl acetate (200 mL × 2), and the organic phase was washed with saturated saline (300 mL), dried, filtered, and concentrated. The residue was purified by flash column chromatography (silica gel, 0-3% gradient of tetrahydrofuran/petroleum ether) to give (E)-4-chloro-2-(4,4-difluoro-3-styrylpiperidin-1-yl)-6-methoxypyrimidine (2.60 g, 7.12 mmol, yield: 85.0%) as yellow oil. LCMS (ESI): [M+H]⁺ = 366.0

**Step 2:** (E)-4-chloro-2-(4,4-difluoro-3-styrylpiperidin-1-yl)-6-methoxypyrimidine (2.10 g, 5.74 mmol) and diphenylmethanimine (1.59 g, 8.61 mmol) were dissolved in 1,4-dioxane (40.0 mL). Cesium carbonate (5.61 g, 17.2 mmol) and 4,5-bis(diphenylphosphine)-9,9-dimethylxanthene (0.66 g, 1.15 mmol) and tris(dibenzylideneacetone)dipalladium (0.53 g, 0.57 mmol) were added. The mixture was stirred at 80°C for 16 hours under nitrogen atmosphere. The reaction mixture was diluted with water (100 mL), extracted with ethyl acetate (200 mL × 2), and the organic phase was dried, filtered and concentrated. A brown oily residue was given and purified by flash column chromatography (silica gel, 0-3% gradient of tetrahydrofuran/petroleum ether) to give (E)-N-(2-(4,4-difluoro-3-styrylpiperidin-1-yl)-6-methoxypyrimidin-4-yl)-1,1-diphenyl methanimine (2.90 g, 5.74 mmol, crude) as a yellow oil. LCMS (ESI): [M+H]⁺ = 511.3

**Step 3:** (E)-N-(2-(4,4-difluoro-3-styrylpiperidin-1-yl)-6-methoxypyrimidin-4-yl)-1,1-diphenyl methanimine (2.90 g, 5.67 mmol) was dissolved in methanol (60.0 mL), sodium acetate (1.40 g, 17.0 mmol) and hydroxylamine hydrochloride (0.79 g, 11.3 mmol) were added and the mixture was stirred at 25°C for 2 hours. The reaction mixture was diluted with water (100 mL), extracted with ethyl acetate (200 mL × 2), and the organic phase was dried, filtered and concentrated. A brown oily residue was given and purified by flash column chromatography (silica gel, 0-10% gradient of tetrahydrofuran/petroleum ether) to give (E)-2-(4,4-difluoro-3-styrylpiperidin-1-yl)-6-methoxypyrimidin-4-amine (1.40 g, 3.46 mmol, yield: 61.0%) as a yellow oil. LCMS (ESI): [M+H]⁺ = 347.5. ¹H NMR (400 MHz, DMSO-*d₆*) δ 7.47 - 7.43 (m, 2H), 7.37 -7.33 (m, 2H), 7.29 - 7.25 (m, 1H), 6.68 (d, *J* = 16.0 Hz, 1H), 6.28 (br s, 2H), 6.23 (br dd, *J* = 8.4, 16.0 Hz, 1H), 5.14 (s, 1H), 4.54 - 4.45 (m, 2H), 3.72 (s, 3H), 3.24 (br t, *J* = 11.2 Hz, 2H), 2.89 - 2.74 (m, 1H), 2.17 - 2.07 (m, 1H), 1.98 - 1.83 (m, 1H) ppm

**Step 4:** (E)-2-(4,4-difluoro-3-styrylpiperidin-1-yl)-6-methoxypyrimidin-4-amine (1.48 g, 4.27 mmol) and 2-(4-allylpiperidin-1-yl)-4-bromobenzoic acid (1.39 g, 4.27 mmol) were dissolved in N,N-dimethylacetamide (28.0 mL), 4-dimethylaminopyridine (28.2 mg, 0.23 mmol) and O-(7-azabenzotriazole-1-yl)-N,N,N,N-tetramethyluronium hexafluorophosphate (4.06 g, 10.6 mmol) were added, and the mixture was stirred at 60°C for 72 h. The reaction mixture was diluted with water (50.0 mL), extracted with ethyl acetate (100 mL × 2), and the organic phase was dried, filtered and concentrated. The residue was purified by flash column chromatography (silica gel, 0-5% gradient of tetrahydrofuran/petroleum ether) to give (E)-2-(4-allylpiperidin-1-yl)-4-bromo-N-(2-(4,4-difluoro-3-styrylpiperidin-1-yl)-6-me thoxypyrimidin-4-yl)benzamide (1.30 g, 1.84 mmol, yield: 43.0%) as a yellow solid. LCMS (ESI): [M+H]⁺ = 654.0. ¹H NMR (400 MHz, CHLOROFORM-d) δ 12.35 (s, 1H), 8.02 (d, *J* = 8.4 Hz, 1H), 7.35 - 7.28 (m, 4H), 7.24 (t, *J* = 7.2 Hz, 2H), 7.20 - 7.09 (m, 2H), 7.08 - 7.04 (m, 1H), 6.57 (d, *J* = 16.0 Hz, 1H), 6.14 (dd, *J* = 8.4, 16.0 Hz, 1H), 5.63 (br dd, *J* = 8.4, 17.2 Hz, 1H), 4.94 - 4.86 (m, 2H), 4.57 - 4.49 (m, 2H), 3.84 (s, 3H), 3.43 - 3.27 (m, 2H), 3.13 - 3.06 (m, 2H), 2.82 - 2.64 (m, 3H), 2.14 - 2.02 (m, 1H), 1.93 (br t, *J =* 6.4 Hz, 2H), 1.77 (br d, *J =* 12.0 Hz, 2H), 1.63 - 1.53 (m, 2H), 1.44 - 1.36 (m, 1H) ppm

**Step 5:** (E)-2-(4-allylpiperidin-1-yl)-4-bromo-N-(2-(4,4-difluoro-3-styrylpiperidin-1-yl)-6-me thoxypyrimidin-4-yl)benzamide (100 mg, 0.15 mmol) was dissolved in anhydrous dichloromethane (80.0 mL), Grubbs catalyst 2^{nd} generation (13.2 mg, 0.02 mmol) was added and the mixture was stirred at 50°C for 16 hours under nitrogen atmosphere. The reaction mixture was concentrated under reduced pressure. The residue was purified by flash column chromatography (silica gel, 0-3% gradient of tetrahydrofuran/petroleum ether) to give (E)-5⁴-bromo-1⁴,1⁴-difluoro-2⁶-methoxy-3-aza-2(2,4)-pyrimidin-1(1,3),6(1,4)-dipiperi din-5(1,2)-benzeneheterocyclononane-8-en-4-one (30.0 mg, 0.05 mmol, yield: 35.5%) as a brown oil. LCMS (ESI): [M+H]⁺ = 549.8

**Step 6:** (E)-5⁴-bromo-1⁴,1⁴-difluoro-2⁶-methoxy-3-aza-2(2,4)-pyrimidin-1(1,3),6(1,4)-dipiperi din-5(1,2)-benzeneheterocyclononane-8-en-4-one (80.0 mg, 0.15 mmol) and 2-hydroxyethane-1-sulfonamide (21.9 mg, 0.18 mmol) were dissolved in 1,4-dioxane (1.00 mL), potassium phosphate (94.7 mg, 0.44 mmol), 2-di-tert-butylphosphino-2',4',6'-triisopropylbiphenyl (12.3 mg, 0.03 mmol) and tris(dibenzylideneacetone)dipalladium (13.6 mg, 0.01 mmol) were added and the mixture was stirred at 100°C for 3 hours under nitrogen atmosphere. The reaction mixture was diluted with water (50.0 mL), extracted with ethyl acetate (100 mL × 2), and the organic phase was dried, filtered and concentrated. The residue was purified by preparative HPLC (C18, 38%-78% gradient of water (formic acid)/acetonitrile) to give (Z)-N-(1⁴,1⁴-difluoro-2⁶-methoxy-4-oxo-3-aza-2(2,4)-pyrimidin-1(1,3),6(1,4)-dipiperi din-5(1,2)-benzeneheterocyclonon-8-en-5⁴-yl)-2-hydroxyethane-1-sulfonamide (30.07 mg, 0.05 mmol, yield: 34.0%) as a white solid. LCMS (ESI): [M+H]⁺ = 593.3. ¹H NMR (400 MHz, DMSO-*d₆*) δ 13.10 - 10.62 (m, 1H), 8.06 - 7.74 (m, 1H), 7.22 - 6.97 (m, 2H), 6.94 - 6.74 (m, 1H), 5.90 - 5.71 (m, 1H), 5.56 - 5.38 (m, 1H), 4.93 - 4.44 (m, 2H), 3.87 (s, 3H), 3.76 (t, *J* = 6.4 Hz, 2H), 3.31 - 3.08 (m, 4H), 3.07 - 2.88 (m, 3H), 2.87 - 2.67 (m, 2H), 2.32 (br dd, *J* = 4.4, 11.2 Hz, 1H), 2.15 - 1.54 (m, 8H) ppm.

### Example 25:

### 2-Hydroxy-N-((1²S)-2⁶-methyl-4-oxo-3-aza-1(4,2)-morpholin-2(2,4)-pyrimidin-6( 1,4)-piperidin-5(1,2)-benzeneheterocyclonon-8-en-5⁴-yl)ethane-1-sulfonamide

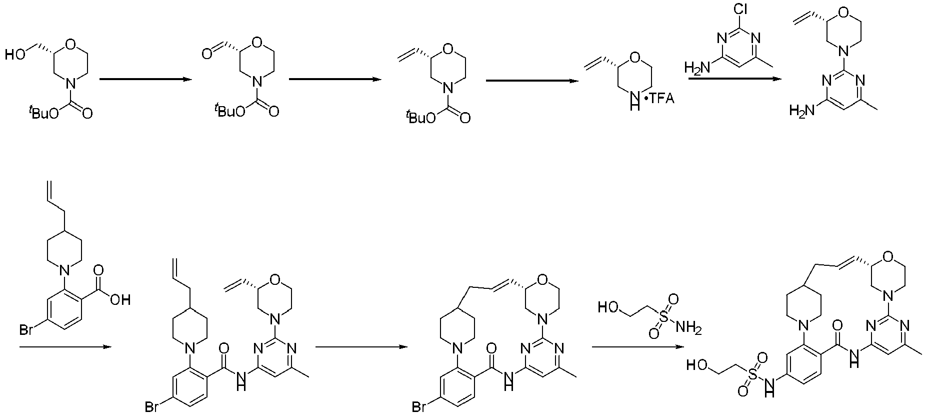

**Step 1:** At 20°C, (R)-N-tert-butoxycarbonyl-2-hydroxymethylmorpholine (5.0 g, 23.01 mmol) was dissolved in dry dichloromethane (150.00 mL). The solution was cooled to 0°C, then Dess-Martin periodinane (11.83 g, 27.62 mmol) was added portionwise, the temperature was raised to 20°C and the mixture was stirred for 1 hour. After the reaction was complete, saturated sodium bicarbonate solution (100 mL) and sodium sulfite solution (100 mL) were added to quench the reaction and the mixture was extracted with dichloromethane (200 mL × 3). The combined organic layer was dried, filtered and concentrated to give (*R*)-N-tert-butoxycarbonyl-2-formylmorpholine (3.70 g, 17.19 mmol, yield: 74.7%, crude) as a colorless liquid.

**Step 2:** Under nitrogen atmosphere at 20°C, methyltriphenylphosphonium bromide (5.99 g, 35.82 mmol) was dissolved in dry tetrahydrofuran (100.00 mL), the mixture was cooled to 0°C, and then a 1.0 M lithium bis(trimethylsilyl)amide solution in tetrahydrofuran (35.82 mL, 35.82 mmol) was slowly added and stirred for 1 hour. To the reaction system was added a solution of crude (*R*)-N-tert-butoxycarbonyl-2-formylmorpholine (3.70 g, 17.19 mmol) in tetrahydrofuran (10.00 mL). The temperature was raised to 20°C and the mixture was stirred for 16 hours. After the reaction was complete, the reaction was quenched with a saturated sodium bicarbonate solution (100 mL) and the mixture was extracted with ethyl acetate (100 mL × 3). The combined organic layer was dried, filtered and concentrated to give a crude product, which was purified by flash column chromatography (silica, 0-10% ethyl acetate/petroleum ether) to give (*S*)-N-tert-butoxycarbonyl-2-vinylmorpholine (1.10 g, 5.16 mmol, yield: 30.0%) as a colorless liquid. ¹H NMR (400 MHz, Chloroform-d) δ 5.79 (ddd, *J* = 17.4, 10.7, 5.4 Hz, 1H), 5.35 (dt, *J* = 17.4, 1.5 Hz, 1H), 5.22 (dt, *J* = 10.7, 1.4 Hz, 1H), 4.00 - 3.76 (m, 4H), 3.55 (td, *J* = 11.6, 2.8 Hz, 1H), 2.94 (t, *J* = 12.6 Hz, 1H), 2.67 (s, 1H), 1.46 (s, 9H) ppm.

**Step 3:** At 20°C, (*S*)-N-tert-butoxycarbonyl-2-vinylmorpholine (1.10 g, 5.16 mmol) was dissolved in dichloromethane (50.00 mL), then trifluoroacetic acid (6.20 mL, 80.98 mmol) was added and the mixture was stirred at 20°C for 16 hours. After the reaction was complete, the solvent and excess trifluoroacetic acid were removed to give 2-vinylmorpholine trifluoroacetate (about 2.40 g, crude). The crude product was then dissolved in N,N-dimethylformamide (50.00 mL), then potassium carbonate (3.64 g, 25.79 mmol) and 2-chloro-6-methylpyrimidin-4-amine (1.11 g, 7.74 mmol) were added and the mixture was heated to 130°C and stirred for 48 hours. After the reaction was complete, the mixture was cooled naturally, and the reaction was quenched with cold water (100 mL). The mixture was extracted with ethyl acetate (100 mL × 3). The combined organic layer was washed with a saturated sodium chloride solution (100 mL), then dried, filtered and concentrated to give a crude product, which was purified by flash column chromatography (silica, 0-20% ethyl acetate/petroleum ether) to give (*S*)-6-methyl-2-(2-vinylmorpholine )pyrimidin-4-amine (1.14 g, 4.18 mmol, yield: 81.0%) as a white solid, LCMS (ESI): [M+H]⁺ =221.2.

**Step 4:** At 20°C, (*S*)-6-methyl-2-(2-vinylmorpholine)pyrimidin-4-amine (0.92 g, 4.18 mmol), 2-(4-allylmorpholin-1-yl)-4-bromobenzoic acid (1.49 g, 4.59 mmol), N,N,N',N'-tetramethyl-O-(7-azabenzotriazol-1-yl)uronium hexafluorophosphate (4.86 g, 12.53 mmol) and 4-dimethylaminopyridine (2.08 g, 16.71 mmol) were dissolved in dry N,N-dimethylacetamide (20.00 mL). The mixture was heated to 60°C and stirred for 24 hours. After the reaction was complete, the mixture was cooled naturally, and the reaction was quenched with cold water (50 mL). The mixture was extracted with ethyl acetate (50 mL × 3). The combined organic layer was washed with saturated sodium chloride solution (20 mL), then dried, filtered and concentrated to give a crude product, which was purified by flash column chromatography (silica, 0-10% ethyl acetate/petroleum ether) to give (*S*)-2-(4-allylmorpholin-1-yl)-4-bromo-*N*-(6-methyl-2-(2-vinylmorpholinyl)pyrimidin -4-yl)benzamide (1.00 g, 1.90 mmol, yield: 45.5%) as a yellow liquid, LCMS (ESI): [M+H]⁺=526.3.

**Step 5:** Under nitrogen atmosphere at 20°C, (*S*)-2-(4-allylmorpholin-1-yl)-4-bromo-N-(6-methyl-2-(2-vinylmorpholinyl)pyrimidin -4-yl)benzamide (1.00 g, 1.90 mmol) was dissolved in dry dichloromethane (100.00 mL), Grubbs catalyst 2^{nd} generation (82 mg, 0.095 mmol) was added and the reaction was heated to reflux for 36 hours. After the reaction was complete, the concentrated reaction solution was purified by flash column chromatography (silica, 0-10% ethyl acetate/petroleum ether) to give (1²S)-5⁴-bromo-2⁶-methyl-3-aza-1(4,2)-morpholin-2(2,4)-pyrimidin-6(1,4)-piperidin-5(1,2)-benzeneheterocyclonon-8-en-4-one (260 mg, 0.52 mmol, yield: 27.4%) as a white solid, LCMS (ESI): [M+H]⁺ = 499.3.

**Step 6:** Under nitrogen atmosphere at 20°C, (1²*S*)-5⁴-bromo-2⁶-methyl-3-aza-1(4,2)-morpholin-2(2,4)-pyrimidin-6(1,4)-piperidin-5(1,2)-benzeneheterocyclonon-8-en-4-one (260 mg, 0.52 mmol), 2-hydroxyethylsulfonamide (307 mg, 2.41 mmol), potassium phosphate (695 mg, 3.21 mmol), cuprous iodide (156 mg, 0.80 mmol) and trans-N,N'-dimethyl-1,2-cyclohexanediamine (231 mg, 1.61 mmol) were dissolved in N,N-dimethylformamide (10.00 mL). The resulting reaction mixture was stirred at 100°C for 5 hours. After the reaction was complete, the reaction was quenched with cold water (20 mL). The mixture was extracted with ethyl acetate (50 mL × 3). The combined organic layer was dried, filtered and concentrated to give a crude product. The product was purified by flash column chromatography (silica, 60-80% ethyl acetate/petroleum ether) to give 2-hydroxy-*N*-((1²S)-2⁶-methyl-4-oxo-3-aza-1(4,2)-morpholin-2(2,4)-pyrimidin-6(1,4) -piperidin-5(1,2)-benzeneheterocyclonon-8-en-5⁴-yl)ethane-1-sulfonamide (200 mg, 0.37 mmol, yield: 70.7%), Z/E=1:7, as a white solid, LCMS (ESI): [M+H]⁺ =543.4. ¹H NMR (400 MHz, DMSO-*d₆*) δ 12.97 (s, 0.15H), 10.82 (s, 0.85H), 10.25 (s, 0.15H), 10.17 (s, 0.85H), 8.03 (d, *J* = 8.7 Hz, 0.15H), 7.95 (s, 0.15H), 7.79 (d, *J* = 8.5 Hz, 0.85H), 7.34 (s, 0.85H), 7.22 - 7.11 (m, 0.3H), 7.07 - 6.95 (m, 1.70H), 5.96 - 5.47 (m, 2H), 4.93 (brs, 1H), 4.56 (t, *J* = 12.8 Hz, 2H), 4.45 - 4.18 (m, 1H), 3.86 - 3.53 (m, 3H), 3.67 - 3.55 (m, 1H), 3.31 - 3.21 (m, 2H), 3.14 (t, *J* = 6.9 Hz, 1H), 3.09 - 2.93 (m, 2H), 2.89 (s, 1H), 2.84 - 2.75 (m, 1H), 2.73 (s, 1H), 2.37 - 2.21 (m, 1H), 2.28 (s, 3H), 1.92 (dd, *J* = 13.4, 8.2 Hz, 1H), 1.82 - 1.49 (m, 4H), 1.33 - 1.15 (m, 1H) ppm.

### Example 26:

### 2-Hydroxy-N-((1²S)-2⁶-methyl-4-oxo-3-aza-1(4,2)-morpholin-2(2,4)-pyrimidin-6( 1,4)-piperidin-5(1,2)-benzeneheterocyclononane-5⁴-yl)ethane-1-sulfonamide

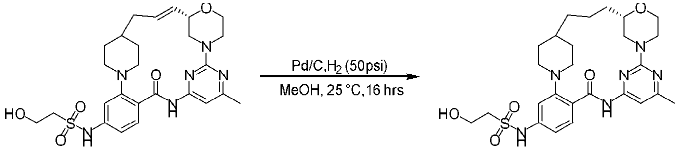

**Step 1:** To a solution of 2-hydroxy-N-((1²S)-2⁶-methyl-4-oxo-3-aza-1(4,2)-morpholin-2(2,4)-pyrimidin-6(1,4) -piperidin-5(1,2)-benzeneheterocyclonon-8-en-4-yl)ethane-1-sulfonamide (140 mg, 0.26 mmol) in methanol (10.00 mL) was added wet palladium on carbon (200 mg, mass fraction 10%). The reaction mixture was purged with argon three times and hydrogen three times, and stirred at 25°C for 16 hours under hydrogen (50 Psi) atmosphere. The reaction mixture was filtered under reduced pressure to remove palladium and then concentrated under reduced pressure to give a crude product. The residue was purified by preparative HPLC (C18, 22%-62% gradient of water (aqueous ammonia + ammonium bicarbonate)/acetonitrile) to give 2-hydroxy-N-((1²S)-2⁶-methyl-4-oxo-3-aza-1(4,2)-morpholin-2(2,4)-pyrimidin-6(1,4) -piperidin-5(1,2)-benzeneheterocyclononane-5⁴-yl)ethane-1-sulfonamide (52.2 mg, 0.09 mmol, yield: 35.0%) as a white solid. LCMS (ESI): [M+H]⁺ = 545.3. ¹H NMR (400 MHz, DMSO-*d₆*) δ 12.05 (s, 1H), 8.00 (d, *J =* 8.4 Hz, 1H), 7.36 (s, 1H), 7.13 (s, 1H), 7.07 (dd, *J* = 1.6, 8.4 Hz, 1H), 4.58 (br d, *J* = 12.8 Hz, 1H), 4.20 (br d, *J* = 12.8 Hz, 1H), 3.75 (t, *J* = 6.8 Hz, 2H), 3.50 - 3.43 (m, 1H), 3.42 - 3.33 (m, 5H), 3.12 (br d, *J* = 10.0 Hz, 2H), 3.03 (br dd, *J* = 8.0, 12.8 Hz, 1H), 2.77 (br t, *J=* 11.2 Hz, 1H), 2.67 (br t, *J* = 11.2 Hz, 1H), 2.28 (s, 3H), 2.01 - 1.84 (m, 2H), 1.80 - 1.70 (m, 2H), 1.62 - 1.37 (m, 7H) ppm.

### Example 27:

### N-(2⁶-methyl-4,9-dioxo-3,8-diaza-2(2,4)-pyrimidin-1(1,3),6(1,4)-dipiperidin-5(1,2) -benzeneheterocyclononane-5⁴-yl)-2-hydroxyethane-1-sulfonamide

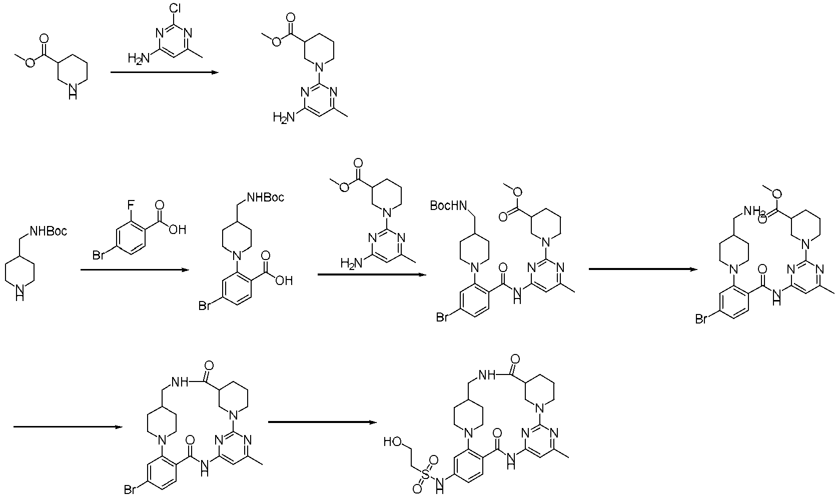

**Step 1:** A solution containing 2-chloro-6-methylpyrimidin-4-amine (5.00 g, 34.83 mmol), methyl piperidine-3-carboxylate (5.50 g, 38.31 mmol), potassium carbonate (4.70 g, 104.48 mmol) and dimethyl sulfoxide (100.00 mL) was held at 100°C for 12 hours. The reaction solution was poured into water (50 mL) and extracted with ethyl acetate (50 mL × 3). The organic layer was dried over anhydrous sodium sulfate, then filtered and concentrated, and purified by column chromatography (silica gel, 0-30% gradient of ethyl acetate/petroleum ether) to give methyl 1-(4-amino-6-methylpyrimidin-2-yl)piperidine-3-carboxylate (5.2 g, 20.78 mmol, yield: 59.7%) as a yellow oil. LCMS (ESI): [M-55]⁺ = 251.2.

Step 2: A solution containing (tert-butoxy)-N-(4-piperidinylmethyl)carboxamide (5.00 g, 23.33 mmol), 4-bromo-2-fluorobenzoic acid (5.60 g, 25.66 mmol), potassium carbonate (9.90 g, 69.99 mmol) and dimethyl sulfoxide (100.00 mL) was stirred at 100°C for 12 hours. The reaction solution was poured into water (50 mL) and extracted with ethyl acetate (50 mL × 3). The organic layer was dried over anhydrous sodium sulfate, then filtered and concentrated, and purified by column chromatography (silica gel, 0-30% gradient of ethyl acetate/petroleum ether) to give 4-bromo-2-(4-(((tert-butoxycarbonyl)amino)methyl)piperidin-1-yl)benzoic acid (4.50 g, crude) as a yellow oil. LCMS (ESI): [M+H]⁺ = 413.2.

Step 3: A solution containing 4-bromo-2-(4-(((tert-butoxycarbonyl)amino)methyl)piperidin-1-yl)benzoic acid (4.00 g, 9.68 mmol), methyl 1-(4-amino-6-methylpyrimidin-2-yl)piperidine-3-carboxylate (2.70 g, 10.65 mmol), 2-(7-azobenzotriazol)-N,N,N',N'-tetramethyluronium hexafluorophosphate (5.60 g, 14.52 mmol), 4-dimethylaminopyridine (3.60 g, 29.03 mmol) and N,N-dimethylacetamide (50.00 mL) was stirred at 60°C for 12 hours. The reaction mixture was poured into water (50 mL) and extracted with ethyl acetate (50 mL × 3). The organic layer was dried over anhydrous sodium sulfate, then filtered and concentrated, and purified by column chromatography (silica gel, 0-50% gradient of ethyl acetate/petroleum ether) to give methyl 1-(4-(4-bromo-2-(4-(((tert-butoxycarbonyl)amino)methyl)piperidin-1-yl)benzamido)-6-methylpyrimidin-2-yl)piperidin-3-carboxylate (700 mg, 1.08 mmol, yield: 11.2%) as a white solid. LCMS (ESI): [M+H]⁺ = 645.4.

Step 4: A reaction solution containing methyl 1-(4-(4-bromo-2-(4-(((tert-butoxycarbonyl)amino)methyl)piperidin-1-yl)benzamido)-6-methylpyrimidin-2-yl)piperidin-3-carboxylate (700 mg, 1.08 mmol) and hydrochloric acid/ethyl acetate solution (10.00 mL, 4.0 M) was stirred at 20°C for 1 hour. The reaction solution was concentrated to give methyl 1-(4-(2-(4-(aminomethyl)piperidin-1-yl)-4-bromobenzamido)-6-methylpyrimidin-2-yl) piperidin-3-carboxylate (500 mg, crude product) as a white solid. LCMS (ESI): [M+H]⁺ = 545.3.

Step 5: A solution containing methyl 1-(4-(2-(4-(aminomethyl)piperidin-1-yl)-4-bromobenzamido)-6-methylpyrimidin-2-yl) piperidin-3-carboxylate (500 mg, 0.92 mmol), 1,5,7-triazabicyclo[4.4.0]dec-5-ene (195 mg, 1.38 mmol) and toluene (250.00 mL) was stirred at 250°C for 48 hours. The reaction mixture was poured into water (30 mL) and extracted with ethyl acetate (30 mL × 3). The organic layer was dried over anhydrous sodium sulfate, then filtered and concentrated, and purified by column chromatography (silica gel, 0-100% gradient of ethyl acetate/petroleum ether) to give 5⁴-bromo-2⁶-methyl-3,8-diaza-2(2,4)-pyrimidin-1(1,3),6(1,4)-dipiperidin-5(1,2)-benz eneheterocyclononane-4,9-dione (260 mg, 0.51 mmol, yield: 55.2%) as a white solid. LCMS (ESI): [M+H]⁺ = 513.3.

Step 6: A solution containing 5⁴-bromo-2⁶-methyl-3,8-diaza-2(2,4)-pyrimidin-1(1,3),6(1,4)-dipiperidin-5(1,2)-benz eneheterocyclononane-4,9-dione (240 mg, 0.47 mmol), 2-hydroxyethanesulfonamide (119 mg, 0.93 mmol), potassium phosphate (304 mg, 1.40 mmol), 2-di-tert-butylphosphino-2',4',6'-triisopropylbiphenyl (20 mg, 0.047 mmol), tris(dibenzylideneacetone)dipalladium (44 mg, 0.047 mmol) and 1,4-dioxane (5.00 mL) was stirred at 100°C for 8 hours under nitrogen atmosphere. The reaction solution was poured into water (30 mL) and extracted with ethyl acetate (30 mL × 3). The organic layer was dried over anhydrous sodium sulfate, then filtered and concentrated, and purified by column chromatography (silica gel, 0-25% gradient of methanol/dichloromethane) to give N-(2⁶-methyl-4,9-dioxo-3,8-diaza-2(2,4)-pyrimidin-1(1,3),6(1,4)-dipiperidin-5(1,2)-b enzeneheterocyclononane-5⁴-yl)-2-hydroxyethane-1-sulfonamide (80 mg, 0.14 mmol, yield: 30.7%). LCMS (ESI): [M+H]⁺ = 558.40.¹H NMR (400 MHz, DMSO) δ 11.22 (s, 1H), 10.21 (s, 1H), 7.89 (d, *J* = 8.4 Hz, 1H), 7.68 (dd, *J= 9.2,* 4.0 Hz, 1H), 7.35 (s, 1H), 7.15 - 7.08 (m, 1H), 7.03 (dd, *J* = 8.4, 2.0 Hz, 1H), 4.97 - 4.84 (m, 2H), 4.74 (d, *J* = 12.8 Hz, 1H), 3.81 - 3.70 (m, 2H), 3.66 - 3.55 (m, 1H), 3.44 - 3.35 (m, 2H), 3.22 - 3.11 (m, 1H), 3.05-2.96 (m, 1H), 2.93 - 2.83 (m, 1H), 2.79 - 2.68 (m, 2H), 2.57 (s, 2H), 2.48 - 2.40 (m, 1H), 2.25 (s, 3H), 2.06 (d, *J* = 12.7 Hz, 1H), 1.94 - 1.68 (m, 4H), 1.63 - 1.52 (m, 2H), 1.51 - 1.40 (m, 1H), 1.32 - 1.21 (m, 1H) ppm.

### Example 28:

### 2-Hydroxy-N-(2⁶-methyl-4-oxo-8-oxa-3-aza-2(2,4)-pyrimidin-1(1,3),6(1,4)-dipiper idin-5(1,2)-benzeneheterocyclononane-5⁴-yl)ethane-1-sulfonamide

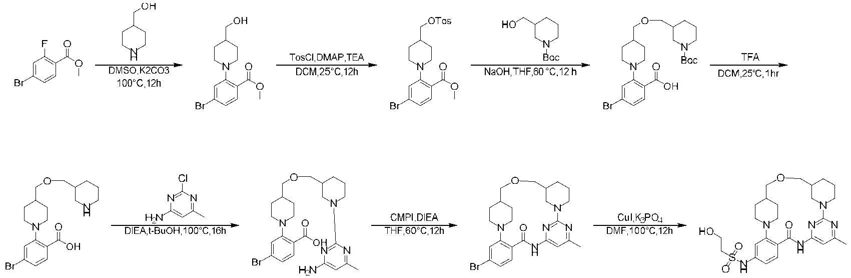

**Step 1:** To a solution of methyl 4-bromo-2-fluorobenzoate (10.0 g, 49.2 mmol) and potassium carbonate (17.80 g, 129.0 mmol) in dimethyl sulfoxide (200.00 mL) was added piperidin-4-ylmethanol (5.93 g, 51.5 mmol), and the mixture was stirred at 100°C for 12 hours. The reaction mixture was poured into water (400 mL) and extracted with ethyl acetate (400 mL × 2). The combined organic layer was washed with saturated saline (250 mL), dried over anhydrous magnesium sulfate, filtered, and concentrated under reduced pressure to give a residue. The residue was purified by flash column chromatography (silica gel, 0-16% gradient of tetrahydrofuran/petroleum ether) to give methyl 4-bromo-2-(4-(hydroxymethyl)piperidin-1-yl)benzoate (12.0 g, 41.70 mmol, yield: 85%) as a yellow oil. LCMS (ESI): [M+H]⁺ = 328.1

**Step 2:** At 25°C, to a solution of methyl 4-bromo-2-(4-(hydroxymethyl)piperidin-1-yl)benzoate (7.00 g, 21.3 mmol) in dichloromethane (140.00 mL) were added triethylamine (4.32 g, 42.7 mmol), 4-dimethylaminopyridine (434 mg, 2.13 mmol) and 4-toluenesulfonyl chloride (4.47 g, 23.5 mmol). The reaction mixture was stirred at 25°C for 12 hours. The reaction mixture was poured into water (200 mL) and extracted with ethyl acetate (200 mL × 2). The combined organic layer was washed with saturated saline (200 mL), dried over anhydrous magnesium sulfate, filtered, and concentrated under reduced pressure to give a residue. The residue was purified by flash column chromatography (silica gel, 0-8% gradient of tetrahydrofuran/petroleum ether) to give methyl 4-bromo-2-(4-(toluenesulfonyloxy)methyl)piperidin-1-yl)benzoate (6.0 g, 12.4 mmol, yield: 58%) as a yellow oil. LCMS (ESI): [M+H]⁺ = 484.1. ¹H NMR (400 MHz, DMSO-d6) δ 7.80 (d, *J* = 8.4 Hz, 2H), 7.49 (d, *J* = 8.4 Hz, 3H), 7.17 - 7.09 (m, 2H), 3.94 (d, *J* = 6.4 Hz, 2H), 3.76 (s, 3H), 3.17 (br d, *J* = 12.0 Hz, 2H), 2.67 (br t, *J* = 11.2 Hz, 2H), 2.42 (s, 3H), 1.80 - 1.68 (m, 1H), 1.62 (br d, *J* = 11.2 Hz, 2H), 1.33 - 1.19 (m, 2H) ppm.

**Step 3:** To a solution of methyl 4-bromo-2-(4-((tosyloxy)methyl)piperidin-1-yl)benzoate (1.0 g, 2.07 mmol) and tert-butyl 3-(hydroxymethyl)piperidine-1-carboxylate (0.67 g, 3.11 mmol) in tetrahydrofuran (20.0 mL) was added sodium hydroxide (0.21 g, 3.11 mmol). The mixture was stirred at 60°C for 12 hours. The reaction solution was diluted with water (80 mL), adjusted to pH 2-3 with a saturated citric acid solution, and extracted with an ethyl acetate solution (100 mL × 2). The organic layer was dried over magnesium sulfate, filtered, and concentrated under reduced pressure to give a residue which was purified by flash column chromatography (silica gel, 0-35% gradient of ethyl acetate/petroleum ether) to give 4-bromo-2-(4-(((1-(tert-butoxycarbonyl)piperidin-3-yl)methoxy)methyl)piperidin-1-yl) benzoic acid (0.45 g, 0.87 mmol, yield: 42%) as a yellow oil. LCMS (ESI): [M+H]⁺ = 513.1

**Step** 4: 4-bromo-2-(4-(((1-(tert-butoxycarbonyl)piperidin-3-yl)methoxy)methyl)piperidin-1-yl) benzoic acid (400 mg, 0.78 mmol) was dissolved in dichloromethane (20.00 mL) and trifluoroacetic acid (5.00 mL). The mixture was stirred at 25°C for 1 hour. The reaction solution was concentrated under reduced pressure to give 4-bromo-2-(4-((piperidin-3-ylmethoxy)methyl)piperidin-1-yl)benzoic acid (300 mg, crude) as a yellow oil. LCMS (ESI): [M+H]⁺ = 413.1

**Step 5:** 4-bromo-2-(4-((piperidin-3-ylmethoxy)methyl)piperidin-1-yl)benzoic acid (0.35 g, 0.85 mmol) was dissolved in tert-butanol (7.00 mL), N,N-diisopropylethylamine (1.10 g, 8.51 mmol) and 2-chloro-6-methylpyrimidin-4-amine (147 mg, 1.02 mmol) were added and the mixture was stirred at 100°C for 16 hours. The reaction solution was diluted with water (20 mL), adjusted to pH 2-3 with 1 N hydrochloric acid, and extracted with chloroform/isopropanol (3:1, 40 mL × 3). The organic layer was dried, filtered and concentrated. A brown oily residue was purified by flash column chromatography (silica gel, 0-10% gradient of dichloromethane/methanol) to give 2-(4-(((1-(4-amino-6-methylpyrimidin-2-yl)piperidin-3-yl)methoxy)methyl)piperidin-1-yl)-4-bromobenzoic acid (470 mg, crude) as a yellow oil. LCMS (ESI): [M+H]⁺ = 518.3

**Step 6:** N,N-diisopropylethylamine (763 mg, 5.78 mmol) and 2-chloro-1-methylpyridinium iodide (444 mg, 1.74 mmol) were dissolved in tetrahydrofuran (30.00 mL), a solution of 2-(4-(((1-(4-amino-6-methylpyrimidin-2-yl)piperidin-3-yl)methoxy)methyl)piperidin-1-yl)-4-bromobenzoic acid (300 mg, 0.58 mmol) in tetrahydrofuran (120.00 mL) was added at 60°C and the mixture was stirred at 60°C for 12 hours. The reaction mixture was diluted with water (100 mL), extracted with ethyl acetate (100 mL × 2), and the organic phase was dried, filtered and concentrated. The residue was purified by flash column chromatography (silica gel, 0-12% gradient of tetrahydrofuran/petroleum ether) to give 5⁴-bromo-2⁶-methyl-8-oxa-3-aza-2(2,4)-pyrimidin-1(1,3),6(1,4)-dipiperidin-5(1,2)-be nzeneheterocyclonon-4-one (60 mg, crude) as a colorless oil. LCMS (ESI): [M+H]⁺ = 501.7

**Step 7:** 5⁴-bromo-2⁶-methyl-8-oxa-3-aza-2(2,4)-pyrimidin-1(1,3),6(1,4)-dipiperidin-5(1,2)-be nzeneheterocyclonon-4-one (50 mg, 0.1 mmol) and 2-hydroxyethane-1-sulfonamide (15 mg, 0.12 mmol) were dissolved in N,N-dimethylformamide (1.00 mL), potassium phosphate (63.6 mg, 0.3 mmol), (1R,2R)-N1,N2-dimethylcyclohexane-1,2-diamine (14.4 mg, 0.1 mmol) and cuprous iodide (19.1 mg, 0.1 mmol) were added and the mixture was stirred at 100°C for 12 hours under nitrogen atmosphere. The reaction mixture was diluted with water (10.0 mL+0.5 mL liquor ammonia) and extracted with ethyl acetate (20 mL × 2), and the organic phase was dried, filtered, and concentrated. The residue was purified by preparative HPLC (C18, 6%-46% gradient of water (formic acid)/acetonitrile) to give 2-hydroxy-N-(2⁶-methyl-4-oxo-8-oxa-3-aza-2(2,4)-pyrimidin-1(1,3),6(1,4)-dipiperidi n-5(1,2)-benzeneheterocyclononane-5⁴-yl)ethane-1-sulfonamide (8 mg, 0.01 mmol, yield: 14.7%) as a white solid. LCMS (ESI): [M+H]⁺ = 545.2. ¹H NMR (400 MHz, DMSO-d6) δ 12.09 (s, 1H), 11.59 - 9.41 (m, 1H), 8.16 (br s, 1H), 7.87 (d, *J* = 8.8 Hz, 1H), 7.22 - 7.16 (m, 1H), 7.10 (d, *J* = 2.0 Hz, 1H), 7.03 (dd, *J* = 2.0, 8.8 Hz, 1H), 5.69 - 5.10 (m, 1H), 5.08 - 4.89 (m, 1H), 4.79 - 4.63 (m, 1H), 3.80 - 3.68 (m, 2H), 3.62 - 3.52 (m, 1H), 3.48 - 3.39 (m, 2H), 3.30 - 3.27 (m, 2H), 3.27 - 3.09 (m, 4H), 2.95 - 2.70 (m, 4H), 2.32 - 2.14 (m, 4H), 1.99 - 1.73 (m, 2H), 1.73 - 1.32 (m, 5H), 1.13 - 0.95 (m, 1H) ppm.

### Example 28A:

### 2-Hydroxy-N-((1³R)-2⁶-methyl-4-oxo-8-oxa-3-aza-2(2,4)-pyrimidin-1(1,3),6(1,4)-d ipiperidin-5(1,2)-benzeneheterocyclononane-5⁴-yl)ethane-1-sulfonamide

### Example 28B:

### 2-Hydroxy-N-((1³S)-2⁶-methyl-4-oxo-8-oxa-3-aza-2(2,4)-pyrimidin-1(1,3),6(1,4)-d ipiperidin-5(1,2)-benzeneheterocyclononane-5⁴-yl)ethane-1-sulfonamide

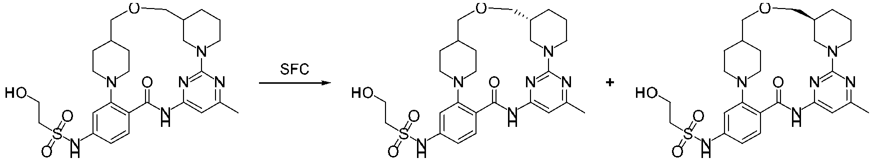

**Step** 1: 2-hydroxy-N-(2⁶-methyl-4-oxo-8-oxa-3-aza-2(2,4)-pyrimidin-1(1,3),6(1,4)-dipiperidi n-5(1,2)-benzeneheterocyclononane-5⁴-yl)ethane-1-sulfonamide (25 mg, 0.046 mmol) was separated by SFC (column: DAICEL CHIRALPAK AD (250 mm*30 mm, 10 um); mobile phase: CO₂-EtOH (0.1% NH₃H₂O); gradient: isocratic 45%; flow rate: 80 mL/min; column temperature: 40°C) to give **Example 28A** and **Example 28B.**

**Example 28A:** SFC (column: Chiralpak AD 50¡Á4.6 mm I.D., 3 um; mobile phase: CO₂-EtOH (0.05% DEA); gradient: from 20% to 40% of B in 1.5 min and hold 40% for 1 min, then 20% of B for 0.5 min; flow rate: 4 mL/min; column temperature: 35°C; retention time Rt=1.204). LCMS (ESI): [M+H]⁺ = 545.2. ¹H NMR (400 MHz, DMSO-d6) δ ppm 12.09 (s, 1H), 10.68 - 9.79 (m, 1H), 7.86 (d, *J=* 8.4 Hz, 1H), 7.19 (s, 1H), 7.10 (d, *J* = 1.6 Hz, 1H), 7.03 (dd, *J=* 2.0, 8.8 Hz, 1H), 5.42 - 4.82 (m, 2H), 4.77 - 4.63 (m, 1H), 3.76 (br t, *J=* 6.4 Hz, 2H), 3.60 - 3.48 (m, 2H), 3.28 (br s, 5H), 2.92 - 2.70 (m, 4H), 2.30 - 2.14 (m, 4H), 1.98 - 1.31 (m, 8H), 1.15 - 0.96 (m, 1H)

**Example 28B:** SFC (column: Chiralpak AD 50¡Á4.6 mm I.D., 3 µm, mobile phase: CO₂-EtOH (0.05% DEA), gradient: from 20% to 40% of B in 1.5 min and hold 40% for 1 min, then 20% of B for 0.5 min, flow rate: 4 mL/min, column temperature: 35°C, retention time Rt=1.942). LCMS (ESI): [M+H]⁺ = 545.2. ¹H NMR (400 MHz, DMSO-d6) δ ppm 12.50 (br s, 1H), 11.13 - 9.09 (m, 1H), 7.93 (d, *J* = 8.8 Hz, 1H), 7.26 - 7.14 (m, 2H), 7.05 (dd, *J* = 2.0, 8.4 Hz, 1H), 5.51 - 5.03 (m, 1H), 4.93 (br d, *J* = 10.4 Hz, 1H), 4.57 (br d, *J* = 12.8 Hz, 1H), 3.75 (t, *J* = 6.4 Hz, 2H), 3.64 (td, *J* = 4.8, 9.2 Hz, 1H), 3.55 (br dd, *J* = 3.2, 10.4 Hz, 1H), 3.30 - 3.25 (m, 2H), 3.22 - 2.97 (m, 5H), 2.90 - 2.75 (m, 3H), 2.30 - 2.18 (m, 4H), 1.96 - 1.29 (m, 8H), 1.11 - 1.00 (m, 1H)

### Example 29:

### 2-Hydroxy-N-(5⁶-methyl-3-oxo-6,9-dioxa-4-aza-5(4,2)-pyrimidin-1(1,4)-piperidin -2(1,2)-benzeneheterocycloundecane-2⁵)ethane-1-sulfonamide

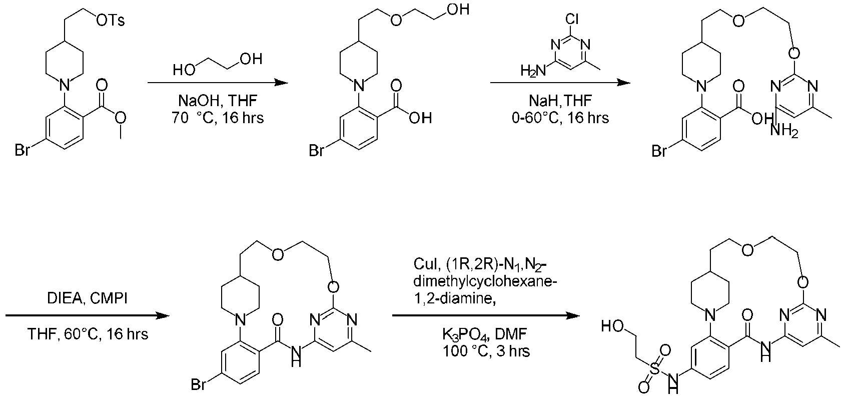

**Step 1:** Methyl 4-bromo-2-(4-(2-(tosyloxy)ethyl)piperidin-1-yl)benzoate (5.00 g, 10.0 mmol) was dissolved in tetrahydrofuran (80.0 mL), ethylene glycol (4.38 g, 70.5 mmol) and sodium hydroxide (1.01 g, 15.1 mmol) were added and the mixture was stirred at 70°C for 16 hours. The reaction solution was filtered and concentrated to give a residue, which was purified by flash column chromatography (silica gel, 0-7% gradient of methanol/dichloromethane) to give 4-bromo-2-(4-(2-(2-hydroxyethoxy)ethyl)piperidin-1-yl)benzoic acid (1.70 g, 3.62 mmol, yield: 36.0%) as a yellow oil. LCMS (ESI): [M+H]⁺ = 374.0. ¹H NMR (400 MHz, CHLOROFORM-d) δ 8.17 (d, *J* = 8.4 Hz, 1H), 7.60 - 7.54 (m, 2H), 3.81 - 3.73 (m, 2H), 3.65 - 3.52 (m, 4H), 3.16 (br d, *J* = 11.6 Hz, 2H), 2.97 (br t, *J* = 11.6 Hz, 2H), 2.00 (br d, *J=* 12.8 Hz, 2H), 1.81 - 1.72 (m, 1H), 1.67 (q, *J=* 6.0 Hz, 2H), 1.63 - 1.52 (m, 2H) ppm.

**Step** 2: 4-bromo-2-(4-(2-(2-hydroxyethoxy)ethyl)piperidin-1-yl)benzoic acid (1.00 g, 2.69 mmol) was dissolved in tetrahydrofuran (20.0 mL), sodium hydride (0.32 g, 8.06 mmol, 60% mass fraction) was added at 0°C and the mixture was stirred at 0°C for 30 minutes. 2-chloro-6-methylpyrimidin-4-amine (0.77 g, 5.37 mmol) was added and the mixture was stirred at 60°C for 16 hours. The reaction was quenched with methanol. The reaction solution was filtered and concentrated to give 2-(4-(2-(2-((4-amino-6-methylpyrimidin-2-yl)oxy)ethoxy)ethyl)piperidin-1-yl)-4-bro mobenzoic acid (1.80 g, 2.68 mmol, crude) as an orange oil. LCMS (ESI): [M+H]⁺ = 480.9

**Step 3:** N,N-diisopropylethylamine (1.05 g, 8.14 mmol) and 2-chloro-1-methylpyridin-1-ium iodide (1.07 g, 2.82 mmol) were dissolved in tetrahydrofuran (120 mL), a solution of 2-(4-(2-(2-((4-amino-6-methylpyrimidin-2-yl)oxy)ethoxy)ethyl)piperidin-1-yl)-4-bro mobenzoic acid (300 mg, 0.63 mmol) in tetrahydrofuran (30.0 mL) was added dropwise at 60°C and the mixture was stirred at 60°C for 16 hours. The reaction mixture was diluted with water (100 mL), extracted with ethyl acetate (200 mL × 2), and the organic phase was dried, filtered and concentrated. The residue was purified by flash column chromatography (silica gel, 0-13% gradient of tetrahydrofuran/petroleum ether) to give 2⁵-bromo-5⁶-methyl-6,9-dioxa-4-aza-5(4,2)-pyrimidin-1(1,4)-piperidin-2(1,2)-benzen eheterocycloundecane-3-one (53.0 mg, 0.11 mmol, yield: 18.0%) as a yellow solid. LCMS (ESI): [M+H]⁺ = 462.9

**Step** 4: 2⁵-bromo-5⁶-methyl-6,9-dioxa-4-aza-5(4,2)-pyrimidin-1(1,4)-piperidin-2(1,2)-benzen eheterocycloundecane-3-one (48.0 mg, 0.10 mmol) and 2-hydroxyethane-1-sulfonamide (2.03 mg, 0.02 mmol) were dissolved in N,N-dimethylformamide (2.50 mL), potassium phosphate (66.2 mg, 0.31 mmol), (1R,2R)-N1,N2-dimethylcyclohexane-1,2-diamine (14.9 mg, 0.10 mmol) and cuprous iodide (19.8 mg, 0.10 mmol) were added and the mixture was stirred at 100°C for 3 hours under nitrogen atmosphere. To the reaction solution was added aqueous ammonia (0.5 mL) and the mixture was filtered and concentrated. The residue was purified by preparative HPLC (C18, 4%-46% gradient of water (aqueous ammonia + ammonium bicarbonate)/acetonitrile) to give 2-hydroxy-N-(5⁶-methyl-3-oxo-6,9-dioxa-4-aza-5(4,2)-pyrimidin-1(1,4)-piperidin-2(1, 2)-benzeneheterocycloundecane-2⁵)ethane-1-sulfonamide (17.71 mg, 0.02 mmol, yield: 31.8%) as a white solid. LCMS (ESI): [M+H]⁺ = 570.2. ¹H NMR (400 MHz, DMSO-*d₆*) δ 12.71 (s, 1H), 7.98 (d, *J=* 8.8 Hz, 1H), 7.77 (s, 1H), 7.19 (s, 1H), 7.08 (br d, *J* = 8.4 Hz, 1H), 4.56 (br t, *J* = 5.6 Hz, 2H), 3.75 (t, *J* = 6.4 Hz, 2H), 3.58 (t, *J* = 5.6 Hz, 2H), 3.50 (br t, *J* = 4.4 Hz, 2H), 3.32 - 3.27 (m, 2H), 3.14 (br d, *J* = 10.8 Hz, 2H), 2.75 - 2.66 (m, 2H), 2.37 (s, 3H), 2.10 - 1.99 (m, 2H), 1.72 - 1.64 (m, 3H), 1.61 (br d, *J* = 12.8 Hz, 2H) ppm

### Assay 1: Enzymatic Activity Test

**Materials:** Human KIF18A (amino acid sequence 1-417) was purchased from Viva Biotech (Shanghai) Ltd.; the ADP-Glo^{™} protein kinase kit was acquired from Promega, USA; tubulin was sourced from Cytoskeleton, USA; and the 384-well assay plate and Envision multifunctional microplate reader were obtained from PerkinElmer, USA.

**Enzymatic Activity Test:** The compound powder was dissolved in DMSO to prepare a stock solution of 10 mM. Then gradient dilution was performed for the compounds in the microplate to achieve a final concentration of 0-10 µM. Then, 2.5 µL each of tubulin, compound, ATP, and KIF18A protein were added to the microplate in sequence for reaction at ambient temperature for 120 min. The final concentrations in the enzyme reaction were 60 µg/mL for microtubules, 25 µM for ATP, and 2.5 nM for KIF18A protein. After the enzyme reaction, 10 µL of ADP-GLO reaction reagent was added to each well and incubated at room temperature for 30 min. After that, 20 µL of detection reagent was added to each well and incubated at room temperature for 30 min in the dark. Finally, chemiluminescence detection was performed using the PerkinElmer Envision.

| Example | KIF 18A IC₅₀ nM | Example | KIF18A IC₅₀ nM | Example | KIF 18A IC₅₀ nM |
|---|---|---|---|---|---|
| Example 1 | 62 | Example 14 | 21 | Example 27 | 265 |
| Example 1A | >3000 | Example 15 | 275 | Example 28 | 229 |
| Example 1B | 34 | Example 16 | 588 | Example 28A | 178 |
| Example 1C | 36 | Example 17 | 605 | Example 28B | 743 |
| Example 1D | 246 | Example 18 | 94 | Example 29 | 141 |
| Example 2 | 8 | Example 18A | >3000 | | |
| Example 3 | 98 | Example 18B | 46 | | |
| Example 3A | 26 | Example 18C | 41 | | |
| Example 3B | 67 | Example 18D | 345 | | |
| Example 4 | 736 | Example 19 | 184 | | |
| Example 5 | 38 | Example 20 | 2917 | | |
| Example 6 | 89 | Example 21 | 305 | | |
| Example 7 | 197 | | | | |
| Example 8 | 2295 | Example 23 | 102 | | |
| Example 9 | 293 | Example 23A | 522 | | |
| Example 10 | 200 | Example 23B | 71.5 | | |
| Example 11 | 233 | Example 24 | 158 | | |
| Example 12 | 28 | Example 25 | 13 | | |
| Example 13 | 23 | Example 26 | 15 | | |

### Assay 2: Cell Proliferation Activity Test

**Materials and Cells:** OVCAR3 cells were purchased from Nanjing Cobioer Biosciences Co., Ltd. RPMI-1640 medium, fetal bovine serum, and the CyQUANT Direct Cell Proliferation Assay kit were purchased from Thermo Fisher Scientific, USA; 96-well cell culture plates were sourced from Coming, USA.

**Cell Culture:** OVCAR3 cells were cultured with RPMI-1640 medium containing 10% fetal bovine serum and incubated in a 5% CO₂ incubator at 37°C. Only cells in the logarithmic growth phase could be used for the test.

### Cell Proliferation Activity Test:

OVCAR3 cells were inoculated into a 96-well cell culture plate at 90 µL per well and incubated overnight in a 5% CO₂ incubator at 37°C. The compound powder was dissolved in DMSO to prepare a stock solution of 10 mM. Then gradient dilution was performed for the compounds in the microplate to achieve a final concentration of 0-10 µM. 10 µL of cell culture medium containing compounds was added to each well to make the final DMSO content 0.2%. The cell plates were incubated in a 5% CO₂ incubator at 37°C for 3 days. 100 µL of CyQUANT detection reagent was added to each well, reacted at 37°C for 60 min, and fluorescence detection was performed using the PerkinElmer Envision.

| Example | OVCAR-3 _IC₅₀_nM | Example | OVCAR-3 _IC₅₀_nM | Example | OVCAR-3 _IC₅₀_nM |
|---|---|---|---|---|---|
| Example 1 | **14** | Example 10 | 136 | Example 21 | 744 |
| Example 1A | >3000 | Example 11 | 150 | | |
| Example 1B | 6 | Example 12 | 124 | Example 23 | 52 |
| Example 1C | 61 | Example 13 | 77 | Example 23A | 662 |
| Example 1D | 506 | Example 14 | 85 | Example 23B | 28 |
| Example 2 | 74 | Example 15 | >3000 | Example 24 | 46 |
| Example 3 | 76 | Example 16 | >3,000 | Example 25 | 8.5 |
| Example 3A | 1609 | Example 17 | >3,000 | Example 26 | ND |
| Example 3B | 36 | Example 18 | 31 | Example 27 | 1484 |
| Example 4 | 1656 | Example 18A | >3000 | Example 28 | 506 |
| Example 5 | 122 | Example 18B | 12 | Example 29 | ND |
| Example 6 | 52 | Example 18C | 29 | | |
| Example 7 | 242 | Example 18D | 458 | | |
| Example 8 | >3000 | Example 19 | 703 | | |
| Example 9 | 548 | Example 20 | >3000 | | |

### Assay 3: CyQuant Cell Proliferation Activity Test

**Materials and Cells:** HT29 cells were purchased from Nanjing Cobioer Biosciences Co., Ltd.; RPMI-1640 medium, fetal bovine serum, Trypsin-EDTA (0.25%), 96-well plates, and CyQuant reagents were purchased from ThermoFisher (USA); DMSO was purchased from SIGMA (USA).

**Cell Culture:** HT29 cells were cultured with RPMI-1640 containing 10% fetal bovine serum under the condition of 37°C and 5% CO₂. Only cells in the logarithmic growth phase could be used for the test.

**Cell Proliferation Activity Test:** CyQuant reagent was used to detect the proliferation inhibitory activity of compounds on HT29 cells. The cell density was adjusted, and 100 µL per well was inoculated into a 96-well plate (HT29 at 2000 cells per well), then incubated overnight at 37°C and 5% CO₂. Add compounds of various target concentrations (initial concentration of 3000 nM, 3-fold dilution, 9 concentration gradients), and make the DMSO content 0.2%. The cell plate was incubated at 37°C in 5% CO₂ for 3 days. CyQuant reagent was added and incubated for 1 h. The plates were read by Envision, and IC₅₀ was calculated using XLFIT.

| Example | HT29_IC50 _nM | Example | HT29_IC50 _nM | Example | HT29_IC50 _nM |
|---|---|---|---|---|---|
| Example 1 | **17** | Example 11 | ND | Example 23 | 47 |
| Example 1A | >3000 | Example 12 | 251 | Example 23A | 688 |
| Example 1B | 7 | Example 13 | 136 | Example 23B | 32 |
| Example 1C | 78 | Example 14 | 151.9 | Example 24 | 63 |
| Example 1D | 746 | Example 15 | >3,000 | Example 25 | 10.9 |
| Example 2 | 178 | Example 16 | >3,000 | Example 26 | ND |
| Example 3 | 68 | Example 17 | >3,000 | Example 27 | >3,000 |
| Example 3A | 1325 | Example 18 | 34 | Example 28 | 639 |
| Example 3B | 43 | Example 18A | >3000 | Example 28A | ND |
| Example 4 | >3000 | Example 18B | 16 | Example 28B | ND |
| Example 5 | 132 | Example 18C | 31 | Example 29 | ND |
| Example 6 | 64 | Example 18D | 660 | | |
| Example 7 | 255 | Example 19 | 644 | | |
| Example 8 | ND | Example 20 | >3000 | | |
| Example 9 | ND | Example 21 | 703 | | |
| Example 10 | 172 | | | | |

## Claims

1. A compound having the structure of Formula I, or pharmaceutically acceptable salts, stereoisomers or isotope isomers thereof: 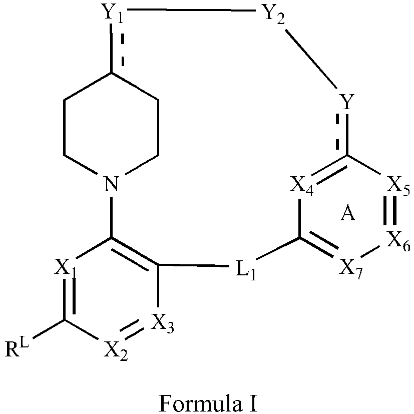
wherein, -̅ -̅ -̅ represents a single bond or double bond;
wherein, X₁ represents CR^{W1} or N;
wherein, X₂ represents CR^{W2} or N;
wherein, X₃ represents CR^{W3} or N;
wherein, X₄ represents CR^{W4} or N;
wherein, X₅ represents CR^{W5} or N;
wherein, X₆ represents CR^{W6} or N;
wherein, X₇ represents CR^{W7} or N;
wherein, R^{W1}, R^{W2}, R^{W3}, R^{W4}, R^{W5}, R^{W6} and R^{W7} each independently represent hydrogen, halogen, cyano, nitro, hydroxy (C₁-C₆) alkyl, C₁-C₆ alkyl, C₃-C₆ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b};
wherein, the chemical bond between X₂ and X₃, or between X₅ and X₆, or between X₆ and X₇ may be fused with ring A to form a 5-6-membered saturated or unsaturated ring which may optionally contain 0, 1 or 2 heteroatoms selected from O, S and N; the ring may be optionally substituted with 0-3 substituents selected from the following: halogen, cyano, nitro, hydroxy (C₁-C₆) alkyl, C₁-C₆ alkyl, C₃-C₈ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b};
wherein, L₁ represents -C(O)NH- or triazolyl;
wherein, Y₁ represents -(CR^{a}R^{b})ₘ₋; Y₂ represents -(CR^{a}R^{b})ₙ₋;
wherein, optionally, CR^{a}R^{b} may be substituted with -O-, -S-, -NR^{a}-, -NR^{a}SO₂-, -NR^{a}C(O)-, -C(O)NR^{a}-, -SO₂NR^{a}-, -S(=O)(NR^{a})-, -P(O)(OR^{a})₂-, -NR^{a}P(O)(OR^{a})₂- or -NR^{a}P(O)(R^{a})₂-, -CR^{a}=CR^{b}-, 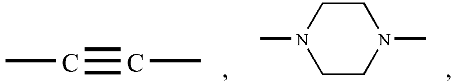 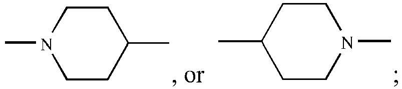
wherein, R^{L} represents L₂-R^{M};
wherein, L₂ represents absence, -C₁-C₆ alkyl-, -NR^{a}-, -NR^{a}SO₂-, -SO₂NR^{a}-, -NR^{a}-S(=O)(=NH), -S(=O) (=NH)-, -S-, -S(=O)-, -SO₂-, -C₁-C₆ alkyl-O-, -(C=O)-, -(C=O)NR^{a}-, -C=N(OH)-, -NR^{a} (C=O), -P(O)(OR^{a})₂, -NR^{a}P(O)(OR^{a})₂ or -NR^{a}P(O)(R^{a})₂-;
wherein, R^{M} represents C₁-C₆ alkyl or C₃-C₆ cycloalkyl, wherein the C₁-C₆ alkyl or C₃-C₆ cycloalkyl can be optionally independently substituted with 0-3 substituents selected from halogen, -OR^{a}, -NR^{a}R^{b}, cyano and -O-C₁-C₆ haloalkyl;
alternatively, L-R^{M} represents -N=S(=O)-(R^{L})₂, wherein the two R^{L} groups, together with the sulfur atom attached thereto, form a saturated or unsaturated 3-, 4-, 5- or 6-membered monocyclic ring containing 0, 1 or 2 heteroatoms selected from N, O and S;
wherein, m and n each independently represent an integer from 0 to 10;
wherein, when -̅ -̅ -̅ represents a single bond, Y represents -O-, -NH-, or the following groups: 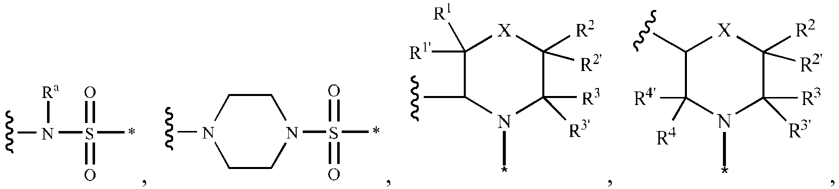 or 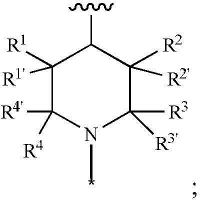
wherein, * represents the site of connection with ring A; the wavy line represents the site of connection with Y₂;
wherein, X represents CR¹R^{1',} O or NR^{a};
wherein, R¹, R^{1'}, R², R^{2'}, R³, R^{3'}, R⁴ and R^{4'} each independently represent hydrogen, C₁-C₆ alkyl, halogen or C₃-C₆ cycloalkyl; hydroxy (C₁-C₆) alkyl;
alternatively, R¹ and R^{1'}, together with the carbon atom attached thereto, form a 3-6 membered ring;
alternatively, R² and R^{2'}, together with the carbon atom attached thereto, form a 3-6 membered ring;
alternatively, R³ and R^{3'}, together with the carbon atom they are attached to, form a 3-6 membered ring;
alternatively, R⁴ and R^{4'}, together with the carbon atom they are attached to, form a 3-6 membered ring;
wherein, R^{a} and R^{b} each independently represent hydrogen, halogen, C₁-C₆ alkyl, C₃-C₆ cycloalkyl, C₁-C₆ haloalkyl, hydroxy (C₁-C₆) alkyl, C₁-C₆ alkyl substituted with saturated or unsaturated 5-6-membered cycloalkyl, or C₁-C₆ alkyl substituted with saturated or unsaturated 5-6-membered heterocycloalkyl; alternatively, R^{a} and R^{b}, together with the atom attached thereto, form a 3-6-membered saturated or unsaturated ring which may optionally contain 0-2 heteroatoms selected from O, S and N.

2. The compound having the structure of Formula I according to claim 1, or pharmaceutically acceptable salts, stereoisomers or isotope isomers thereof, wherein X₁, X₂, X₃, X₄ and X₅ represent CH or N.

3. The compound having the structure of Formula I according to claim 1, or pharmaceutically acceptable salts, stereoisomers or isotope isomers thereof, wherein X₁ and X₂ represent CH or N.

4. The compound having the structure of Formula I according to claim 1, or pharmaceutically acceptable salts, stereoisomers or isotope isomers thereof, wherein X₄ and X₅ represent CH or N.

5. The compound having the structure of Formula I according to claim 1, or pharmaceutically acceptable salts, stereoisomers or isotope isomers thereof, wherein Y₁ represents -(CR^{a}R^{b})ₘ-.

6. The compound having the structure of Formula I according to claim 1, or pharmaceutically acceptable salts, stereoisomers or isotope isomers thereof, wherein Y₁ represents -NH-C(=O)-.

7. The compound having the structure of Formula I according to claim 1, or pharmaceutically acceptable salts, stereoisomers or isotope isomers thereof, wherein Y₁ represents -O(CR^{a}R^{b})ₘ- or -(CR^{a}R^{b})ₘO-.

8. The compound having the structure of Formula I according to claim 1, or pharmaceutically acceptable salts, stereoisomers or isotope isomers thereof, wherein Y₁ represents -CR^{a}=CR^{a}- or -CR^{a}R^{b}-CR^{a}=CR^{b}-.

9. The compound having the structure of Formula I according to claim 1, or pharmaceutically acceptable salts, stereoisomers or isotope isomers thereof, wherein Y₂ represents -(CR^{a}R^{b})ₙ-.

10. The compound having the structure of Formula I according to claim 1, or pharmaceutically acceptable salts, stereoisomers or isotope isomers thereof, wherein Y₂ represents -NR^{a}-C(=O)-.

11. The compound having the structure of Formula I according to claim 1, or pharmaceutically acceptable salts, stereoisomers or isotope isomers thereof, wherein Y₂ represents -O(CR^{a}R^{b})ₙ- or -(CR^{a}R^{b})ₙO-.

12. The compound having the structure of Formula I according to claim 1, or pharmaceutically acceptable salts, stereoisomers or isotope isomers thereof, wherein Y₂ represents -CR^{a}=CR^{a}-, -CR^{a}=CR^{b}-CR^{a}R^{b}-, or -CR^{a}R^{b}-CR^{a}=CR^{a}-.

13. The compound having the structure of Formula I according to claim 1, or pharmaceutically acceptable salts, stereoisomers or isotope isomers thereof, wherein -Y₁-Y₂ represents -CR^{a}=CR^{a}-.

14. The compound having the structure of Formula I according to claim 1, or pharmaceutically acceptable salts, stereoisomers or isotope isomers thereof, wherein -Y₁-Y₂ represents -CR^{a}R^{b}-NR^{a}C(O)-.

15. The compound having the structure of Formula I according to claim 1, or pharmaceutically acceptable salts, stereoisomers or isotope isomers thereof, wherein -Y₁-Y₂ represents -

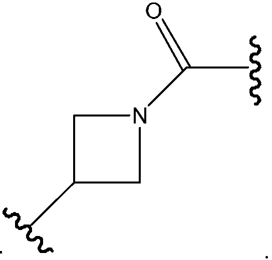

16. The compound having the structure of Formula I according to claim 1, or pharmaceutically acceptable salts, stereoisomers or isotope isomers thereof, wherein Y represents -O- or -NH-.

17. The compound having the structure of Formula I according to claim 1, or pharmaceutically acceptable salts, stereoisomers or isotope isomers thereof, wherein Y represents

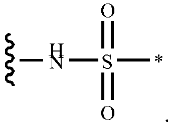

18. The compound having the structure of Formula I according to claim 1, or pharmaceutically acceptable salts, stereoisomers or isotope isomers thereof, wherein Y represents

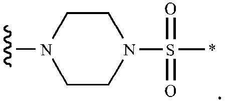

19. The compound having the structure of Formula I according to claim 1, or pharmaceutically acceptable salts, stereoisomers or isotope isomers thereof, wherein Y represents

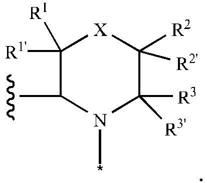

20. The compound having the structure of Formula I according to claim 1, or pharmaceutically acceptable salts, stereoisomers or isotope isomers thereof, wherein Y represents

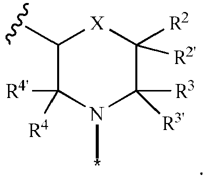

21. The compound having the structure of Formula I according to claim 1, or pharmaceutically acceptable salts, stereoisomers or isotope isomers thereof, wherein Y represents

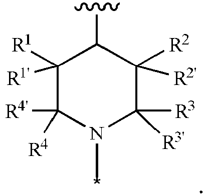

22. The compound having the structure of Formula I according to claim 17 or 18, or pharmaceutically acceptable salts, stereoisomers or isotope isomers thereof, wherein X represents O, NH, CR¹R^{1'}.

23. The compound having the structure of Formula I according to claim 20, or pharmaceutically acceptable salts, stereoisomers or isotope isomers thereof, wherein X represents CH₂ or CF₂.

24. The compound having the structure of Formula I according to any one of claims 17-19, or pharmaceutically acceptable salts, stereoisomers or isotope isomers thereof, wherein R², R^{2'}, R³, R^{3'}, R⁴ and R^{4'} each independently represent hydrogen, CH₃ or together with the carbon atom attached thereto form a cyclopropyl.

25. The compound having the structure of Formula I according to claim 1, or pharmaceutically acceptable salts, stereoisomers or isotope isomers thereof, wherein the chemical bond between X₅ and X₆ in ring A is fused with ring A to form a 5-6-membered saturated or unsaturated ring, which may optionally contain 0, 1 or 2 heteroatoms selected from O, S, N; the ring may be optionally substituted with 0-3 substituents selected from the following: halogen, cyano, nitro, hydroxy (C₁-C₆) alkyl, C₁-C₆ alkyl, C₃-C₈ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b}.

26. The compound having the structure of Formula I according to claim 1, or pharmaceutically acceptable salts, stereoisomers or isotope isomer thereof, wherein the chemical bond between X₅ and X₆ in ring A is fused with a benzene ring or a pyridine ring; the ring may be optionally substituted with 0-3 substituents selected from the following: halogen, cyano, nitro, hydroxy (C₁-C₆) alkyl, C₁-C₆ alkyl, C₃-C₈ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b}.

27. The compound having the structure of Formula I according to claim 1, or pharmaceutically acceptable salts, stereoisomers or isotope isomer thereof, wherein ring A is fused with a pyrrole ring at the bond between X₅ and X₆; the ring may be optionally substituted with 0-3 substituents selected from the following: halogen, cyano, nitro, hydroxy (C₁-C₆) alkyl, C₁-C₆ alkyl, C₃-C₈ cycloalkyl, C₁-C₆ haloalkyl, -OR^{a}, -SO₃R^{a}, -S(O)R^{a}, -O-C₁-C₆ haloalkyl, -SR^{a}, -C(=O)OR^{a}, -C(=O)NR^{a}R^{b}, -SF₅ or -NR^{a}R^{b}.

28. The compound having the structure of Formula I according to claim 1, or pharmaceutically acceptable salts, stereoisomers or isotope isomers thereof, wherein R^{L} represents L-R^{M}; wherein L represents -NR^{a}SO₂- or -SO₂NR^{a}-.

29. The compound having the structure of Formula I according to claim 1, or pharmaceutically acceptable salts, stereoisomers or isotope isomers thereof, wherein R^{M} represents C₁-C alkyl substituted with 0, 1, 2 or 3 OH groups.

30. Compounds with the following structures:
